(19) Europäisches Patentamt / European Patent Office / Office européen des brevets

(11) **EP 4 501 911 A1**

(12) **EUROPEAN PATENT APPLICATION**
published in accordance with Art. 153(4) EPC


(43) Date of publication:
**05.02.2025 Bulletin 2025/06**

(21) Application number: **23781324.1**

(22) Date of filing: **28.03.2023**

(51) International Patent Classification (IPC):
***C07D 211/60*** (2006.01) ***C07D 401/10*** (2006.01)
***C07D 403/10*** (2006.01) ***C07D 401/06*** (2006.01)
***C07D 405/06*** (2006.01) ***C07D 409/10*** (2006.01)
***C07D 405/10*** (2006.01) ***C07D 413/10*** (2006.01)
***A61K 31/445*** (2006.01) ***A61P 25/28*** (2006.01)

(52) Cooperative Patent Classification (CPC):
**A61K 31/40; A61K 31/401; A61K 31/445; A61K 31/4525; A61K 31/4535; A61K 31/4545; A61K 31/506; A61P 25/00; A61P 25/28; C07D 207/08; C07D 207/16; C07D 211/16; C07D 211/60; C07D 401/06; C07D 401/10;** (Cont.)

(86) International application number:
**PCT/KR2023/004139**

(87) International publication number:
**WO 2023/191469 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.03.2022 US 202263324221 P**



(71) Applicant: **BNH Research Co., Ltd.**
**Goyang-si, Gyeonggi-do 10594 (KR)**

(72) Inventors:
- **CHUNG, Seung Soo**
  **Goyang-si, Gyeonggi-do 10596 (KR)**
- **KIM, Young Hwan**
  **Goyang-si, Gyeonggi-do 10218 (KR)**
- **JEONG, Ji Hyun**
  **Seoul 03974 (KR)**

(74) Representative: **Ullrich & Naumann PartG mbB**
**Schneidmühlstrasse 21**
**69115 Heidelberg (DE)**


Remarks:
The complete document including Reference Table(s) and the Sequence Listing(s) can be downloaded from the EPO website

(54) **NITROGEN-CONTAINING HETEROCYCLIC COMPOUNDS HAVING PHENYL GROUP AND USES THEREOF**


(57) The present invention relates to nitrogen-containing heterocyclic compounds having a phenyl group and uses thereof. The compounds, according to the present invention, effectively enhance synaptic plasticity without side-effects and thus may exhibit treatment effects for various neurological disorders such as neurodegenerative disease, neurodegenerative pathology, neuropsychiatric disorder, amnesia, cognitive impairment/damage, and extinction learning disorder.


[FIG. 5]

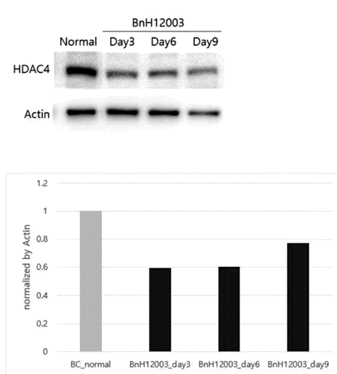


EP 4 501 911 A1

(52) Cooperative Patent Classification (CPC): (Cont.)
**C07D 403/10; C07D 405/06; C07D 405/10;**
**C07D 409/06; C07D 409/10; C07D 413/10;**
**G01N 33/487**

## Description

### Technical Field

The present disclosure relates to nitrogen-containing heterocyclic compounds having a phenyl group and uses thereof. More specifically, the present disclosure relates to compounds, compositions or methods for preventing or treating a neurological disorder or a neurodegenerative disorder.

### Background Art

Synaptic plasticity and critical period

A neural network is a network made of neurons. A synapse is a node in the neural network through which neurons exchange information with each other. Within a network, synapses are "plastic," because their numbers and strengths change continuously throughout life, as synaptic inputs are added, removed, or refined in response to ongoing neural activities. Synaptic plasticity refers to these neural activity-dependent modifications of the synapses, which is manifested by changes in the strength and/or efficiency of synaptic transmission.

Higher-order functions, such as learning, cognition, synthesis, judgment, art, or creativity, depend on the ability to form neural networks in the cerebral cortex (called the cortical neural network). Known as the critical period, synaptic plasticity of the cortical neural network peaks during the early days after birth. After the critical period, the cortical synaptic plasticity decreases gradually with age, eventually leading to a state in which experiences no longer results in active changes in the cerebral cortex. Interestingly, though, recent studies have demonstrated that under certain conditions, synaptic plasticity could be restored to heightened levels even after the critical period had been closed: in an animal model, peripheral nerve injury reactivated synaptic plasticity in the somatosensory thalamocortical circuits that transmits peripheral sensory signals to the cerebral cortex (Yu et al., 2012, Neuron. 74:731-42; Petrus et al., 2014, Neuron. 81:664-73; Gao et al., 2014, JNeurosci. 34:10770-9).

Reduced synaptic plasticity is also related to degeneration of the brain function in adults. In particular, the synaptic plasticity of the thalamic cortical circuit is significantly limited with age. If reopening the critical period is possible, as mentioned above, reactivating or increasing cortical synaptic plasticity via therapeutical means may be useful for treating conditions caused by reduced synaptic plasticity.

Histone deacetylase inhibitors

The bulk of the DNA in the cell exists as packed in a denser and more compacted form known as chromatin. The compaction occurs by winding the DNA around a group of protein called histones. The degree of chromatin compaction varies according to the level of acetylation of histone. In general, histone acetyltransferase (HAT) performs acetylation, which opens the access to the DNA, and histone deacetylase (HDAC) performs deacetylation, which closes the access to the DNA. Since transcription can only occur when the access is open, chromatin compaction works as a regulatory mechanism for gene expression (Verdin and Ott, 2015, Nat Rev Mol Cell Biol 16:258-264).

Eleven types of mammalian HDACs have been identified to date. These are largely classified into four classes based on the degree of homology with yeast HDAC. Class I includes HDACs 1, 2, 3 and 8. Class II HDACs are divided into Class IIa (HDACs 4, 5, 7, and 9) and Class IIb (HDACs 6 and 10) based on structural similarity. Class III HDACs includes SIRT1-7, and Class IV includes HDAC11.

While class I HDACs are mainly confined in the nucleus, Class II HDACs shuttle between the nucleus and the cytoplasm through the phosphorylation of specific serine residues. The N-terminal domains of HDACs interact with several tissue-specific transcription factors, and act as a transcriptional repressor. The C-terminal domains of HDACs possess the deacetylase activity. Class IIa HDACs, however, do not possess deacetylase activity because they carry a loss-of-function mutation in which their tyrosine (Tyr) residues in the C-terminal catalytic domains are substituted with histidine (His).

HDAC4

HDAC4 is expressed throughout the body. HDAC4 is particularly highly expressed in the brain, heart and skeletal muscle (Grozinger et al., 1999, Proc. Natl. Acad. Sci. USA 96, 4868-4873; Darcy et al., 2010, J. Comp. Neurol. 518, 722-740). In the brain, HDAC4 level peaks immediately after birth (Sando etal., 2012, Cell 151, 821-834). HDAC4 is detected in the corticotropin-releasing hormone (CRH) neurons, (100$\pm$0%), the oxytocin neurons (98$\pm$2%), the vasopressin neurons (100$\pm$0%), orexin neurons (100$\pm$0%), the AgRP neurons (100$\pm$0%), the POMC neurons (92$\pm$4%), the histamine neurons (85$\pm$7%), the dopamine neurons (100$\pm$0%), the serotonin neurons (93$\pm$2%), and the noradrenaline neurons (100$\pm$0%) (Takase et al., 2013, PloS ONE 8:e58473).

Cytoplasmic HDAC4 is rapidly translocated to the nucleus in response to a low potassium or excitotoxic glutamate condition that induces apoptosis. Ectopic overexpression of HDAC4 in the nucleus promoted neuronal cell death and inhibited the transcriptional activities of survival factors, such as MEF2 and CREB (Bolger and Yao, 2005, JNeurosci. 2005; 25 :9544-9553). Neurons containing HDAC4 are uniform in size and are widely distributed in neuropils including paraventricular nucleus (PVN), lateral hypothalamus (LHA), hypothalamic arcuate nucleus (ARC), tuberous mammary nucleus (TMN), dorsal raphe (DR) and locus coeruleus (LC). In particular, HDAC4 is co-localized with PSD95 (Mielcarek et al., 2013, PLoS Biol. 11:e1001717; Takase et al., 2013, PloS ONE 8:e58473), suggesting a role for HDAC4 in synaptic plasticity (Sando et al., 2012, Cell 151, 821-834; Mielcarek et al., 2013, PLoS Biol. 11:e1001717).

In animal experimental models, tissue-specific deletion of HDAC4 in rat hippocampus abolished long-lasting, stressinduced behavioral changes (Sailaja etal., 2012, Proc. Natl. Acad. Sci. USA 109, E3687-E3695). HDAC4 overexpression accelerated the death of cerebellar granule neurons by increasing susceptibility to $H_2O_2$ damage through inhibition of the activity of peroxisome proliferator-activated receptor $\alpha$ (PPAR$\alpha$) (Bolger and Yao, 2005, J. Neurosci. 25, 9544-9553; Li et al., 2012, Nat. Med. 18, 783-790; Sando et al., 2012; Cell 151, 821-834; Yang et al., 2011, FEBS Lett. 585, 761-766). HDAC4 overexpression in the rat hippocampus induced depression-like behaviors (Sarkar et al., 2014, Neuropsychopharmacology 39, 2221-2232).

In the Drosophila model, the overexpression of HDAC4 in adult mushroom body neurons, which are structures important for memory formation, caused damage to long-term courtship memory, but had no effect on short-term memory (Fitzsimons et al., 2013, PloS ONE 8:e83903). A subsequent study examining the relationship with the subcellular distribution of HDAC4 showed that mushroom body morphogenesis, eye development, and deficits in long-term memory were associated with nuclear HDAC4 abundance (Main et al., 2021, Front. Mol. Neurosci. 14:616-642).

In humans, an increase in HDAC4 levels was confirmed in the post-mortem HD frontotemporal lobar degeneration (FTLD) brain (Yeh et al., 2013, Brain Res. 1504, 16-24; Whitehouse et al., 2014, Neuropathol. Appl. Neurobiol. 41, 245257). HDAC4 was identified as a component of Lewy bodies in Parkinson's disease brains and of intranuclear inclusions in the neuronal intranuclear inclusion disease (Takahashi-Fujigasaki and Fujigasaki, 2006, Neuropathol. Appl. Neurobiol. 32, 562-566). HDAC4 was observed to be significantly overexpressed in the specific cortical regions of autistic patients (Nardone et al., 2014, Transl. Psychiatry 4:e433).

<u>HDAC inhibitors and synaptic plasticity</u>

Histone modification have been proposed as a potential method to restore cortical plasticity in adulthood. Valproic acid is known as a histone deacetylase (HDAC) inhibitor. In a clinical study that participants took valproic acid capsules, the critical period was reopened and absolute pitch learning, which was only possible during the critical period, became possible even in adults (Gervain et al., 2013, Front Syst Neurosci. 7:102). When adult rats were exposed to an enriching environment as compared to a normal cage condition, the total amount of H3 acetylation of the visual cortex was increased compared to the control group, and ocular dominance plasticity, which appears only during the critical period, was also observed after the critical period (Baroncelli L et al., 2016, JNeurosci. 36:3430-40).

Treatment with suberoylanilide hydroxamic acid (SAHA), which is an HDAC inhibitor, increased H3 acetylation in the visual cortex to the level typically observed in young rats, and in particular, the H3 acetylation of the P3 promotor of brain-derived neurotrophic factor (BNDF), which is known for its importance in developmental synaptic plasticity and longterm memory in the visual cortex, was also increased (Baroncelli L etal., 2016, JNeurosci. 36:3430-40). The increased BDNF expression was also observed in neurospheres as well (Bagheri et al., 2019, Int. J. Mol. Sci. 20: 1109).

SAHA or other HDAC inhibitors have been found to have an effect of improving symptoms in a mouse model of Huntington's disease (HD) (Ferrante *et al.,* 2003; Hockly et al., 2003, J Biol. Chem. 278, 16059-16072; Gardian et al., 2005, J. Biol. Chem. 280, 556-563). HDAC4 is correlated with the length of polyglutamine expressed in Huntington's disease. It is co-localized with cytoplasmic inclusions, and a wide range of peripheral organ pathologies such as skeletal muscle atrophy and heart failure that appear in Huntington's disease (Zielonka et al., 2014, Front. Physiol. 5:380; Mielcarek et al., 2014, PLoS Genet. 10:e1004550; Mielcarek et al., 2014, PloS ONE 9:e108961; Zielonka et al., 2014, Exp. Clin. Cardiol. 20, 25472554). Reducing HDAC4 levels in R6/2, which is a mouse model of Huntington's disease, delayed cytoplasmic aggregate formation in various regions of the brain, improved motor co-ordination and neurological phenotype, and had a lifespan extension effect (Mielcarek et al., 2013, PLoS Biol. 11:e1001717).

Interestingly, SAHA treatment in R6/2 mice restored cortical transcript levels of BDNF (Mielcarek et al., 2011, PloS ONE 6:e27746). Since HDAC4 and HDAC5 can inhibit specific BDNF transcripts and this effect has been shown to be reversed by SAHA treatment, memory enhancing and neuroprotective effects by HDAC inhibitors may be associated with increased BDNF expression (Koppel and Timmusk, 2013, Neuropharmacology 75, 106-11).

HDAC4 may play a central role in brain physiology and could be a useful target for treating several neurodegenerative disorders. However, currently available HDAC inhibitors are broad spectrum HDAC inhibition, rendering them unsuitable for inhibiting a specific HDAC. Although SAHA increases the synaptic plasticity, it causes severe side effects including fatigue, nausea, diarrhea, or thrombocytopenia that renders it unsuitable for human use (Meghan E. et al., 2011,

Bone. 48: 1117-1126).

Therefore, it would be useful to develop selective HDAC inhibitors for targeted therapy.

## Disclosure of Invention

## Technical Problem

The object of the present disclosure is to solve all of the above-mentioned problems.

An object of the present disclosure is to provide a nitrogen-containing heterocyclic compound having a phenyl group, a stereoisomer thereof, or a pharmaceutically acceptable salt thereof for improving synaptic plasticity.

Another object of the present disclosure is to provide a pharmaceutical composition for treating or preventing a neurological disorder or a neurodegenerative disorder that is mediated by HDAC, comprising, as an active ingredient, a nitrogen-containing heterocyclic compound having a phenyl group, a stereoisomer thereof, or a pharmaceutically acceptable salt thereof for improving synaptic plasticity.

Yet another object of the present disclosure is to provide a method for treating or preventing a neurological disorder or a neurodegenerative disorder that is mediated by HDAC, comprising administering to a subject a nitrogen-containing heterocyclic compound having a phenyl group, a stereoisomer thereof, or a pharmaceutically acceptable salt thereof for improving synaptic plasticity.

Still yet another object of the present disclosure is to provide a pharmaceutical composition for combined administration, comprising a nitrogen-containing heterocyclic compound having a phenyl group, a stereoisomer thereof, or a pharmaceutically acceptable salt thereof for improving synaptic plasticity, and a second therapeutic agent, and a combined administration method using the same.

The object of the present disclosure is not limited to the above-mentioned objects. The objects of the present disclosure will become clearer from the following description and may be realized by means and combinations thereof as set forth in the claims.

## Solution to Problem

Representative configurations of the present disclosure to achieve the above-mentioned objects are as follows.

According to an aspect of the present disclosure, there is provided a compound of Formula (1), a stereoisomer thereof, or a pharmaceutically acceptable salt thereof:

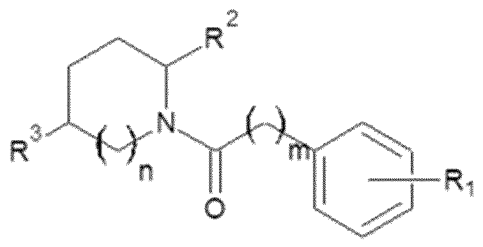

(1)

in the formula, $R^1$ is aryl or heteroaryl, or is combined with an adjacent phenyl group to form a saturated or partially saturated heterocycle, wherein at least one hydrogen atom in the aryl, heteroaryl, or heterocycle is unsubstituted or substituted with a halogen atom, $C_{1-6}$ alkyl, $C_{3-12}$ cycloalkyl, $C_{1-6}$ alkoxy, aryl, heteroaryl, amino, or nitro, and the substituent is unsubstituted or substituted with at least one halogen atom or $C_{1-6}$ alkyl,

$R^2$ is hydrogen, $C_{1-6}$ alkyl, or aryl,

$R^3$ is hydrogen, carboxyl, N-hydroxycarboxamide, or carboxamide, wherein hydrogen in the group may be substituted with $C_{1-6}$ alkyl,

n is 0 or 1, and

m is 0 to 6.

In an embodiment, $R^1$ may be phenyl, pyridinyl, pyridazinyl, pyrimidinyl, pyrazinyl, furanyl, thiophenyl, pyrrolyl, imidazolyl, or oxazolyl, or may be combined with an adjacent phenyl group to form quinoxalinyl or dihydrobenzodioxynyl.

In another embodiment, at least one hydrogen atom in $R^1$ may be substituted with a halogen atom, $C_{1-6}$ alkyl or

$C_{3-12}$ cycloalkyl unsubstituted or substituted with at least one halogen atom, $C_{1-6}$ alkoxy, phenyl, pyridinyl, pyridazinyl, pyrimidinyl, amino unsubstituted or substituted with at least one $C_{1-6}$ alkyl, or nitro.

In an embodiment, $R^1$ may be phenyl, biphenyl, pyridin-4-yl, pyridin-3-yl, pyridin-2-yl, methyl pyridin-3-yl, 6-methoxy-pyridin-3-yl, 6-cyclopropyl-pyridin-3-yl, trifluoromethylpyridin-3-yl, phenylpyridin-3-yl, pyridazin-4-yl, pyridazin-3-yl, pyridazin-2-yl, pyrimidin-2-yl, pyrimidin-4-yl, pyrimidin-5-yl, methylpyrimidin-5-yl, 5-fluoropyrimidin-2-yl, 2-(trifluoromethyl)pyrimidin-5-yl, 2-cyclopropylpyrimidin-5-yl, pyrazin-2-yl, thiophen-2-yl, furan-2-yl, oxazol-2-yl, 3-nitrophenyl, 4-nitrophenyl, 3-aminophenyl, 4-aminophenyl, or 4-diethylaminophenyl.

In an embodiment, $R^2$ may be H or $C_{1-6}$ alkyl.

In an embodiment, $R^3$ may be H, carboxyl, methyl carboxyl, ethyl carboxyl, N-hydroxycarboxamide, or carboxamide.

In an embodiment, the steroisomer may be an optical isomer.

In an embodiment, the stereoisomer may be (3R,6S)-optical isomer.

In an embodiment, the compound may be a compound selected from the compounds as shown in Tables 2 and 3, a stereoisomer thereof, or a pharmaceutically acceptable salt thereof.

In another embodiment, the compound may be selected from the group consisting of BnH12003, BnH12017, BnH12045, BnH12046, BnH12047, BnH12048, BnH12051, BnH12053, BnH12057, BnH12058, BnH12064, BnH12066, BnH12067, BnH12071, BnH12072, BnH12078, BnH12085, BnH12086, BnH12088, BnH12089, BnH12090, BnH12091, BnH12092, BnH12093, BnH12095, BnH12097, and BnH12098.

In an embodiment, the compound may be an inhibitor of HDAC. In another embodiment, the HDAC may be HDAC4.

In an embodiment, the compound may increase synaptic plasticity.

In another aspect of the present disclosure, there is provided a pharmaceutical composition comprising, as an active ingredient, the compound, a stereoisomer thereof, or a pharmaceutically acceptable salt thereof.

In an embodiment, the composition may be for prevention or treatment of a neurological disorder or a neurodegenerative disorder.

In another embodiment, the compound, a stereoisomer thereof, or a pharmaceutically acceptable salt thereof may be delivered to the brain at a concentration of about 0.1 to about 3 $\mu$M.

In another embodiment, the disorder may be Alzheimer's disease.

In another embodiment, the disorder may be PTSD.

In an embodiment, the composition may further comprise a second therapeutic agent.

In another embodiment, the second therapeutic agent may be solanezumab.

According to still yet another aspect of the present disclosure, there is provided a method for preventing or treating a neurological disorder or a neurodegenerative disorder, comprising administering, to a subject in need thereof, the compound, a stereoisomer thereof, or a pharmaceutically acceptable salt thereof.

According to still yet another aspect of the present disclosure, there is provided a use of the compound, a stereoisomer thereof or a pharmaceutically acceptable salt thereof as a medicament or for manufacture of a medicament.

According to an embodiment, the use may be for reactivating or increasing brain synaptic plasticity.

According to another embodiment, the use may be for prevention or treatment of a neurological disorder or a neurodegenerative disorder.

## Advantageous Effects of Invention

The compound according to the present disclosure can exert a therapeutic effect on various neurological disorders such as neurodegenerative diseases, neurodegenerative pathologies, neuropsychiatric disorders, memory loss, cognitive dysfunction/impairment, and extinction learning disorders by effectively enhancing or reactivating synaptic plasticity without any adverse effects.

## Brief Description of Drawings

FIG. 1A illustrates a measurement of cell viability in response to the administration of BnH12003 (0, 0.1, 1, 10, 100 $\mu$M) in SH-SY5Y cells.

FIG. 1B illustrates a measurement of cell viability in response to the administration of BnH12003 (0, 0.1, 1, 10, 100 $\mu$M) in 293T cells.

FIG. 2A illustrates an osmotic pump (Os pump) injection site in a mouse experiment.

FIG. 2B illustrates a patch recording site in a mouse experiment.

FIG. 3A illustrates a measurement of excitatory/inhibitory balance in the mouse barrel cortex (layer 4) in response to the administration of BnH12003 (Os pump, ventricle injection, 3 μM).

FIG. 3B illustrates changes in TC EPSC potency in the mouse barrel cortex (layer 4) in response to the in response to the administration of BnH12003 (Os pump, ventricle injection, 3 μM).

FIG. 4A illustrates a measurement of excitation/inhibition balance in the mouse barrel cortex (layer 4) in response to the intraperitoneal administration of BnH12003 (25 mg/kg).

FIG. 4B illustrates changes in TC EPSC potency in the mouse barrel cortex (layer 4) in response to the intraperitoneal administration of BnH12003 (25 mg/kg).

FIG. 5 illustrates a measurement of changes in the expression levels of HDAC4 protein in the mouse barrel cortex (layer 4) in response to the administration of BnH12003 (Os pump, barrel cortex injection).

FIG. 6 illustrates the degree of improvement in symptoms in a rodent (rat) model of post-traumatic stress disorder in response to the intraperitoneal administration of BnH12003 (25mg/kg).

FIG. 7A illustrates Input/Output curve (I/O curve) changes of field excitatory postsynaptic potentials (fEPSP) in the hippocampus of Alzheimer's model mice in response to administration of BnH12003 and solanezumab (Os pump; 3 μM, 200 μg).

FIG. 7B illustrates an LTP measurement of field excitatory postsynaptic potentials (fEPSP) in the hippocampus of Alzheimer's model mice in response to administration of BnH12003 and solanezumab (Os pump; 3 μM, 200 μg).

FIG. 8A illustrates a measurement of excitatory/inhibitory balance in the barrel cortex (layer 4) of Alzheimer's model mice in response to administration of BnH12003 and solanezumab (Lateral ventricle, Os pump; 3 μM, 200 μg).

FIG. 8B illustrates changes in TC EPSC potency in the barrel cortex (layer 4) of Alzheimer's model mice the compound in response to the administrations of BnH12003 and solanezumab (Os pump; 3 μM, 200 μg).

FIG. 9 illustrates the binding properties of BnH12003 to HDAC4.

FIGS. 10A and 10B illustrate HDAC4 shRNA's knockdown effect on HDAC4, BDNF, and GluN2B expressions in human cell line SH-SY5Y.

FIGS. 11A to 11C illustrate the effect of BnH12003 on the expression of BDNF and GluN2B.

FIGS. 12A and 12B illustrate the time course of BnH12003's effects on HDAC4 expression.

**Modes for Carrying out Invention**

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. Unless otherwise indicated, terms used in describing the present disclosure are to be understood in their ordinary meaning and apply to the same terms used herein as well as to the aspect or embodiment of the disclosure for which the term is defined.

Throughout the description, where compositions and kits are described as having, including, or comprising specific components, it is contemplated that, additionally, there are compositions and kits of the present disclosure that consist essentially of, or consist of, the recited components.

In the application, where an element or component is said to be included in and/or selected from a list of recited elements or components, it should be understood that the element or component can be any one of the recited elements or components, or the element or component can be selected from a group consisting of two or more of the recited elements or components.

The order of steps or order for performing certain actions is immaterial so long as the present teachings remain

operable. Moreover, two or more steps or actions may be conducted simultaneously.

At various places in the present specification, variable or parameters are disclosed in groups or in ranges. It is specifically intended that the description include each and every individual subcombination of the members of such groups and ranges.

The use of any and all examples, or exemplary language herein, for example, "such as" or "including," is intended merely to illustrate better the present disclosure and does not pose a limitation on the scope of the disclosure unless claimed. No language in the specification should be construed as indicating any non-claimed element as essential to the practice of the present disclosure.

## I. Definition

The articles "a" and "an" are used in this disclosure to refer to one or more than one (that is, at least one), unless the context is inappropriate.

"And/or" is used in this disclosure to mean either "and" or "or" unless indicated otherwise.

"At least one" includes individually each of the recited objects after the expression and the various combinations of two or more of the recited objects unless otherwise understood from the context and use.

"Comprise," "comprises," "comprising," "include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

"About" means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

"Individual," "patient," and "subject" are used interchangeably and include any animal, including mammals, for example, mice, rats, other rodents, rabbits, dogs, cats, swine, cattle, sheep, horses, or primates, including humans.

"Treat", "treating," or "treatment" includes any effect for example, lessening, reducing, modulating, ameliorating, alleviating or eliminating, that results in the improvement of the condition, disease, disorder, and the like, or ameliorating a symptom thereof. Treating can be curing, improving, or at least partially ameliorating the disorder. In certain embodiments, treating is curing the disease.

"Active agent" is used to indicate a compound or a pharmaceutically acceptable salt thereof which has biological activity. In some embodiments, the "active agent" is a compound or pharmaceutically acceptable salt thereof having pharmaceutical utility. For example, an active agent may be an anti-neurodegenerative therapeutic.

"Compound" means a chemical having a structure represented by chemical formula described herein, a stereoisomer thereof including a mixture of the stereoisomer, or a pharmaceutically acceptable salt thereof,.

"Pharmaceutically acceptable" includes molecular entities and formulations that do not produce an adverse, allergic or other untoward reaction when administered to an animal, or a human, as appropriate.

"Pharmaceutically acceptable salt" refers to those salts which retain the biological effects and properties of the free base or free acid. For example, pharmaceutically acceptable salts may be prepared by the addition of an inorganic base or an organic base to the free acid. Salts derived from inorganic bases include, but are not limited to, sodium, potassium, lithium, ammonium, calcium, magnesium salts, and the like. Salts derived from organic bases include, but are not limited to, primary, secondary, and tertiary amines, substituted amines including naturally occurring substituted amines, cyclic amines and basic ion exchange resins such as isopropylamine, trimethylamine, diethylamine, triethylamine, tripropylamine, ethanolamine, diethylamine, lysine, arginine, N-ethylpiperidine, piperidine, piperazine, and the like.

"Pharmaceutically acceptable excipient" and "pharmaceutically acceptable carrier" refer to a substance that aids the administration of an active agent to and absorption by a subject and can be included in the compositions of the present disclosure without causing a significant adverse toxicological effect on the patient. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethylcellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the disclosure. One of skill in the art will recognize that other pharmaceutical excipients are useful in the present disclosure.

Exemplary pharmaceutically acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; synthetic oils; vegetable oils, such as peanut oil, cottonseed oil, sesame oil,

olive oil, and corn oil; polyols such as propylene glycol, glycerin, sorbitol, mannitol, and polyethylene glycol; alginic acid; phosphate buffer solutions; emulsifiers, such as the TWEENs®; wetting agents, such sodium lauryl sulfate; coloring agents; flavoring agents; tableting agents; stabilizers; antioxidants; preservatives; pyrogen-free water; isotonic saline; and phosphate buffer solutions.

"Pharmaceutical composition" or "pharmaceutical formulation" refers to the combination of an active agent with a carrier, inert or active.

"Effective amount" refers to the amount of a compound or composition (for example, a compound or composition of the present disclosure) sufficient to effect beneficial or desired results. An effective amount may be administered in one or more administrations, applications or dosages and is not intended to be limited to a particular formulation or administration route.

"Administering" means oral administration, administration as a suppository, topical contact, intravenous, parenteral, intraperitoneal, intramuscular, intralesional, intrathecal, intracranial, intracerebroventricular, intranasal or subcutaneous administration, or the implantation of a slow-release device, for example, a mini-Os pump, to a subject. Administration is by any route, including parenteral and transmucosal (for example, buccal, sublingual, palatal, gingival, nasal, vaginal, rectal, or transdermal). Parenteral administration includes, for example, intravenous, intramuscular, intraarterial, intradermal, subcutaneous, intraperitoneal, intraventricular, and intracranial. Other modes of delivery include, but are not limited to, the use of liposomal formulations, intravenous infusion, transdermal patches, and the like. By "co-administration" or "combinational administration" it is meant that a composition described herein is administered at the same time, just prior to, or just after the administration of one or more additional therapies (for example, anticancer agent, chemotherapeutic, or treatment for a neurodegenerative disease). The compounds of the disclosure can be administered alone or can be co-administered to the patient. Coadministration is meant to include simultaneous or sequential administration of the compounds individually or in combination (more than one compound or agent). Thus, the preparations can also be combined, when desired, with other active substances (for example, to reduce metabolic degradation).

The pharmaceutical formulations of the disclosure may be administered to a mammal, such as a human, but may also be administered to other mammals such as an animal in need of veterinary treatment, for example, domestic animals (for example, dogs, cats, and the like), farm animals (for example, cows, sheep, pigs, horses, and the like), and laboratory animals (for example, rats, mice, guinea pigs, and the like).

"Cognitive function" of a subject may be defined as an intellectual activity or process. Examples of intellectual activities or processes include, but are not limited to, attention, processing speed, learning and memory, executive function, verbal fluency and working memory. For example, the composition of the present disclosure may improve cognitive function if it improves one or more intellectual activities or processes in a subj ect with impaired cognitive function.

"Histone deacetylase" or "HDAC" are intended to refer to any one of a family of enzymes that remove Nε-acetyl groups from the ε-amino groups of lysine residues of a protein (for example, a histone, or tubulin). Unless otherwise indicated by context, the term "histone" is meant to refer to any histone protein, including H1, H2A, H2B, H3, H4, and H5, from any species.

"A condition or disorder mediated by HDAC" or "a condition or disorder mediated by histone deacetylase" refers to a condition or disorder in which HDAC and/or the action of HDAC is important or necessary, for example, for the onset, progress, or expression.

"Inhibiting HDAC enzymatic activity" or "inhibiting HDAC" means reducing the HDAC's catalytic activity or reducing the HDAC's activities not related to its catalytic activity.

"Neurological disorder" includes neurodegenerative disease, neuropsychiatric disorder, memory loss, cognitive function disorders/impairment, extinction learning disorders.

"Neurodegenerative disease" implies any disorder that might be reversed, deterred, managed, treated, improved, or eliminated with agents that stimulate the generation of new neurons.

"Memory" refers to the ability to recover information about past events or knowledge.

"Age related memory loss" refers to any of a continuum of conditions characterized by a deterioration of neurological functioning that does not rise to the level of a dementia, as further defined herein and/or as defined by the Diagnostic and Statistical Manual of Mental Disorders: 4th Edition of the American Psychiatric Association (DSM-IV, 1994).

"Injury related memory loss" refers to a loss of memory wherein there is damage to the brain, and there may have also been neurological damage.

"Impaired cognitive function" refers to cognitive function that is not as robust as that observed in an age-matched normal subject and includes states in which cognitive function is reduced.

"Alkyl", as used herein, unless otherwise specified, refers to a saturated straight, branched, or cyclic, primary, secondary, or tertiary hydrocarbon of typically $C_1$ to $C_{10}$, and specifically includes methyl, $CF_3$, $CCl_3$, $CFCl_2$, $CF_2Cl$, ethyl, $CH_2CF_3$, $CF_2CF_3$, propyl, isopropyl, cyclopropyl, butyl, isobutyl, sec-butyl, t-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. The term

includes both substituted and unsubstituted alkyl groups, and particularly includes halogenated alkyl groups, and even more particularly fluorinated alkyl groups. Non-limiting examples of moieties with which the alkyl group can be substituted are selected from the group consisting of halogen (fluoro, chloro, bromo, or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

As used herein, unless otherwise specified, "aryl" refers to an aromatic monocyclic or polycyclic system containing from 6 to 14 carbon atoms, and specifically includes phenyl, biphenyl, or naphthyl, preferably phenyl. The term includes both substituted and unsubstituted moieties. Aryl groups may be substituted with any described moiety, including, but not limited to, one or more moieties selected from the group consisting of halogen (fluoro, chloro, bromo, or iodo), hydroxyl, amino, alkylamino, arylamino, cycloalkyl, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

As used herein, "heteroaryl", unless otherwise specified, refers to an aromatic monocyclic or polycyclic system having 5 to 14 ring atoms, wherein 1 to 4 of the ring atoms are independently O, N, or S and the remaining ring atoms are carbon atoms, preferably may be an aromatic monocyclic system having 5 or 6 ring atoms, wherein 1 to 4 of the ring atoms are independently O, N or S and the remaining ring atoms are carbon atoms, and specifically includes pyridinyl, pyridazinyl, pyrimidinyl, pyrazinyl, pyrrolyl, furanyl, thiophenyl, imidazolyl, or oxazolyl. Heteroaryl groups may be substituted with any described moiety, including, but not limited to, one or more moieties selected from the group consisting of halogen (fluoro, chloro, bromo, or iodo), hydroxyl, amino, alkylamino, arylamino, cycloalkyl, alkoxy, aryloxy, thiol, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

## II. Active compounds

In an aspect of the present disclosure, there is provided a compound of Formula (1), a stereoisomer thereof, or a pharmaceutically acceptable salt thereof:

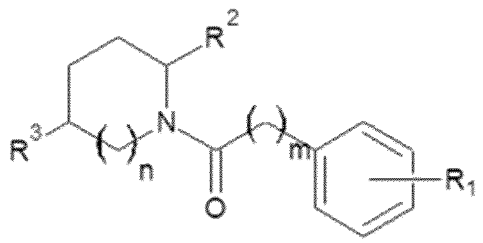

(1)

in the formula,

$R^1$ is aryl or heteroaryl, or is combined with an adjacent phenyl group to form a saturated or partially saturated heterocycle, wherein at least one hydrogen atom in the aryl, heteroaryl, or heterocycle is unsubstituted or substituted with a halogen atom, $C_{1-6}$ alkyl, $C_{3-12}$ cycloalkyl, $C_{1-6}$ alkoxy, aryl, heteroaryl, amino, or nitro, and the substituent is unsubstituted or substituted with at least one halogen atom or $C_{1-6}$ alkyl,

$R^2$ is hydrogen, $C_{1-6}$ alkyl, or aryl,

$R^3$ is hydrogen, carboxyl, N-hydroxycarboxamide, or carboxamide, wherein hydrogen in the group may be unsubstituted or substituted with $C_{1-6}$ alkyl,

n is 0 or 1, and

m is 0 to 6.

In an embodiment, the saturated or partially saturated heterocycle may be a bicyclic ring having 9 to 12 ring atoms, wherein at least one (preferably one or two) of the ring atoms are independently O, N or S and the remaining ring atoms are carbon atoms.

In an embodiment, the stereoisomer may be an optical isomer. Preferably, the compound may be in the form of a (3R,6S)-optical isomer or a (3S,6R)-optical isomer.

In an embodiment, $R^1$ may be phenyl, pyridinyl, pyridazinyl, pyrimidinyl, pyrazinyl, furanyl, thiophenyl, pyrrolyl, imidazolyl, or oxazolyl, or may be combined with an adjacent phenyl group to form quinoxalinyl or dihydrobenzodioxynyl.

In an embodiment, at least one hydrogen atom in $R^1$ may be unsubstituted or substituted with a halogen atom, $C_{1-6}$ alkyl, $C_{3-12}$ cycloalkyl, $C_{1-6}$ alkoxy, aryl (preferably phenyl), heteroaryl (preferably pyridinyl, pyridazinyl, pyrimidinyl, pyrrolyl, furanyl, thiophenyl, imidazolyl, or oxazolyl), amino, or nitro, in which the substituent may be further substituted with at least one group selected from a halogen atom and $C_{1-6}$ alkyl.

In an embodiment, at least one hydrogen atom in $R^1$ may be substituted with a halogen atom, $C_{1-6}$ alkyl or $C_{3-12}$ cycloalkyl unsubstituted or substituted with at least one halogen atom, $C_{1-6}$ alkoxy, phenyl, pyridinyl, pyridazinyl, pyrimidinyl, amino unsubstituted or substituted with at least one $C_{1-6}$ alkyl, or nitro.

In one embodiment, $R^1$ may be phenyl, biphenyl, pyridin-4-yl, pyridin-3-yl, pyridin-2-yl, methylpyridin-3-yl, 6-methoxy-pyridin-3-yl, 6-cyclopropyl-pyridin-3-yl, trifluoromethylpyridin-3-yl, phenylpyridin-3-yl, pyridazin-4-yl, pyridazin-3-yl, pyridazin-2-yl, pyrimidin-2-yl, pyrimidin-4-yl, pyrimidin-5-yl, methylpyrimidin-5-yl, 5-fluoropyrimidin-2-yl, 2-(trifluoromethyl)pyrimidin-5-yl, 2-cyclopropylpyrimidin-5-yl, thiophen-2-yl, furan-2-yl, oxazol-2-yl, 3-nitrophenyl, 4-nitrophenyl, 3-aminophenyl, 4-aminophenyl, or 4-diethylaminophenyl.

In an embodiment, $R^1$ may be substituted at position 3 or 4 of the phenyl group.

In an embodiment, $R^2$ may be H or $C_{1-6}$ alkyl.

In an embodiment, $R^3$ may be H, carboxyl, methyl carboxyl, ethyl carboxyl, N-hydroxy carboxamide, or carboxamide.

In an embodiment, m may be 0 to 3. In another embodiment, m may be 0 or 1.

In an embodiment, the compound of Formula (1) may be provided in the form of a pharmaceutically acceptable salt thereof. Preferably, the salt may be a sodium salt.

In an embodiment, the compound of Formula (1) includes, but is not limited to, the compounds set forth in Table 1:

[Table 1]

| Name | Structure | Name | Structure |
|---|---|---|---|
| (1) BnH 12002 | 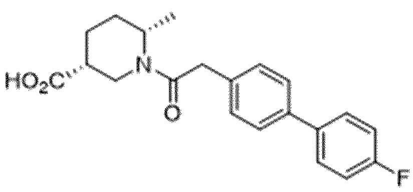 | (2) BnH 12003 | 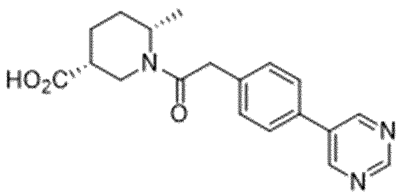 |
| (3) BnH 12015 | 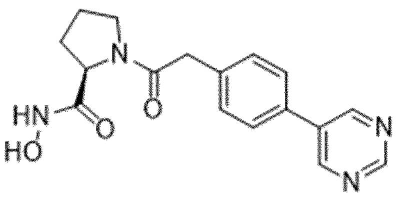 | (4) BnH 12017 | 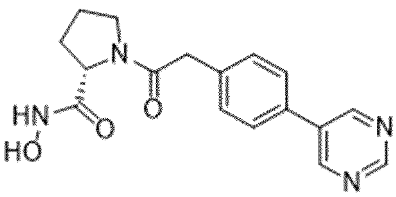 |
| (5) BnH 12036 | 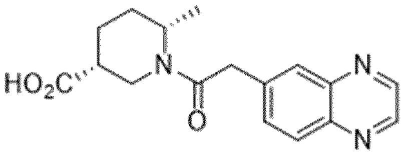 | (6) BnH 12037 | 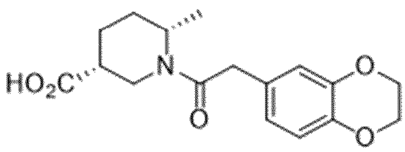 |
| (7) BnH 12042 | 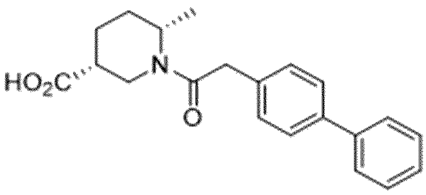 | (8) BnH 12043 | 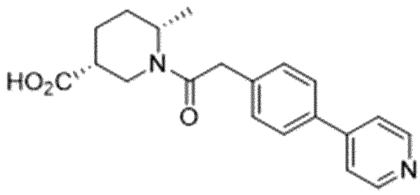 |

(continued)

| Name | Structure | Name | Structure |
|---|---|---|---|
| (9) BnH 12044 | 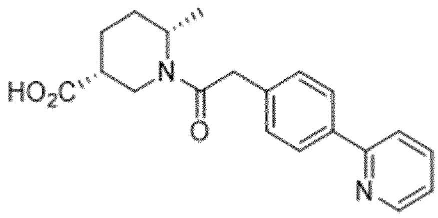 | (10) BnH 12045 | 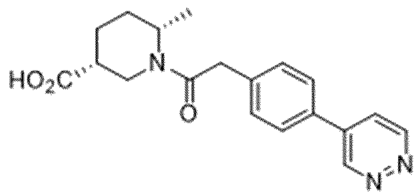 |
| (11) BnH 12046 | 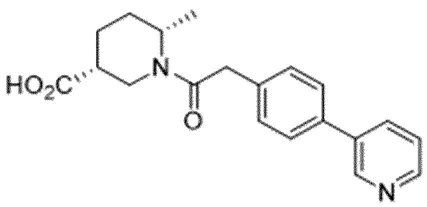 | (12) BnH 12047 | 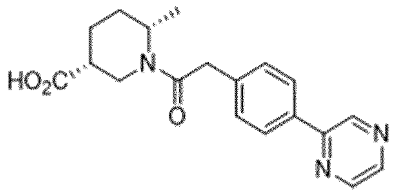 |
| (13) BnH 12048 | 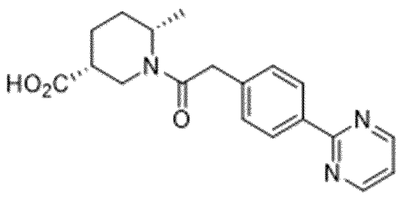 | (14) BnH 12049 | 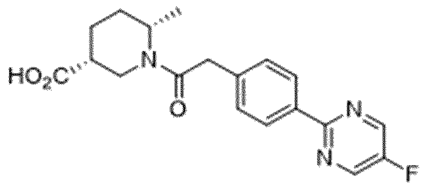 |
| (15) BnH 12050 | 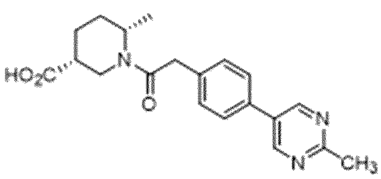 | (16) BnH 12051 | 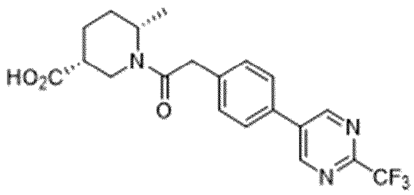 |
| (17) BnH 12052 | 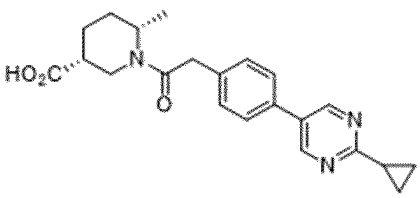 | (18) BnH 12053 | 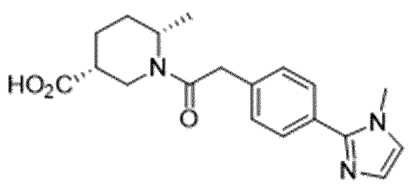 |
| (19) BnH 12054 | 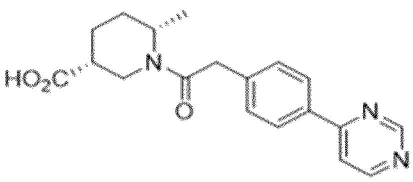 | (20) BnH 12055 | 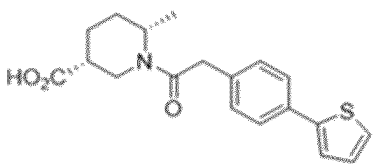 |
| (21) BnH 12056 | 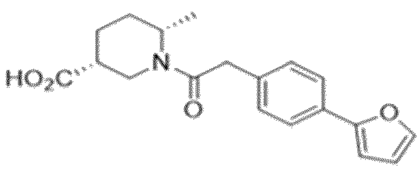 | (22) BnH 12057 | 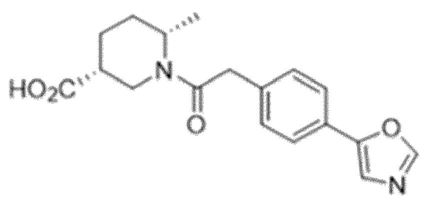 |

(continued)

| Name | Structure | Name | Structure |
|---|---|---|---|
| (23) BnH 12058 | 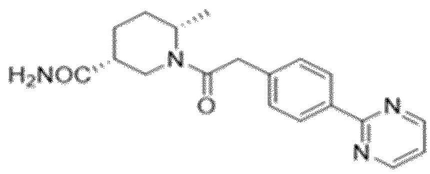 | (24) BnH 12059 | 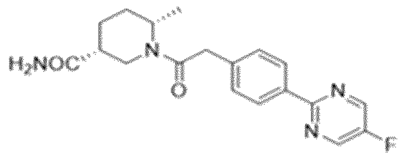 |
| (25) BnH 12064 | 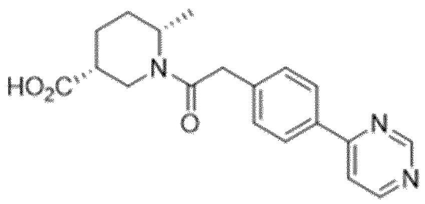 | (26) BnH 12066 | 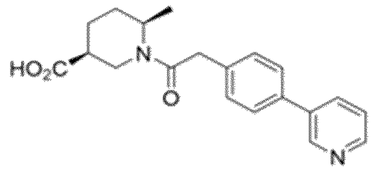 |
| (27) BnH 12067 | 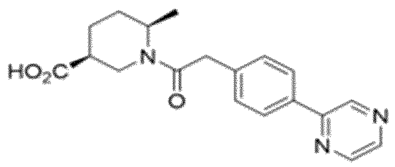 | (28) BnH 12071 | 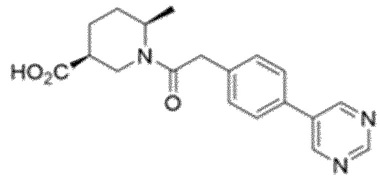 |
| (23) BnH 12072 | 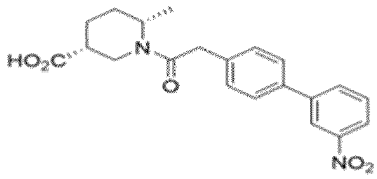 | (30) BnH 12077 | 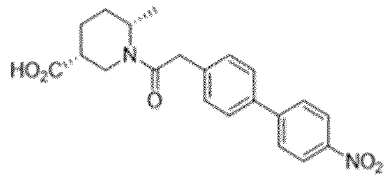 |
| (31) BnH 12078 | 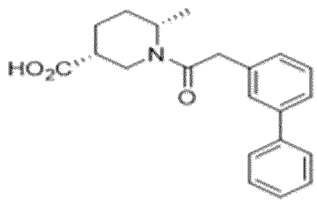 | (32) BnH 12080 | 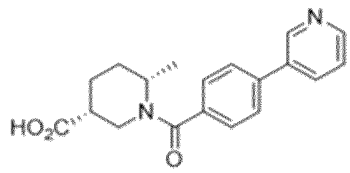 |
| (33) BnH 12081 | 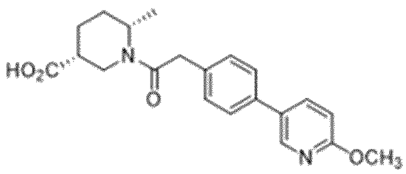 | (34) BnH 12082 | 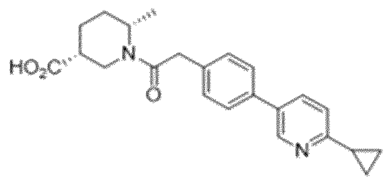 |
| (35) BnH 12083 | 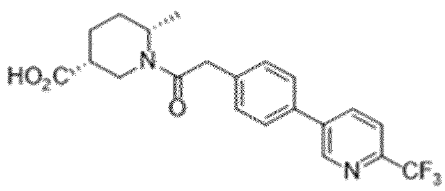 | (36) BnH 12084 | 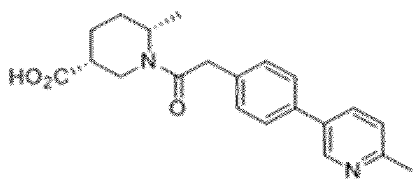 |
| (37) BnH 12085 | 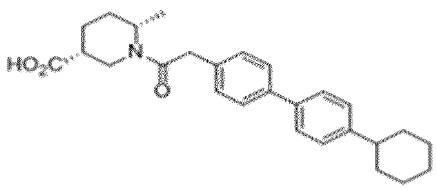 | (38) BnH 12086 | 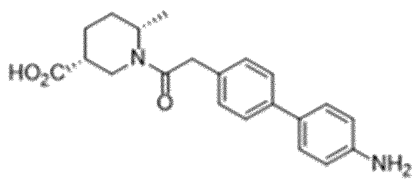 |

(continued)

| Name | Structure | Name | Structure |
|---|---|---|---|
| (39) BnH 12087 | 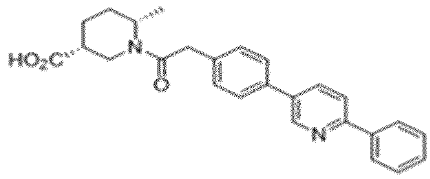 | (40) BnH 12088 | 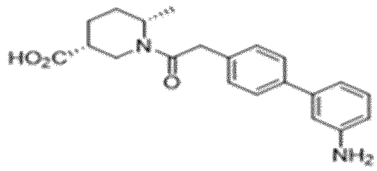 |
| (41) BnH 12089 | 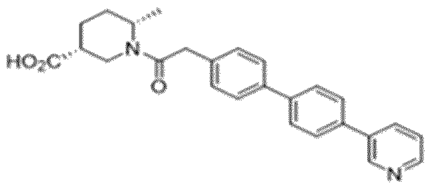 | (42) BnH 12090 | 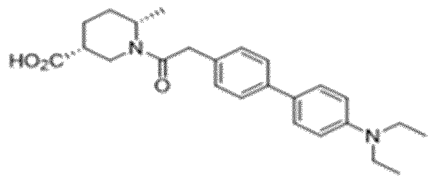 |
| (43) BnH 12091 | 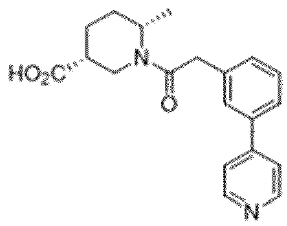 | (44) BnH 12092 | 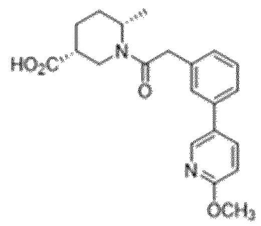 |
| (45) BnH 12093 | 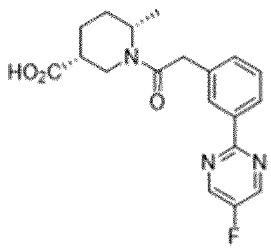 | (46) BnH 12095 | 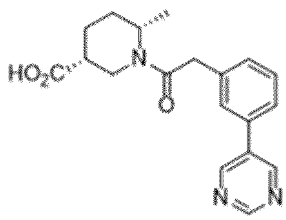 |
| (47) BnH 12097 | 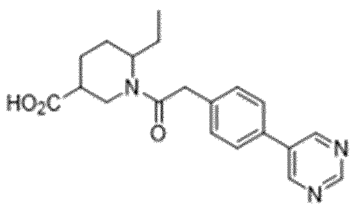 | (48) BnH 12098 | 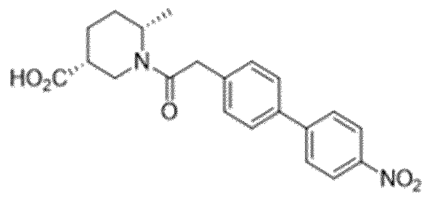 |

In an example, the compounds and compositions described herein may be inhibitors of HDAC. Preferably, the HDAC is HDAC4.

In another aspect, the compounds and compositions described herein are synaptic plasticity modulators. Preferably, the synaptic plasticity is synaptic plasticity in the barrel cortex (for example, layer 4) of the brain.

**III. Pharmaceutical composition**

In general, the compounds described herein may be formulated into pharmaceutical compositions using techniques well known to those skilled in the art. The concentration of active compound in the pharmaceutical composition will vary depending on absorption, inactivation and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once or may be divided into a number of smaller doses to be administered at varying intervals of time.

The concentration of inhibitor which reduces the activity of an HDAC to 50% of that of the uninhibited HDAC is determined as the $IC_{50}$ value. In some embodiments, such reduction of HDAC is at least 50%, such as at least about 75%, for example, at least about 90%. In some embodiments, HDAC activity is reduced by at least 95%, such as by at least 99%. In some embodiments, the compounds described herein have an $IC_{50}$ value less than 100 nanomolar. In some embodiments, the compounds described herein have an $IC_{50}$ value from 100 nanomolar to 1 micromolar. In some embodiments, the compounds described herein have an $IC_{50}$ value from 1 to 25 micromolar.

In some embodiments, such inhibition is specific, that is, the histone deacetylase inhibitor reduces the ability of a histone deacetylase to remove an acetyl group from a protein at a concentration that is lower than the concentration of the inhibitor that is required to produce another, unrelated biological effect. In some embodiments, the concentration of the inhibitor required for histone deacetylase inhibitory activity is at least 2-fold lower, such as at least 5-fold lower, for example, at least 10-fold lower, such as at least 20-fold lower than the concentration required to produce an unrelated biological effect.

In some embodiments, the pharmaceutical composition comprises about 25 mg, about 50 mg, about 75 mg, about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, about 1000 mg, about 1250 mg, about 1500 mg, about 1750 mg, about 2000 mg, about 2250 mg, about 2500 mg, about 2750 mg, or about 3000 mg of the compound.

In some embodiments, the pharmaceutical composition comprises about 0.01 mg/kg to about 125 mg/kg and preferably from about 1 mg/kg to about 50 mg/kg of subject body weight per day, one or more times a day, to obtain the desired therapeutic effect.

In certain embodiments, the composition comprises, for example, 0.0001 to 99.9 wt %, preferably 0.001 to 50 wt % of the compound based on the total composition weight. The content ratio is a value based on the dry amount from which the solvent is removed.

In certain embodiments, the composition comprises a 5% (w/v), 10% (w/v), 15% (w/v), 20% (w/v), 25% (w/v), 30% (w/v), 35% (w/v) or 40% (w/v) aqueous solution of one or more compounds. In some embodiments, the effective amount of the composition comprises a 25% (w/v) solution of one or more compounds.

In certain embodiments, the pharmaceutical compositions of the present disclosure further comprise one or more pharmaceutically acceptable excipients. In some embodiments, the one or more pharmaceutically acceptable excipients are selected from the group comprising a diluent, a buffering agent, a preservative, a stabilizer, a solubilizing agent or any combination thereof.

In certain embodiments, the pharmaceutical composition may be formulated for administration as a liquid dosage form suitable for intracavitary, intradermal, intramuscular, intrathecal, intravenous, subcutaneous, or intracerebroventricular administration.

In certain embodiments, a liquid dosage form of a pharmaceutical composition as described herein further comprises a diluent. In some embodiments, the insert diluent is a saline solution.

In certain embodiments, a liquid dosage form of a pharmaceutical composition as described herein further comprises a buffering agent. For example, suitable buffering agents for use with the present disclosure include, but are not limited to, both organic and inorganic acids and salts thereof, such as citrate buffers (for example, monosodium citrate-disodium citrate mixture, citric acid-trisodium citrate mixture, citric acid-monosodium citrate mixture), succinate buffers (for example, succinic acid-monosodium succinate mixture, succinic acid-sodium hydroxide mixture, succinic acid-disodium succinate mixture), tartrate buffers (for example, tartaric acid-sodium tartrate mixture, tartaric acid-potassium tartrate mixture, tartaric acid-sodium hydroxide mixture), fumarate buffers (for example, fumaric acid-monosodium fumarate mixture, fumaric acid-disodium fumarate mixture, monosodium fumarate-disodium fumarate mixture), gluconate buffers (for example, gluconic acid-sodium gluconate mixture, gluconic acid-sodium hydroxide mixture, gluconic acid-potassium gluconate mixture), oxalate buffer (for example, oxalic acid-sodium oxalate mixture, oxalic acid-sodium hydroxide mixture, oxalic acid-potassium oxalate mixture, and the like), lactate buffers (for example, lactic acid-sodium lactate mixture, lactic acid-sodium hydroxide mixture, lactic acid-potassium lactate mixture, and the like), and acetate buffers (for example, acetic acid-sodium acetate mixture, acetic acid-sodium hydroxide mixture, and the like). Additionally, phosphate buffers, histidine buffers and trimethylamine salts such as Tris may be used.

In certain embodiments, a liquid dosage form of a pharmaceutical composition as described herein further comprises a preservative. For example, suitable preservatives for use with the present disclosure include, but are not limited to phenol, benzyl alcohol, meta-cresol, methyl paraben, propyl paraben, octadecyldimethylbenzyl ammonium chloride, benzalconium halides (for example, chloride, bromide, and iodide), hexamethonium chloride, and alkyl parabens (for example, methyl or propyl paraben, catechol, resorcinol, cyclohexanol, and 3-pentanol).

In certain embodiments, a liquid dosage form of a pharmaceutical composition as described herein further comprises a stabilizer. For example, suitable stabilizers include, but are not limited to, polyhydric sugar alcohols, trihydric or higher sugar alcohols, amino acids, organic sugars or sugar alcohols, polyvinylpyrrolidone monosaccharides, trisaccacharides, polysaccharides, proteins, sulfur containing reducing agents, amino acid polymers, and polyethylene glycol.

In certain embodiments, a liquid dosage form of a pharmaceutical composition as described herein further comprises a solubilizing agent. In some embodiments, the solubilizing agent is an ionic surfactant. Examples of non-ionic surfactants include, but are not limited to, polysorbates, polyoxamers, pluronic polyols, and polyoxyethylene sorbitan monoethers.

In certain embodiments, pharmaceutical compositions disclosed herein may be prepared for storage as lyophilized formulations or aqueous solutions by mixing the composition with optional pharmaceutically acceptable carriers, excipients or stabilizers typically employed in the art (for example, buffering agents, stabilizing agents, preservatives, isotonifiers, non-ionic detergents, antioxidants, and other miscellaneous additives).

In a case where the compound is administered orally, it may be formulated into pills, capsules, cachets, tablets or the like, together with pharmaceutically acceptable excipients.

Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom. Tablets, troches, lozenges, aqueous or oil suspensions, dispersible powders or granules, emulsions, hard or soft capsules, for example, gelatin capsules, syrups or elixirs may be prepared for oral use. Formulations of the compounds of the present disclosure intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient are in admixture with nontoxic pharmaceutically acceptable excipient which are suitable for manufacture of tablets. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid, or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

In certain embodiments, the pharmaceutical compositions of the present disclosure are administered to the subject by intracavitary, intradermal, intramuscular, intrathecal, intravenous, subcutaneous, or intracerebroventricular administration.

## IV. Biological assay

Described herein are methods of selecting compounds useful for the present disclosure.

In an aspect, compounds for the present disclosure are selected for their ability to interact with an HDAC. In an embodiment, the HDAC is HDAC4. In another embodiment, binding of the compounds to the HDAC is measured by surface plasmon resonance assay. In an embodiment, the compound is BnH12003.

In an aspect, compounds are selected for their ability to increase the expression of BDNF and/or GluN2B in the targeted neurons. In an aspect, compounds are selected for their ability to increase the expression of BDNF in the targeted neurons. In another embodiment, compounds are selected for their ability to increase the expression of GluN2B in the targeted neurons. In an embodiment, RT-PCR is used to measure the level of expressions. In an embodiment, the compound is BnH12003, BnH12017, BnH12045, BnH12046, BnH12047, BnH12048, BnH12051, BnH12053, BnH12057, BnH12058, BnH12064, BnH12066, BnH12067, BnH12071, BnH12072, BnH12078, BnH12085, BnH12086, BnH12088, BnH12089, BnH12090, BnH12091, BnH12092, BnH12093, BnH12095, BnH12097, or BnH12098. In another embodiment, the compound is BnH12003, BnH12046, BnH12057, BnH12067, BnH12091, BnH12092, BnH12095, BnH12097, or BnH12098.

In an aspect, compounds for the present disclosure are selected for their non-toxicity toward human cells. In an embodiment, compounds of the present disclosure are selected for not significantly affecting the cell viability of human cell line SH-SY5Y. In another embodiment, compounds for the present disclosure are selected for not significantly affecting the cell viability of human cell line 293T. In an embodiment, the compounds do not lower cell viabilities of SH-SY5Y or 293T at a concentration of about 0.1, 1, 10, 100 $\mu$M. In an embodiment, the compound may be one selected from the compounds described in Table 4. Preferably, the compound is BnH12003.

In an aspect, compounds for the present disclosure are selected for their ability to increase the potency, when measured via TC EPSC potency in the rodent barrel cortex (layer 4). In an embodiment, the compounds increase the measured potency at a concentration of about 0.1 to about 0.5 $\mu$M, when delivered by Os pump through ventricle injection. In another embodiment, the concentration may be about 0.1 to about 3 $\mu$M. In another embodiment, the concentration may be about 3 $\mu$M. In another embodiment, the concentration is about 0.03, 0.1, 0.3, 1, 3, or 10 mg/kg. In an embodiment, the compound is BnH12003. In another embodiment, the compound is delivered via intraperitoneal injection, wherein the

compound is BnH12003. In yet another embodiment, the intraperitoneal injection delivers about 25 mg/kg of the compound.

In an aspect, the compound of the present disclosure is selected for its ability to increase the I/O slope ($ms^{-1}$) of field excitatory postsynaptic potential (fEPSP) in the hippocampus of a rodent model of Alzheimer's disease, alone or in combination with a monoclonal antibody to amyloid beta peptide. In an embodiment, the compound increases the I/O slope in a case of being administered together with a monoclonal antibody to amyloid beta peptide via an Os pump at a rate of about 3 μM and 200 μg for the compound and the antibody, respectively. In another embodiment, the monoclonal antibody is solanezumab. In yet another embodiment, the compounds are BnH12003 and solanezumab.

In an aspect, the compound of the present disclosure is selected for its ability to increase LTP of the fEPSP slope in the hippocampus of a rodent model of Alzheimer's disease in combination with a monoclonal antibody to amyloid beta peptide. In an embodiment, the compound increases LTP of the fEPSP slope in a case of being administered together with a monoclonal antibody to amyloid beta peptide via an Os pump at a rate of about 3 μM and 200 μg for the compound and the antibody, respectively. In another embodiment, the monoclonal antibody is solanezumab. In yet another embodiment, the compound is BnH12003.

In an aspect, the compound of the present disclosure is selected for its ability to increase potency as measured via TC EPSC potency in the barrel cortex (layer 4) of a rodent model of Alzheimer's disease. In an embodiment, the compound increases potency as measured at a concentration of about 3 μM in a case of being delivered by an Os pump via intraventricular infusion. In another embodiment, the compound increases TC EPSC potency in a case of being delivered together with a monoclonal antibody to amyloid beta peptide. In yet another embodiment, the monoclonal antibody is solanezumab. In still yet another embodiment, solanezumab is delivered at a concentration of about 200 μg. In still yet another embodiment, the compound is BnH12003.

In an aspect, the compound of the present disclosure is selected for its ability to decrease expression of HDAC4 in the rodent barrel cortex (layer 4). In an embodiment, the compound is BnH12003.

In an aspect, the compound of the present disclosure is selected for its ability to decrease symptoms of post-traumatic stress disorder. In an embodiment, the selection is performed by measuring the freezing percentage of a rodent model of post-traumatic stress disorder in which the rodent has been conditioned to freeze in response to an environmental cue. In an embodiment, the compound is BnH12003.

Described herein are detailed examples of methods useful for selecting the compounds of the present disclosure.

## V. Therapeutic uses of compounds

The compounds of the present disclosure may be useful for inhibiting at least one type of HDAC. In an aspect, the compounds described herein may be therapeutically useful, as certain HDACs are known to be involved in biological pathways governing higher-order brain functions. The compounds described herein may therefore be useful when brain pathologies are associated with or mediated by HDACs.

Recent reports have detailed the importance of histone acetylation in central nervous system (CNS) functions such as neuronal differentiation, memory formation, drug addiction, and depression (Citrome, Psychopharmacol. Bull. 2003, 37, Suppl. 2, 74-88; Johannessen, CNS Drug Rev. 2003, 9, 199-216; Tsankova et al, 2006, Nat. Neurosci. 9, 519-525).

A neurological disorder is a condition having as a component a central or peripheral nervous system malfunction. Neurological disorders may cause a disturbance in the structure or function of the nervous system resulting from developmental abnormalities, disease, genetic defects, injury or toxin. These disorders may affect the central nervous system (for example, the brain, brainstem and cerebellum), the peripheral nervous system (for example, the cranial nerves, spinal nerves, and sympathetic and parasympathetic nervous systems) and/or the autonomic nervous system (for example, the part of the nervous system that regulates involuntary action and that is divided into the sympathetic and parasympathetic nervous systems).

Accordingly, provided herein is a method of treating or preventing a neurological disorder or neurodegenerative disease mediated by HDAC in a subject in need of such a treatment or prevention, comprising administering to the subject a therapeutically effective amount of at least one compound described herein. In some embodiments, the compounds described herein may be useful for modulating the activities of Class IIa HDACs. In some embodiments, the compound is an inhibitor of HDAC4.

Most neurodegenerative disorders are caused by the degeneration of the neurons in the brain. Neurons containing HDAC4 are widely distributed in the brain (Mielcarek et al., 2013, PLoSBiol. 11:e1001717; Takase etal., 2013, PloS ONE 8:e58473). Treatments as the present disclosure that modulate the activities of HDAC4 in the brain could, therefore, be useful for any neurodegenerative disease of the brain since the mechanism of intervention contemplated by the present disclosure can target HDAC4 in the neuron regardless of the observed pathology of the brain. There can be no neurodegenerative disease that the compounds of the present disclosure would have no utility. The compounds of the present disclosure are not bound by the current understanding of the molecular mechanisms involving HDAC4 in the

neurons of the brain. As such, the compounds of the present disclosure may be useful for any chronic or acute neurodegenerative disorders, so long as the neurons affected by the pathological manifestation of the disease express HDAC4.

Examples of neurodegenerative disorders include: chronic neurodegenerative diseases such as familial and sporadic amyotrophic lateral sclerosis (FALS and ALS, respectively), familial and sporadic Parkinson's disease, Huntington's disease, familial and sporadic Alzheimer's disease, multiple sclerosis, muscular dystrophy, olivopontocerebellar atrophy, multiple system atrophy, Wilson's disease, progressive supranuclear palsy, diffuse Lewy body disease, corticodentatonigral degeneration, progressive familial myoclonic epilepsy, striatonigral degeneration, torsion dystonia, familial tremor, Down's Syndrome, Gilles de la Tourette syndrome, Hallervorden-Spatz disease, diabetic peripheral neuropathy, dementia pugilistica, AIDS dementia, age related dementia, age associated memory impairment, and amyloidosis-related neurodegenerative diseases such as those caused by the prion protein (PrP) which is associated with transmissible spongiform encephalopathy (Creutzfeldt-Jakob disease, Gerstmann-Straussler-Scheinker syndrome, scrapie, and kuru), and those caused by excess cystatin C accumulation (hereditary cystatin C angiopathy). Examples of acute neurodegenerative disorders include: traumatic brain injury (for example, surgery- related brain injury), cerebral edema, peripheral nerve damage, spinal cord injury, Leigh's disease, Guillain-Barre syndrome, lysosomal storage disorders such as lipofuscinosis, Alper's disease, restless leg syndrome, vertigo as result of CNS degeneration; pathologies arising with chronic alcohol or drug abuse including, for example, the degeneration of neurons in locus coeruleus and cerebellum, drug-induced movement disorders; pathologies arising with aging including degeneration of cerebellar neurons and cortical neurons leading to cognitive and motor impairments; and pathologies arising with chronic amphetamine abuse to including degeneration of basal ganglia neurons leading to motor impairments; pathological changes resulting from focal trauma such as stroke, focal ischemia, vascular insufficiency, hypoxic-ischemic encephalopathy, hyperglycemia, hypoglycemia or direct trauma; pathologies arising as a negative side-effect of therapeutic drugs and treatments (for example, degeneration of cingulate and entorhinal cortex neurons in response to anticonvulsant doses of antagonists of the NMD A class of glutamate receptor) and Wernicke-Korsakoff's related dementia.

In addition to acute or chronic degenerative disorders, the compounds of the present disclosure may be useful for treating other types of diseases of the brain, so long as the affected neurons are regulated in part by HDAC4. Examples of these conditions include: neuropsychiatric disorders related to cognition, dementia, social behavior, anxiety, or other personality disorders, for example, schizophrenia, delirium, attention deficit disorder (ADD), bipolar disorders, obsessive-compulsive disorders, or eating disorders; fear and anxiety disorders, for example, panic disorder, various phobias, post-traumatic stress disorder (PTSD), psychogenic erectile dysfunction, or insomnia; various forms of addictions; and mood disorders such as depression.

The types of diseases that could benefit from the use of the compounds described herein are not limited to the diseases of the brain. Any neurological diseases in which the affected neurons are regulated by HDAC4 could potentially benefit from the compounds of the present disclosure. As described herein, Alzheimer's disease and PTSD, though vastly different in their pathology and the known causes of the diseases, benefited from the treatment of compounds described herein. In the case of PTSD, BnH12003 liberated such rodents from trauma at a significantly faster rate than untreated fear-conditioned rodents. Examples of neurological diseases that can benefit from the compounds of the present disclosure include: diseases of the peripheral nervous system due to degeneration or injury, for example, ataxia or trauma; and an acute or chronic degenerative disease of the eye, for example, glaucoma.

Memory loss or reduced cognitive function can also benefit from a treatment utilizing compounds of the present disclosure, so long as the affected neuron's plasticity is regulated in part by HDAC4. Examples of these disorders include: conditions related to cognitive functions such as memory loss/impairment, acts of creativity, problem-solving, and possibly intuition.

Memories include short-term memory (also referred to as working or recent memory) and long-term memory. Short-term memories involve recent events, while long-term memories relate to the recall of events of the more distant past. Methods of assessing the ability to recall a memory are known to those of skill in the art and may involve routine cognitive tests.

Age related memory loss is characterized by objective loss of memory in an older subject compared to his or her younger years, but cognitive test performance that is within normal limits for the subject's age. Age related memory loss subjects score within a normal range on standardized diagnostic tests for dementias, as set forth by the DSM-IV. Moreover, the DSM-IV provides separate diagnostic criteria for a condition termed Age-Related Cognitive Decline. Age-related memory loss may include decreased brain weight, gyral atrophy, ventricular dilation, and selective loss of neurons within different brain regions. For purposes of some embodiments of the present disclosure, more progressive forms of memory loss are also included under the definition of age-related memory disorder. Thus, persons having greater than age-normal memory loss and cognitive impairment, yet scoring below the diagnostic threshold for frank dementia, may be referred to as having a mild neurocognitive disorder, mild cognitive impairment, late-life forgetfulness, benign senescent forgetfulness, incipient dementia, provisional dementia, and the like. Such subjects may be slightly more susceptible to developing frank dementia in later life (see also US patent application 2006/008517).

Symptoms associated with age-related memory loss include but are not limited to alterations in biochemical markers associated with the aging brain, such as IL-1 beta, IFN-gamma, p-JNK, p-ERK, reduction in synaptic activity or function, such as synaptic plasticity, evidenced by reduction in long term potentiation, diminution of memory and learning. In some embodiments, the compounds of the present disclosure modulate the activities of IL-1 beta, IFN-gamma, p-JNK, or p-ERK.

Methods for enhancing memories may include reestablishing access to memories as well as recapturing memories. The term reestablishing access as used herein refers to increasing retrieval of a memory. While not bound by a mechanism of action, it is believed that the compounds of the disclosure are effective in increasing retrieval of memories by re-establishing a synaptic network. The process of reestablishing a synaptic network may include an increase in the number of active brain synapses and or a reversal of neuronal loss.

In some instances, learning is referred to as memory. Learning, unlike memory enhancement, refers to the ability to create new memories that had not previously existed. In some embodiments, in order to test the ability of a compound to influence the ability of a subject to learn rather than recall old memories, at least one compound of the present disclosure is administered prior to or at the same time as the memory is created. In some embodiments, to test the ability of a compound to affect recall of a previously created memory, at least one of the compounds described herein is administered after the memory is created and preferably after the memory is lost.

Also provided is a method of enhancing learning and memory formation in a subject suffering from memory loss comprising administering to the subject an effective amount of at least one compound described herein. The method may also be equally useful for enhancing learning and memory formation in a normal subject.

Neurogenesis, or the birth of new neuronal cells, was thought to occur only in developing organisms. However, recent research has demonstrated that neurogenesis continues into and throughout adult life. On-going neurogenesis is thought to be an important mechanism underlying neuronal plasticity, enabling organisms to adapt to environmental changes and influencing learning and memory throughout life. In an aspect, the disclosure includes a method of increasing synaptic density in a subject comprising administering to the subject in need of such increase a compound of the disclosure or a pharmaceutically acceptable salt, hydrate, solvate, or prodrug thereof. In an aspect, the disclosure includes a method of increasing synaptic plasticity in a subject comprising administering to the subject in need of such increase a compound of the disclosure or a pharmaceutically acceptable salt, hydrate, solvate, or prodrug thereof. In an aspect, the disclosure includes a method of increasing dendritic density in neurons in a subject comprising administering to the subject in need of such increase a compound of the disclosure or a pharmaceutically acceptable salt, hydrate, solvate, or prodrug thereof.

The present disclosure provides methods for enhancing memory in a subject having a memory disorder. Examples of types of memory disorders include Alzheimer’s disease, absent-minded professor, absent-mindedness, amnesia, anterograde amnesia, blackout (alcohol-related amnesia), bromism, childhood amnesia, false memory syndrome, fugue state, hyperthymesia, Korsakoff s syndrome, lacunar amnesia, memory distrust syndrome, memory loss, post-traumatic amnesia, prosopamnesia, psychogenic amnesia, repressed memory, retrograde amnesia, Ribot’ s Law, selective memory loss, source amnesia, sourcemonitoring error, the seven sins of memory, tip of the tongue, transient epileptic amnesia, transient global amnesia, and twilight sleep.

In an embodiment, the memory disorder is Alzheimer’s disease. Such methods optionally involve administering the inhibitor and monitoring the subject to identify recapture of a memory that was previously lost. Subjects may be monitored by routine tests known in the art.

In other embodiments the Alzheimer’s subject is one that has late-stage Alzheimer’s disease. Many of the compounds suggested for treating Alzheimer’s disease are designed to treat the early stages of the disease by preventing plaque buildup. The compounds of the disclosure are useful for treating both early stages and late stages of dementia because they improve memory and cognition rather than preventing only plaque accumulation.

In an embodiment, a condition or disorder mediated by HDAC is cognitive function disorders/impairments. Accordingly, also provided is a method of promoting cognitive function in a subject suffering from cognitive function disorders/impairments comprising administering to the subject an effective amount of at least one compound described herein. The present disclosure also provides a method of promoting cognitive function in a normal subj ect.

In some cases, cognitive function is reduced by about 5%, about 10%, about 30%, or more, compared to cognitive function measured in an age-matched normal subject. Cognitive function may be promoted to any detectable degree, but in humans preferably is promoted sufficiently to allow an impaired subject to carry out daily activities of normal life.

Cognitive function may be assessed, and thus optionally defined, via one or more tests or assays for cognitive function. Non-limiting examples of a test or assay for cognitive function include CANTAB (see for example Fray et al. "CANTAB battery: proposed utility in neurotoxicology. "Neurotoxicol Teratol 1996; 18(4):499-504), Stroop Test, Trail Making, Wechsler Digit Span, or the CogState computerized cognitive test (see also Dehaene et al. "Reward-dependent learning in neuronal networks for planning and decision making. " Brain Res. 2000; 126:21729; Iverson et al. "Interpreting change on the WAIS-III/ WMS-I11 in clinical samples." Arch Clin Neuropsychol. 2001; 16(2):183- 91; and Weaver et al. "Mild memory impairment in healthy older adults is distinct from normal aging." Cogn. 2006;60(2): 146-55). The methods of

the disclosure may be used to promote cognitive function in a normal subject or to treat, alleviate and/or prevent a subject from having a cognitive dysfunction. A normal subject, as used herein, is a subject that has not been diagnosed with a disorder associated with impaired cognitive function.

In some embodiments, the cognitive function disorders or impairments are associated with, but not limited to, Alzheimer's disease, Huntington's disease, seizure induced memory loss, schizophrenia, Rubinstein Taybi syndrome, Rett Syndrome, Fragile X, Lewy body dementia, vascular dementia, bipolar disorder and social, cognitive and learning disorders associated with autism spectrum disorders (ASD), attention deficit hyperactivity disorder (ADHD), dyslexia, learning disorders, traumatic head injury, stroke induced cognitive and motor impairment, traumatic brain injury, neurodegeneration and neuronal loss mediated cognitive impairment, and attention deficit disorder.

In some embodiments, the cognitive function disorders or impairments are associated with, but not limited to, anxiety disorders, conditioned fear response, panic disorders, obsessive compulsive disorders, post-traumatic stress disorder, phobias, social anxiety disorders, substance dependence recovery or Age Associated Memory Impairment (AAMI), and Age Related Cognitive Decline (ARCD).

It has been demonstrated that administration of the HDAC inhibitors sodium butyrate or Trichostatin A facilitates fear extinction in mice and this enhancement mirrors that caused by commonly used behavioral manipulation and is consistent with other studies demonstrating a role for the hippocampus in the extinction of contextual fear (Lattal, et al., 2007, Behav. Neurosci. 121, 5, 1125-1131). In an embodiment, a condition or disorder mediated by HDAC is extinction learning disorders, for example, a fear extinction deficit. Accordingly, also provided is a method of treating or preventing extinction learning disorders in a subject suffering from extinction learning disorders comprising administering to the subject an effective amount of at least one compound described herein.

The compounds of the disclosure may be used to facilitate the psychological process of extinction learning and thus are useful for treating, alleviating, and/or preventing neuropsychiatric disorders and other related disorders. Unlike traditional anti-anxiety drugs that are administered on a chronic basis and address physiological symptoms of anxiety, the compounds of the disclosure may be used on a chronic or acute basis in conjunction with a second therapy, for example, psychotherapy.

Although not thoroughly explored in the present disclosure, HDAC4 mediates gene expression in other cell types, including the immune cells. The compounds of the present disclosure are not bound by the current understanding of the molecular mechanisms involving HDAC4 in immune cells, or in other types of cells found in cancer. As such, the compounds of the present disclosure may be useful for any diseases including: hyperproliferative diseases, for example, malignant tumors of various origins; diseases of the immune system, for example, autoimmune disease, inflammation, or allergies; infectious disease; graft rejection; and fibrotic disease.

In some embodiments, the condition or disorder mediated by HDAC is an immune disorder that may be co-treated with TNF$\alpha$ or other immune modulators, upon administering to the subject a therapeutically effective amount of at least one compound as described herein. Accordingly, also provided is a method of treating an immune disorder that is mediated by HDAC in a subject in need of such a treatment, comprising administering to the subject a therapeutically effective amount of at least one compound described herein, either before, during, or after a treatment with TNF$\alpha$ or other immune modulators.

In some embodiments, the condition or disorder mediated by HDAC comprises a disorder that may also be treated by progenitor/stem cell based therapies such as: disorders related to diabetes (organ failure, cirrhosis, and hepatitis); central nervous system (CNS) disorders associated with dysregulation of progenitor cells in the brain (for example, PTSD); tumors (for example, retinoblastomas); disorders affecting oligodendrocyte progenitor cells (for example, astrocytomas and ependymal cell tumors); multiple sclerosis; demyelinating disorders such as leukodystrophies; neuropathies associated with white matter loss; cerebellar disorders such as ataxia; and olfactory progenitor disorders (for example, anosmic conditions). Accordingly, also provided is a method of treating a disorder that is mediated by HDAC in a subject in need of such a treatment, comprising administering to the subject a therapeutically effective amount of at least one compound described herein, either before, during, or after a treatment with progenitor/stem cell based therapies.

In some embodiments, the condition or disorder mediated by HDAC comprises a blood pressure disorder related to nitric oxide (NO) regulation (for example, hypertension, erectile dysfunction, asthma; and ocular disorders as glaucoma). Accordingly, also provided is a method of treating a blood pressure disorder related to nitric oxide (NO) regulation that is mediated by HDAC in a subject in need of such a treatment, comprising administering to the subject a therapeutically effective amount of at least one compound described herein.

In some embodiments, the condition or disorder is a cardiac hypertrophic disorder. Accordingly, also provided is a method of treating a cardiac hypertrophic disorder that is mediated by HDAC in a subject in need of such a treatment, comprising administering to the subject a therapeutically effective amount of at least one compound described herein.

In some embodiments, the condition or disorder mediated by HDAC comprises a hematological disorder such as thalassemia, anemia, and sickle cell anemia. Accordingly, also provided is a method of treating a hematological disorder mediated by HDAC in a subject in need of such a treatment, comprising administering to the subject a therapeutically

effective amount of at least one compound described herein.

In some embodiments, the condition or disorder mediated by HDAC comprises a metabolic disease such as prediabetes or diabetes (type I or II). Accordingly, also provided is a method of treating a metabolic disease, such as prediabetes or diabetes (type I or II), mediated by HDAC in a subject in need of such a treatment, comprising administering to the subject a therapeutically effective amount of at least one compound described herein.

In an aspect, the present disclosure provides pharmaceutical compositions further comprising a second therapeutic agent for the treatment of condition or disorder mediated by HDAC in a subject suffering from the condition or disorder mediated by HDAC. In some embodiments, the second therapeutic agent is indicated to treat condition or disorder mediated by HDAC, preferably HDAC4.

Also provided are methods of treatment of condition or disorder mediated by HDAC in a subject suffering from the condition or disorder mediated by HDAC in which at least one compound, or pharmaceutically acceptable salt thereof described herein is given to the subject in combination with one or more additional active agents.

In some embodiments, the second therapeutic agent is selected from the group consisting of donepezil, rivastigmine, galantamine, memantine, verubecestat, solanezumab, bapineuzumab, aducanumab, lecanemab, tideglusib, epothilone D, and ABBV-8E12.

In some embodiments, the second therapeutic agent is selected from the group consisting of a cholinesterase inhibitor, an NMDA receptor antagonist, a humanized antibody which targets tau protein, a humanized antibody which targets amyloid beta protein, and a BACE inhibitor.

Typical treatments encompassed by the present disclosure include combination therapies. For instance, the combination therapy may be a pharmacotherapy (that is, a compound of the disclosure) and a behavioral therapy. Behavioral therapy comprises, but is not limited to, electroconvulsive seizure therapy, exercise, group therapy, talk therapy, or conditioning. In another embodiment, the behavioral therapy is cognitive-behavioral therapy. Examples of behavioral therapy that may be used in the ongoing methods are described, for example, in Cognitive-Behavioral Therapies by K. Dobson, ed., Guilford Publications, Inc., 2002; The new Handbook of Cognitive Therapy: Basics and Beyond by Judith S.S. Beck, Guilford Publications, Inc. 1995, which are herein incorporated by reference in their entireties. Any pharmaceutical active ingredient that is recognized by the skilled artisan as being a pharmacologic agent that enhances learning or conditioning can be used in the methods of the present disclosure. For example, one such class of pharmaceutical active ingredients contemplated herein comprises compounds that increase the level of norepinephrine in the brain. Such compounds include those acting as norepinephrine reuptake inhibitors, for example tomoxetine, reboxetine, duloxetine, venlafaxine, and milnacipran, and those compounds that cause release of norepinephrine, for example amphetamine, dextroamphetamine, pemoline, and methylphenidate. Another class of such pharmaceutical active ingredients is those compounds that increase the level of acetylcholine in the brain, including, for example, compounds that block its breakdown. Examples of such compounds include, but are not limited to, donepezil HC1 or Aricept™ and tacrine, which inhibit cholinesterase activity.

The methods for combination therapy may comprise subjecting the subject to one or more sessions of a combination therapy protocol, where the combination therapy protocol comprises an acute administration of a therapeutically effective amount of a compound of the disclosure that enhances learning or conditioning in combination with a session of psychotherapy. In an aspect, the exposure to the compound occurs within about 24 hours prior to initiating the session of psychotherapy, preferably within about 12 hours, and more preferably within about 6 hours prior to initiating the session of psychotherapy. A full course of treatment for the neuropsychiatric disorder entails at least one session of this combination therapy protocol.

Hereinafter, the present disclosure will be described in more detail by the following examples. The following examples are presented to aid in the understanding of the present disclosure, and are not intended to limit the scope thereof in any way, and should not be construed as limiting.

Methods for obtaining the compounds described herein or pharmaceutically acceptable salts thereof will be apparent to those skilled in the art, and suitable procedures are described, for example, in the preparation examples below and in the references cited herein.

## VI. Method for producing active compound

Methods of synthesizing the compounds of the present disclosure are provided herein. The names and structures of the specific compounds illustrated are presented in Tables 2 and 3.

[Table 2]

| No. | Compound name |
|---|---|
| (1) BnH12002 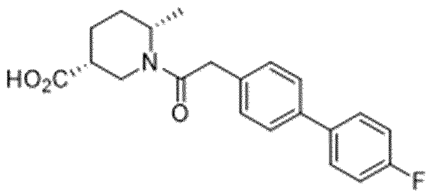 | (3R,6S)-1-(2-(4'-fluoro-[1,1-biphenyl]-4-yl)-6-methylpiperidine-3-carboxylic acid |
| (2) BnH12003 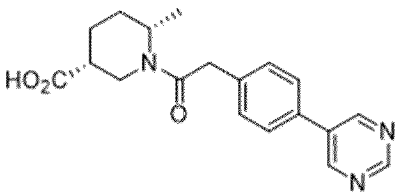 | (3R,6S)-6-methyl-1-(2-(4-(pyrimidin-5-yl)phenyl)acetyl)piperidine-3-carboxylic acid |
| (3) BnH12015 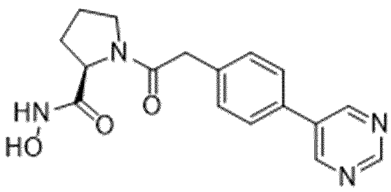 | (R)-N-hydroxy-1-(2-(4-(pyrimidin-5-yl)phenyl)acetyl)pyrrolidine-2-carboxamide |
| (4) BnH12017 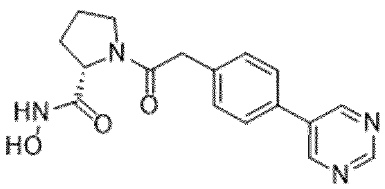 | (S)-N-hydroxy-1-(2-(4-(pyrimidin-5-yl)phenyl)acetyl)pyrrolidine-2-carboxamide |
| (5) BnH12036 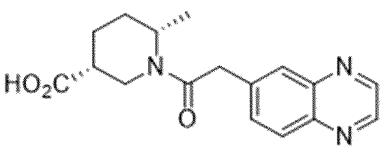 | (3R,6S)-6-methyl-1-(2-(quinoxalin-6-yl)acetyl)piperidine-3-carboxylic acid |
| (6) BnH12037 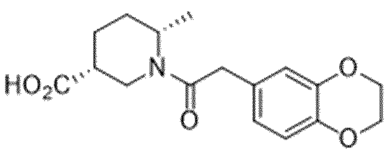 | (3R,6S)-1-(2-(2,3-dihydrobenzo[b][1,4]dioxin-6-yl)acetyl)-6-methylpiperidine-3-carboxylic acid |

(continued)

| No. | Compound name |
|---|---|
| (7) BnH12042 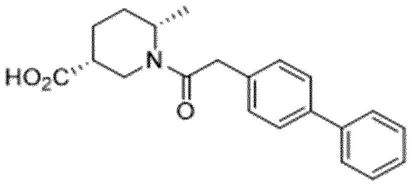 | (3R,6S)-1-(2-([1,1'-biphenyl]-4-yl)acetyl)-6-methylpiperidine-3-carboxylic acid |
| (8) BnH12043 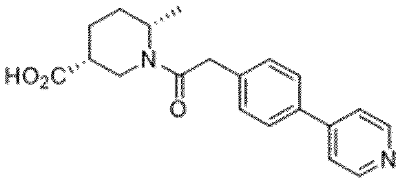 | (3R,6S)-6-methyl-1-(2-(4-(pyridin-4-yl)phenyl)acetyl)piperidine-3-carboxylic acid |
| (9) BnH12044 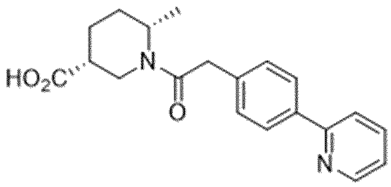 | (3R,6S)-6-mthyl-1-(2-(4-(pyridin-2-yl)phenyl)acetyl)piperidine-3-carboxylic acid |
| (10) BnH12045 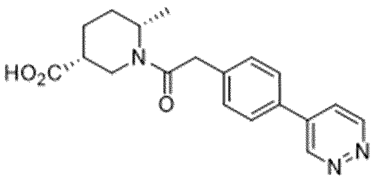 | (3R,6S)-6-methyl-1-(2-(4-(pyridazin-4-yl)phenyl)acetyl)piperidine-3-carboxylic acid |
| (11) BnH12046 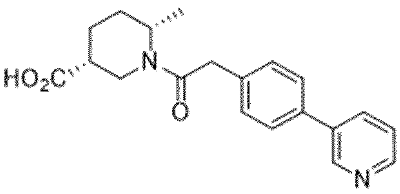 | (3R,6S)-6-methyl-1-(2-(4-(pyridin-3-yl)phenyl)acetyl)piperidine-3-carboxylic acid |
| (12) BnH12047 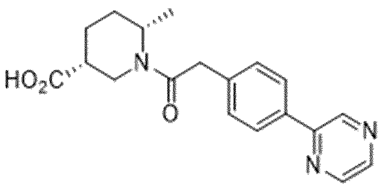 | (3R,6S)-6-methyl-1-(2-(4-(pyrazin-2-yl)phenyl)acetyl)piperidine-3-carboxylic acid |

(continued)

| No. | Compound name |
|---|---|
| (13) BnH12048 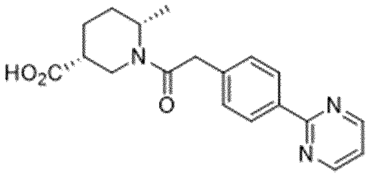 | (3R,6S)-6-methyl-1-(2-(4-(pyrimidin-2-yl)phenyl)acetyl)piperidine-3-carboxylic acid |
| (14) BnH12049 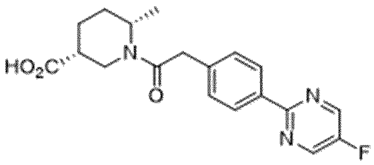 | (3R,6S)-1-(2-(4-(5-fluoropyrimidin-2-yl)phenyl)acetyl)-6-methylpiperidine-3-carboxylic acid |
| (15) BnH12050 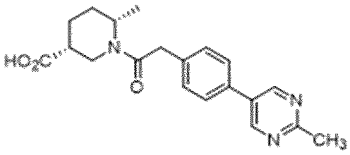 | (3R,6S)-6-methyl-1-(2-(4-(2-methylpyrimidin-5-yl)phenyl)acetyl)piperidine-3-carboxylic acid |
| (16) BnH12051 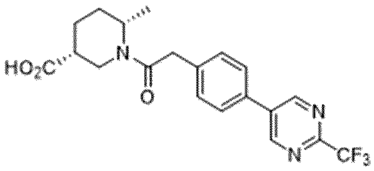 | (3R,6S)-6-methyl-1-(2-(4-(5-(trifluoromethyl)pyrimidin-2-yl)phenyl)acetyl)piperidine-3-carboxylic acid |
| (17) BnH12052 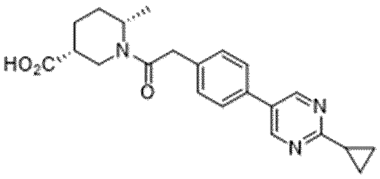 | (3R,6S)-1-(2-(4-(2-cyclopropylpyrimidin-5-yl)phenyl)acetyl)-6-methylpiperidine-3-carboxylic acid |
| (13) BnH12053 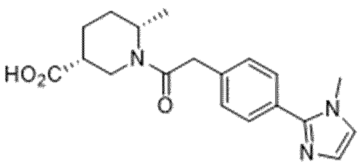 | Sodium (3R,6S)-6-methyl-1-(2-(4-(1-methyl-1H-imidazol-2-yl)phenyl)acetyl)piperidine-3-carboxylate |
| (19) BnH12054 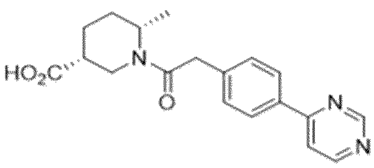 | (3R,6S)-6-methyl-1-(2-(4-(pyrimidin-4-yl)phenyl)acetyl)piperidine-3-carboxylic acid |

(continued)

| No. | Compound name |
|---|---|
| (20) BnH12055 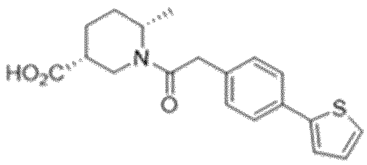 | (3R,6S)-6-methyl-1-(2-(4-(thiopeti-2-yl)phenyl)acetyl)piperidine-3-carboxylic acid |

[Table 3]

| | |
|---|---|
| (21) BnH12056 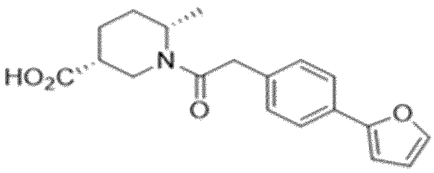 | (3R,6S)-1-(2-(4-(furan-2-yl)phenyl)acetyl)-6-methylpiperidine-3-carboxylic acid |
| (22) BnH12057 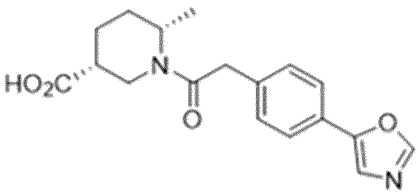 | (3R,6S)-6-methyl-1-(2-(4-(oxazol-5-yl)phenyl)acetyl)piperidine-3-carboxylic acid |
| (23) BnH12058 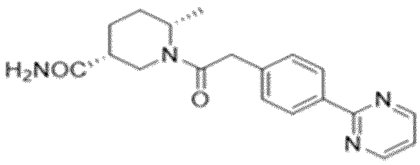 | (8R,6S)-6-methyl-1-(2-(4-(pyrimidin-2-yl)phenyl)acetyl)piperidine-3-carboxamide |
| (24) BnH12059 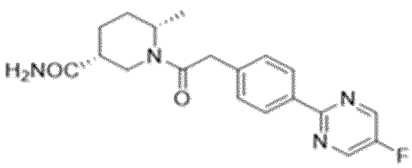 | (3R,6S)-1-(2-(4-(5-fluoropyrimidin-2-yl)phenyl)acetyl)-6-methylpiperidine-3-carboxamide |
| (25) BnH12064 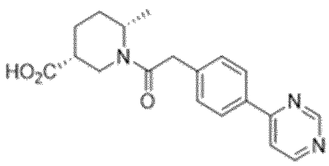 | (S)-1-(2-methylpiperidine-1-yl)-2-(4-(pyridin-3-yl)phenyl)ethan-1-one |
| (26) BnH12066 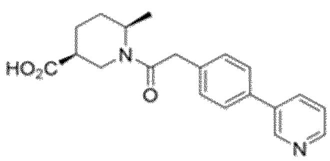 | (3S,6R)-6-methyl-1-(2-(4-(pyridin-3-yl)phenyl)acetyl)piperidine-3-carboxylic acid |

(continued)

| | |
|---|---|
| (27) BnH12067<br>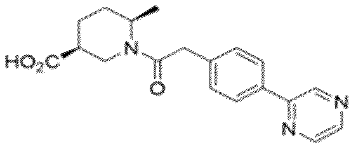 | (3S,6R)-6-methyl-1-(2-(4-(pyrazine-yl)phenyl)acetyl)piperidine-3-carboxylic acid |
| (28) BnH12071<br>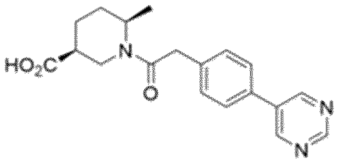 | (3S,6R)-6-methyl-1-(2-(4-(pyrimidin-5-yl)phenyl)acetyl)piperidine-3-carboxylic acid |
| (29) BnH12072<br>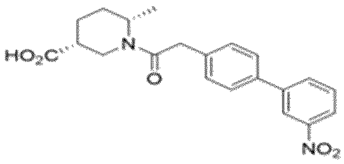 | (3R,6S)-6-methyl-1-(2-(3'-nitro-[1,1'-biphenyl]-4-yl)acetyl)piperidine-3-carboxylic acid |
| (30) BnH 12077<br>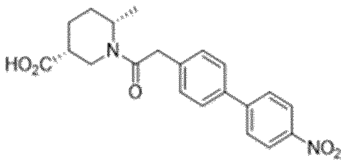 | (3R,6S)-6-methyl-1-(2-(4'-nitro-[1,1'-biphenyl]-4-yl)acetyl)piperidine-3-carboxylic acid |
| (31) BnH12078<br>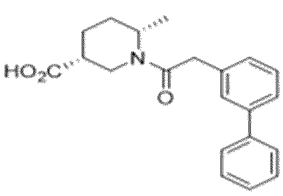 | (3R,6S)-1-(2-([1,1'-biphenyl]-3-yl)acetyl)-6-methylpiperidine-8-carboxylic acid |
| (32) BnH12030<br>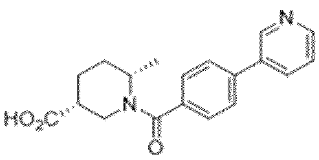 | (3R,6S)-6-methyl-1-(4-(pyridin-3-yl)benzoyl)piperidine-3-carboxylic acid |
| (33) BnH12031<br>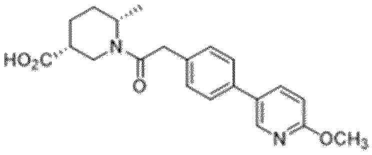 | (3R,6S)-1-(2-(4-(6-methoxypyridin-3-yl)phenyl)acetyl)-6-methylpiperidine-3-carboxylic acid |
| (34) BnH12032<br>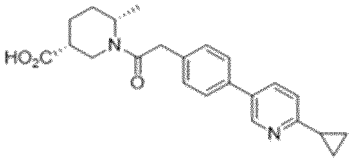 | (3R,6S)-1-(2-(4-(6-cyclopropylpyridin-3-yl)phenyl)acetyl)-6-methylpiperidine-3-carboxylic acid |

(continued)

| | |
|---|---|
| (35) BnH12083 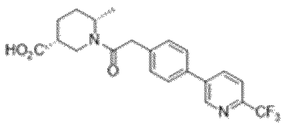 | (3R,6S)-6-methyl-1-(2-(4-(6-(trifluoromethyl)pyridin-3-yl)phenyl)acetyl)piperidine-3-carboxylic acid |
| (36) BnH12034 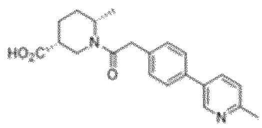 | (3R,6S)-6-methyl-1-(2-(4-(6-methylpyridin-3-yl)phenyl)acetyl)piperidine-3-carboxylic acid |
| (37) BnH12035 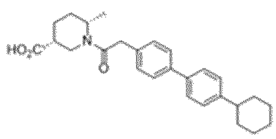 | (3R,6S)-1-(2-(4'-cyclohexyl-[1,1'-biphenyl]-4-yl)acetyl)-6-methylpiperidine-3-carboxylic acid |
| (38) BnH12086 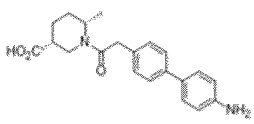 | (3R,6S)-1-(2-(4'-amino-[1,1'-biphenyl]-4-yl)acetyl)-6-methylpiperidine-3-carboxylic acid |
| (39) BnH12087 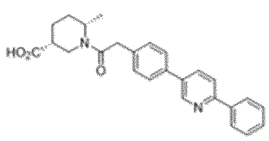 | (3R,6S)-6-methyl-1-(2-(4-(6-phenylpyridin-8-yl)phenyl)acetyl)piperidine-3-carboxylic acid |
| (40) BnH12088 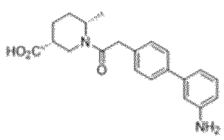 | (8R,6S)-1-(2-(8'-amino-[1,1'-biphenyl]-4-yl)acetyl)-6-methylpiperidine-3-carboxylic acid |
| (41) BnH12089 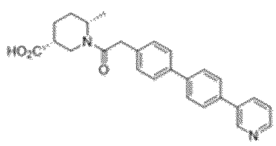 | (3R,6S)-6-methyl-1-(2-(4'-(pyridin-3-yl)-[1,1'-biphenyl]-4-yl)acetyl)piperidine-3-carboxylic acid |
| (42) BnH12090 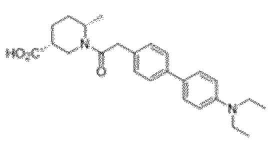 | (3R,6S)-1-(2-(4'-(diethylamino)-[1,1'-biphenyl]-4-yl)acetyl)-6-methylpiperidine-3-carboxylic acid |
| (43) BnH12091 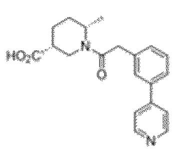 | (3R,6S)-6-methyl-1-(2-(3-(pyridin-4-yl)phenyl)acetyl)piperidine-3-carboxylic acid |

(continued)

| | |
|---|---|
| (44) BnH12092 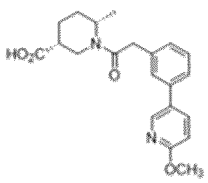 | (3R,6S)-1-(2-(3-(6-methoxypyridin-3-yl)phenyl)acetyl)-6-methylpiperidine-3-carboxylic acid |
| (45) BnH12093 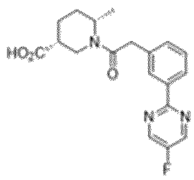 | (3R,6S)-1-(2-(3-(5-fluoropyrimidin-2-yl)phenyl)acetyl)-6-methylpiperidine-3-carboxylic acid |
| (46) BnH12095 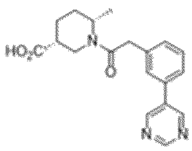 | (3R,6S)-6-methyl-1-(2-(3-(pyrimidin-5-yl)phenyl)acetyl)piperidine-3-carboxylic acid |
| (47) BnH12097 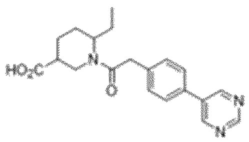 | 6-ethyl-1-(2-(4-(pyrimidin-5-yl)phenyl)acetyl)piperidine-3-carboxylic acid |
| (43) BnH12098 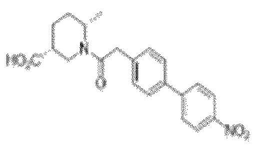 | 6-isopropyl-1-(2-(4-(pyrimidin-5-yl)phenyl)acetyl)piperidine-3-carboxylic acid |

Methods for obtaining the compounds described herein, pharmaceutically acceptable salts thereof, or stereoisomers thereof will be apparent to those skilled in the art, and suitable procedures are described, for example, in the following preparation examples and examples as well as in the references cited herein.

**Preparation Example**

**Preparation Example 1. Preparation of active compounds according to representative reaction scheme 1**

[Reaction scheme 1]

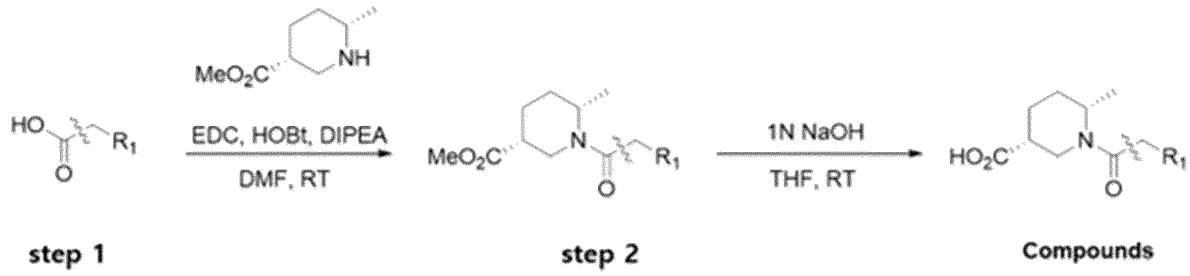


| Compound | R1 | Compound | R1 |
|---|---|---|---|
| BnH12036 | 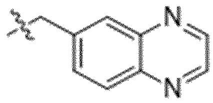 | BnH12037 | 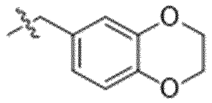 |
| BnH12042 | 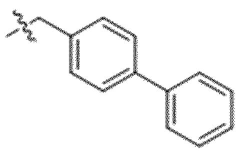 | BnH12078 | 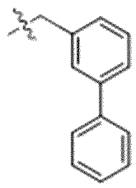 |
| BnH12080 | 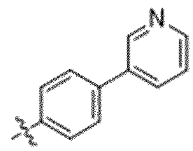 | | |

**(1) Synthesis of compound BnH12036**

(3R,6S)-6-methyl-1-(2-(quinoxalin-6-yl)acetyl)piperidine-3-carboxylic acid

Step 1: Synthesis of methyl (3R,6S)-6-methyl-1-(2-(quinoxalin-6-yl)acetyl)piperidine-3-carboxylate

To a solution 2-(quinoxalin-6-yl)acetic acid (100 mg, 0.531 mmol) in DMF (2.50 mL) were added methyl (3R,6S)-rel-6-methyl-3-piperidinecarboxylate (87.7 mg, 0.558 mmol), DIPEA (0.139 mL, 0.797 mmol), HOBt (86.2 mg, 0.638 mmol) and EDC·HCl (99.0 mg, 0.638 mmol). The mixture was stirred at room temperature for overnight. The mixture was diluted with EtOAc (5.00 mL), washed with sat. aq. $NH_4Cl$ (5.00 mL) and brine (5.00 mL). The organic layer was dried over $MgSO_4$, filtered, and concentrated under reduced pressure. The residue was purified by column chromatography on $SiO_2$ (DCM : MeOH = 0 to 5 %) to afford desired compound (80.4 mg, 46 %) as a deep yellow oil.

Step 2: Synthesis of compound BnH12036

To a solution of methyl (3R,6S)-6-methyl-1-(2-(quinoxalin-6-yl)acetyl)piperidine-3-carboxylate (72.5 mg, 0.221 mmol) in THF (1.50 mL) was added 1N NaOH (0.332 mL, 0.332 mmol). The mixture was stirred at room temperature for 2 hours. The mixture was diluted with $H_2O$ (5.00 mL) and washed two times with DCM (5.00 mL × 2). The aqueous layer was acidified with 1N HCl until pH 2~3 at 0 °C and extracted with EtOAc (5.00 mL). The organic layer was washed with brine (5.00 mL), dried over $MgSO_4$, filtered, and concentrated under reduced pressure to afford compound BnH12036 (20.0 mg, 28 %) as a white solid.

$^1H$ NMR (400 MHz, DMSO-$d_6$) δ 12.42 (s, 1H), 8.92 (d, $J$ = 4.1 Hz, 2H), 8.04 (d, $J$ = 8.6 Hz, 1H), 7.94 (d, $J$ = 23.0 Hz,

1H), 7.73 (dd, *J* = 14.3, 8.6 Hz, 1H), 4.76 (d, *J* = 8.4 Hz, 0.5H), 4.55 (dd, *J* = 13.5, 4.2 Hz, 0.5H), 4.36 (d, *J* = 8.9 Hz, 0.5H), 1.44 - 3.97 (m, 2.5H), 3.08 (t, *J* = 12.8 Hz, 0.5H), 2.65 (t, *J* = 12.6 Hz, 0.5H), 2.29 - 2.17 (m, 1H), 1.83 - 1.78 (m, 1H), 1.73 - 1.62 (m, 1H), 1.56 - 1.54 (m, 2H), 1.10 (dd, *J* = 18.1, 6.9 Hz, 3H).

**(2) Synthesis of compound BnH12037**

(3R,6S)-1-(2-(2,3-dihydrobenzo[b][1,4]dioxin-6-yl)acetyl)-6-methylpiperidine-3-carboxylic acid

Step 1: Synthesis of methyl (3R,6S)-1-(2-(2,3-dihydrobenzo[b][1,4]dioxin-6-yl)acetyl)-6-methylpiperidine-3-carboxylate

To a solution of 2-(2,3-dihydrobenzo[b][1,4]dioxin-6-yl)acetic acid (100 mg, 0.515 mmol) in DMF (2.50 mL) were added methyl (3R,6S)-rel-6-methyl-3-piperidinecarboxylate (85.0 mg, 0.541 mmol), DIPEA (0.135 mL, 0.772 mmol), HOBt (83.5 mg, 0.618 mmol) and EDC·HCl (95.9 mg, 0.618 mmol). The mixture was stirred at room temperature for overnight. The mixture was diluted with EtOAc (5.00 mL), washed with sat. aq. $NH_4Cl$ (5.00 mL) and brine (5.00 mL). The organic layer was dried over $MgSO_4$, filtered, and concentrated under reduced pressure. The residue was purified by column chromatography on $SiO_2$ (DCM : MeOH = 0 to 5 %) to afford desired compound (62.0 mg, 38 %) as a deep yellow oil.

Step 2: Synthesis of compound BnH12037

To a solution of methyl (3R,6S)-1-(2-(2,3-dihydrobenzo[b][1,4]dioxin-6-yl)acetyl)-6-methylpiperidine-3-carboxylate (135 mg, 0.405 mmol) in THF (2.00 mL) was added 1N NaOH (0.607 mL, 0.607 mmol). The mixture was stirred at room temperature for 2 hours. The mixture was diluted with $H_2O$ (5.00 mL) and washed two times with DCM (5.00 mL × 2). The aqueous layer was acidified with 1N HCl until pH 1-2 at 0 °C and extracted with EtOAc (5.00 mL). The organic layer was washed with brine (5.00 mL), dried over $MgSO_4$, filtered, and concentrated under reduced pressure to afford compound BnH12037 (90.0 mg, 69 %) as a white solid.

$^1H$ NMR (400 MHz, DMSO-$d_6$) δ 12.36 (s, 1H), 6.78 - 6.61 (m, 3H), 4.71 (s, 0.5H), 4.51 (dd, *J* = 13.3, 4.3 Hz, 0.5H), 4.20 (s, 4H), 3.88 - 3.84 (m, 0.5H), 3.63 - 3.54 (m, 2H), 2.97 (t, *J* = 12.9 Hz, 0.5H), 2.58 (t, *J* = 12.7 Hz, 0.5H), 2.18 - 2.09 (m, 1H), 1.81 - 1.75 (m, 1H), 1.69 - 1.59 (m, 1H), 1.50 - 1.43 (m, 2H), 1.03 (dd, *J* = 7.0, 3.4 Hz, 3H).

**(3) Synthesis of compound BnH12042**

(3R,6S)-1-(2-([1,1'-biphenyl]-4-yl)acetyl)-6-methylpiperidine-3-carboxylic acid

Step 1: Synthesis of methyl (3R,6S)-1-(2-([1,1'-biphenyl]-4-yl)acetyl)-6-methylpiperidine-3-carboxylate

To a solution of 4-biphenylacetic acid (110 mg, 0.518 mmol) in DMF (1.73 mL) were added methyl (3R,6S)-rel-6-methyl-3-piperidinecarboxylate (85.6 mg, 0.544 mmol), DIPEA (0.135 mL, 0.777 mmol), HOBt (84.0 mg, 0.622 mmol) and EDC·HCl (96.6 mg, 0.622 mmol). The mixture was stirred at room temperature for 17 hours. The mixture was diluted with EtOAc (5.00 mL), washed with sat. aq. $NH_4Cl$ (5.00 mL) and brine (5.00 mL). The organic layer was dried over $MgSO_4$, filtered, and concentrated under reduced pressure. The residue was purified by column chromatography on $SiO_2$ (*n*-Hexane : EtOAc = 3 : 1) to afford desired compound (145 mg, 79 %) as a colorless oil.

Step 2: Synthesis of compound BnH12042

To a solution of methyl (3R,6S)-1-(2-([1,1'-biphenyl]-4-yl)acetyl)-6-methylpiperidine-3-carboxylate (145 mg, 0.413 mmol) in THF (1.42 mL) was added 1N NaOH (0.619 mL, 0.619 mmol). The mixture was stirred at room temperature for 2 hours. The mixture was diluted with $H_2O$ (3.00 mL) and washed two times with DCM (3.00 mL × 2). The aqueous layer was acidified with 1N HCl until pH 1-2 at 0 °C and extracted with EtOAc (3.00 mL). The organic layer was washed with brine (3.00 mL), dried over $MgSO_4$, filtered, and concentrated under reduced pressure to afford compound BnH12042 (120 mg, 86 %) as a white solid.

$^1H$ NMR (400 MHz, Chloroform-d) δ 7.57 (dd, J = 10.6, 7.8 Hz, 4H), 7.43 (t, J = 7.5 Hz, 2H), 7.33 (dd, J = 10.8, 7.6 Hz, 3H), 4.99 (s, 0.5H), 4.84 (d, J = 13.7 Hz, 0.5H), 4.22 (s, 0.5H), 3.96 (d, J = 13.8 Hz, 0.5H), 3.79 (s, 2H), 3.17 (t, J = 12.9 Hz, 0.5H), 2.79 (t, J = 12.9 Hz, 0.5H), 2.41 (s, 0.5H), 2.14 (s, 0.5H), 1.92 (d, J = 13.0 Hz, 1H), 1.80 (d, J = 13.6 Hz, 1H), 1.58 (d, J = 24.0 Hz, 2H), 1.15 (d, J = 6.8 Hz, 3H).

**(4) Synthesis of compound BnH12078**

(3R,6S)-1-(2-([1,1'-biphenyl]-3-yl)acetyl)-6-methylpiperidine-3-carboxylic acid

Step 1: Synthesis of methyl (3R,6S)-1-(2-([1,1'-biphenyl]-3-yl)acetyl)-6-methylpiperidine-3-carboxylate

To a solution of 3-Biphenylylacetic acid (70.0 mg, 0.328 mmol) in DMF (1.09 mL) were added methyl (3R,6S)-rel-6-methyl-3-piperidinecarboxylate (54.2 mg, 0.345 mmol), DIPEA (0.086 mL, 0.492 mmol), HOBt (53.2 mg, 0.394 mmol) and EDC·HCl (61.2 mg, 0.394 mmol). The mixture was stirred at room temperature for overnight. The mixture was diluted with EtOAc (5.00 mL), washed with sat. aq. $NH_4Cl$ (5.00 mL) and brine (5.00 mL). The organic layer was dried over $MgSO_4$, filtered, and concentrated under reduced pressure. The residue was purified by column chromatography on $SiO_2$ (n-Hexane : EtOAc = 2 : 1) to afford desired compound (69.2 mg, 60 %) as a colorless oil.

Step 2: Synthesis of compound BnH12078

To a solution of (3R,6S)-1-(2-([1,1'-biphenyl]-3-yl)acetyl)-6-methylpiperidine-3-carboxylate (69.2 mg, 0.196 mmol) in THF (0.675 mL) was added 1N NaOH (0.294 mL, 0.294 mmol). The mixture was stirred at room temperature for 2 hours. The mixture was diluted with $H_2O$ (3.00 mL) and washed two times with EtOac (3.00 mL × 2). The aqueous layer was acidified with 1N HCl until pH 1~2 at 0 °C and extracted with EtOAc (3.00 mL). The organic layer was washed with brine (3.00 mL), dried over $MgSO_4$, filtered, and concentrated under reduced pressure. The residue was purified by column chromatography on $SiO_2$ (10% MeOH in DCM) to afford compound BnH12078 (50.0 mg, 76 %) as a white solid.

$^1$H NMR (400 MHz, DMSO-d6) δ 12.43 (s, 1H), 7.63 (t, J = 6.1 Hz, 2H), 7.49 (dt, J = 15.4, 9.1 Hz, 4H), 7.39 (dt, J = 14.3, 7.5 Hz, 2H), 7.22 (dd, J = 16.7, 7.6 Hz, 1H), 4.75 (s, 0.5H), 4.54 (d, J = 13.6 Hz, 0.5H), 4.32 (s, 0.5H), 3.96 (d, J = 13.8 Hz, 0.5H), 3.89 - 3.72 (m, 2H), 3.03 (t, J = 12.9 Hz, 0.5H), 2.62 (t, J = 12.7 Hz, 0.5H), 2.16 (d, J = 12.8 Hz, 1H), 1.79 (d, J = 12.8 Hz, 1H), 1.71 - 1.59 (m, 1H), 1.57 - 1.42 (m, 2H), 1.06 (t, J = 6.5 Hz, 3H).

**(5) Synthesis of compound BnH12080**

(3R,6S)-6-methyl-1-(4-(pyridin-3-yl)benzoyl)piperidine-3-carboxylic acid

Step 1: Synthesis of methyl (3R,6S)-6-methyl-1-(4-(pyridin-3-yl)benzoyl)piperidine-3-carboxylate

To a suspension of 4-(pyridin-3-yl)benzoic acid (100 mg, 0.502 mmol) in DMF (1.67 mL) were added methyl (3R,6S)-6-methylpiperidine-3-carboxylate (82.9 mg, 0.527 mmol), DIPEA (0.132 mL, 0.753 mmol), HOBt (81.4 mg, 0.602 mmol) and EDC·HCl (93.5 mg, 0.602 mmol). The mixture was stirred at room temperature for 2 hours. The mixture was diluted with EtOAc (4.00 mL), washed with sat. aq. $NH_4Cl$ (4.00 mL) and brine (4.00 mL). The organic layer was dried over $MgSO_4$, filtered, and concentrated under reduced pressure. The residue was purified by column chromatography on $SiO_2$ (DCM : MeOH = 0 to 2 %) to afford desired compound (82.0 mg, 48.3 %) as a pale yellow oil.

Step 2: Synthesis of compound BnH12080

To a solution of methyl (3R,6S)-6-methyl-1-(4-(pyridin-3-yl)benzoyl)piperidine-3-carboxylate (82.0 mg, 0.242 mmol) in THF (0.836 mL) was added 1N NaOH (0.363 mL, 0.363 mmol). The mixture was stirred at room temperature for 2 hours. The mixture was diluted with $H_2O$ (5.00 mL) and washed two times with DCM (5.00 mL × 2). The aqueous layer was acidified with 1N HCl until pH 3~4 at 0 °C and extracted with EtOAc (5.00 mL). The organic layer was washed with brine (5.00 mL), dried over $MgSO_4$, filtered, and concentrated under reduced pressure to afford compound BnH12080 (47.0 mg, 59.8 %) as a white solid.

$^1$H NMR (400 MHz, DMSO-$d_6$) δ 12.47 (s, 1H), 8.95 (d, *J* = 2.4 Hz, 1H), 8.62-8.60 (dd, *J* = 4.8, 1.6 Hz, 1H), 8.15-8.12 (m, 1H), 7.83-7.81 (d, *J* = 8.0 Hz, 2H), 7.53-7.50 (dd, *J* = 7.9, 4.5 Hz, 3H), 2.45-2.34 (m, 2H), 1.92-1.88 (d, *J* = 15.5 Hz, 1.5H), 1.80-1.69 (dt, *J* = 20.7, 7.5 Hz, 2H), 1.57 (s, 1.5H), 1.21-1.18 (dd, *J* = 7.1, 2.8 Hz, 4H).

**Preparation Example 2. Preparation of active compounds according to representative reaction scheme 3**

[Reaction scheme 3]

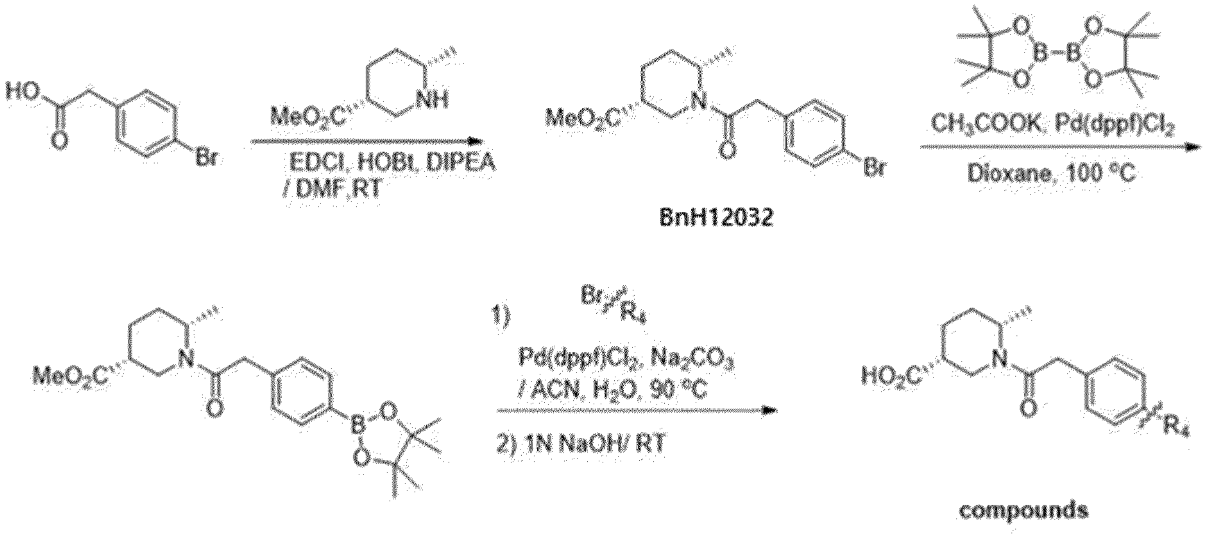


BnH12032

compounds

| Compound | $R_4$ | Compound | $R_4$ |
|---|---|---|---|
| BnH12043 | 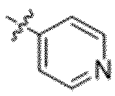 | BnH12044 | 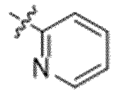 |
| BnH12045 | 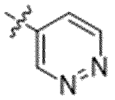 | BnH12046 | 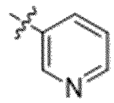 |
| BnH12047 | 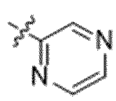 | BnH12048 | 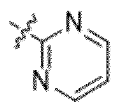 |
| BnH12049 | 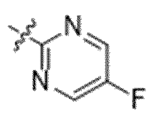 | BnH12050 | 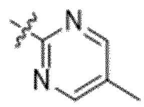 |
| BnH12051 | 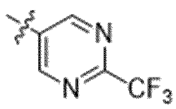 | BnH12052 | 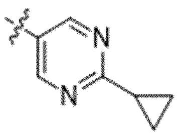 |
| BnH12053 | 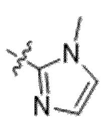 | BnH12054 | 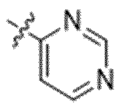 |

(continued)

| Compound | $R_4$ | Compound | $R_4$ |
|---|---|---|---|
| BnH12055 | 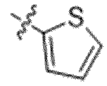 | BnH12056 | 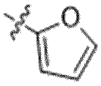 |
| BnH12072 | 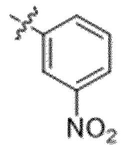 | BnH12077 | 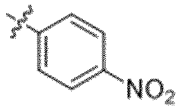 |
| BnH12081 | 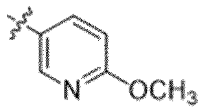 | BnH12082 | 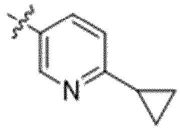 |
| BnH12083 | 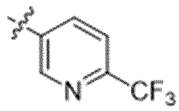 | BnH12084 | 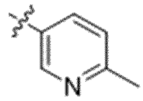 |
| BnH12085 | 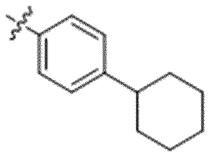 | BnH12086 | 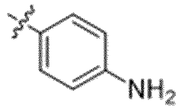 |
| BnH12087 | 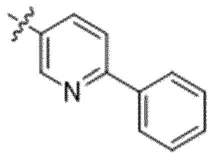 | BnH12088 | 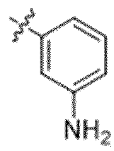 |
| BnH12089 | 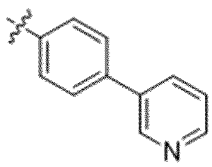 | BnH12090 | 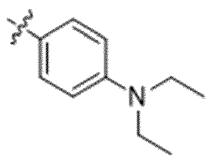 |

**Step 1: Synthesis of methyl (3R,6S)-6-methyl-1-(2-(4-(4,4,5,5-tetramethyl-1,3,2-dioxaborolan-2-yl)phenyl)acetyl)piperidine-3-carboxylate (compound 3-1)**

The first step in the reaction scheme 3 was performed to obtain compound BnH12032. Then, a suspension of compound BnH12032 (1.60 g, 4.52 mmol), bis(pinacolato)diboron (1.62 g, 4.97 mmol), and KOAc (1.33 g, 13.6 mmol) in 1,4-dioxane (15.6 mL) was stirred vigorously and bubbled with nitrogen gas for 5 minutes then Pd(dppf)$Cl_2$ (327 mg, 0.447 mmol) was added. The mixture was bubbled with nitrogen gas for 5 minutes and refluxed at 100 °C for 17 hours. After completion of reaction, the reaction mixture was filtered through a celite pad and washed with DCM (10.0 mL × 3). The combined organic layer was washed with $H_2O$ (40.0 mL), dried over $MgSO_4$, filtered and concentrated under reduced pressure. The residue was purified by column chromatography on $SiO_2$ (n-Hexane : EtOAc = 1 : 1) to afford compound 3-1 (1.40 g, 77 %) as a brown oil.

**(1) Synthesis of compound BnH12043**

(3R,6S)-6-methyl-1-(2-(4-(pyridin-4-yl)phenyl)acetyl)piperidine-3-carboxylic acid

Step 1: Synthesis of compound BnH12043

A solution of compound 3-1 (235 mg, 0.586 mmol), 4-bromopyridine (125 mg, 0.644 mmol) and $Na_2CO_3$ (186 mg, 1.76 mmol) in MeCN/$H_2O$ (2:1, 7.55 mL) was stirred vigorously and bubbled with $N_2$ gas for 5 minutes and the Pd(dppf)$Cl_2$ (42.9 mg, 0.059 mmol) was added. The mixture was bubbled with $N_2$ gas for 5 minutes and refluxed at 90 °C for 3 hours. Then, the mixture was cooled to room temperature, 1N NaOH (1.17 mL, 1.17 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 4~5 and the mixture was extracted with EtOAc (3.00 mL × 3). The combined organic layer was dried over $MgSO_4$, filtered, and concentrated under reduced pressure. The residue was purified by column chromatography on $SiO_2$ (3% MeOH in DCM) to afford compound BnH12043 (85.0 mg, 42 %) as a white solid.

$^1$H NMR (400 MHz, DMSO-d6) δ 8.65 - 8.59 (m, 2H), 7.76 (d, J = 7.9 Hz, 2H), 7.71 (d, J = 5.6 Hz, 2H), 7.37 (dd, J = 18.7, 7.9 Hz, 2H), 4.75 (s, 0.5H), 4.60 - 4.50 (m, 0.5H), 4.27 (d, J = 7.7 Hz, 0.5H), 3.92 (d, J = 13.7 Hz, 0.5H), 3.88 - 3.72 (m, 2H), 3.04 (t, J = 12.9 Hz, 0.5H), 2.63 (d, J = 12.6 Hz, 0.5H), 2.24 - 2.12 (m, 1H), 1.79 (dt, J = 16.9, 5.7 Hz, 1H), 1.65 (d, J = 12.6 Hz, 1H), 1.57 - 1.49 (m, 2H), 1.08 (dt, J = 11.7, 4.9 Hz, 3H).

**(2) Synthesis of compound BnH12044**

(3R,6S)-6-methyl-1-(2-(4-(pyridin-2-yl)phenyl)acetyl)piperidine-3-carboxylic acid

Step 1: Synthesis of compound BnH12044

A solution of compound 3-1 (235 mg, 0.586 mmol), 2-bromopyridine (125 mg, 0.644 mmol) and $Na_2CO_3$ (186 mg, 1.76 mmol) in MeCN/$H_2O$ (2:1, 7.55 mL) was stirred vigorously and bubbled with $N_2$ gas for 5 minutes and the Pd(dppf)$Cl_2$ (42.9 mg, 0.059 mmol) was added. The mixture was bubbled with $N_2$ gas for 5 minutes and refluxed at 90 °C for 3 hours. Then, the mixture was cooled to room temperature, 1N NaOH (1.17 mL, 1.17 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 4-5 and the mixture was extracted with EtOAc (3.00 mL × 3). The combined organic layer was dried over $MgSO_4$, filtered, and concentrated under reduced pressure. The residue was purified by column chromatography on $SiO_2$ (3% to 5 % MeOH in DCM) to afford compound BnH12044 (85.0 mg, 42 %) as a white solid.

$^1$H NMR (400 MHz, Chloroform-d) δ 8.75 (d, J = 23.7 Hz, 1H), 7.89 (dd, J = 26.8, 7.9 Hz, 2H), 7.83 - 7.67 (m, 2H), 7.40 (dd, J = 16.2, 7.9 Hz, 2H), 7.26 (m, 1H), 4.97 (s, 0.5H), 4.81 (d, J= 13.9 Hz, 0.5H), 4.18 (s, 0.5H), 3.99 (d, J = 13.7 Hz, 0.5H), 3.83 (s, 2H), 3.13 (t, J = 12.9 Hz, 0.5H), 2.80 (t, J = 12.9 Hz, 0.5H), 2.40 (s, 0.5H), 2.02 (s, 0.5H), 1.88 (s, 1H), 1.81 1.69 (m, 1H), 1.62 - 1.40 (m, 2H), 1.14 (d, J = 6.4 Hz, 3H).

**(3) Synthesis of compound BnH12045**

(3R,6S)-6-methyl-1-(2-(4-(pyridazin-4-yl)phenyl)acetyl)piperidine-3-carboxylic acid

Step 1: Synthesis of compound BnH12045

A solution of compound 3-1 (240 mg, 0.598 mmol), 4-bromopyridazine hydrobromide (158 mg, 0.658 mmol) and $Na_2CO_3$ (190 mg, 1.79 mmol) in MeCN/$H_2O$ (2:1, 7.47 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 4-5 and the mixture was extracted with EtOAc (3.00 mL × 3). The combined organic layer was dried over $MgSO_4$, filtered, and concentrated under reduced pressure. The residue was purified by column chromatography on $SiO_2$ (5 % MeOH in DCM) to afford compound BnH12045 (65.0 mg, 32 %) as a pink solid.

$^1$H NMR (400 MHz, Chloroform-d) δ 9.48 (s, 1H), 9.23 (d, J = 5.4 Hz, 1H), 7.65 (d, J = 8.1 Hz, 3H), 7.44 (d, J = 7.9 Hz, 2H), 4.98 (s, 0.5H), 4.84 (d, J = 13.6 Hz, 0.5H), 4.21 (s, 0.5H), 3.94 (d, J = 14.9 Hz, 0.5H), 3.82 (s, 2H), 3.20 (t, J = 12.9 Hz, 0.5H), 2.82 (s, 0.5H), 2.43 (s, 0.5H), 2.20 (s, 0.5H), 1.97 (d, J= 13.7 Hz, 1H), 1.81 (s, 1H), 1.62 (s, 2H), 1.24 - 1.13 (m,

3H).

**(4) Synthesis of compound BnH12046**

(3R,6S)-6-methyl-1-(2-(4-(pyridin-3-yl)phenyl)acetyl)piperidine-3-carboxylic acid

Step 1: Synthesis of compound BnH12046

A solution of compound 3-1 (235 mg, 0.586 mmol), 3-bromopyridine (102 mg, 0.644 mmol) and $Na_2CO_3$ (124 mg, 1.17 mmol) in MeCN/$H_2O$ (2:1, 7.55 mL) was stirred vigorously and bubbled with $N_2$ gas for 5 minutes and the Pd(dppf)$Cl_2$ (42.9 mg, 0.059 mmol) was added. The mixture was bubbled with $N_2$ gas for 5 minutes and refluxed at 90 °C for 3 hours. Then, the mixture was cooled to room temperature, 1N NaOH (1.17 mL, 1.17 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 4-5 and the mixture was extracted with EtOAc (3.00 mL × 3). The combined organic layer was dried over $MgSO_4$, filtered, and concentrated under reduced pressure. The residue was purified by column chromatography on $SiO_2$ (3% to 5 % MeOH in DCM) to afford compound BnH12046 (90.2 mg, 45 %) as a white solid.

$^1$H NMR (400 MHz, DMSO-d6) δ 12.41 (s, 1H), 8.89 (d, J = 2.4 Hz, 1H), 8.56 (dd, J = 4.8, 1.6 Hz, 1H), 8.07 (dt, J = 8.0, 2.0 Hz, 1H), 7.68 (d, J = 7.8 Hz, 2H), 7.48 (dd, J = 7.9, 4.7 Hz, 1H), 7.35 (dd, J = 18.4, 7.8 Hz, 2H), 4.75 (s, 0.5H), 4.57 - 4.49 (m, 0.5H), 4.29 (s, 0.5H), 3.98 - 3.87 (m, 0.5H), 3.87 - 3.71 (m, 2H), 3.04 (t, J = 12.9 Hz, 0.5H), 2.62 (t, J = 12.7 Hz, 0.5H), 2.20 (dq, J = 12.0, 8.1, 7.1 Hz, 1H), 1.80 (dd, J = 13.4, 3.9 Hz, 1H), 1.66 (d, J = 13.6 Hz, 1H), 1.53 (s, 2H), 1.12 - 1.03 (m, 3H).

**(5) Synthesis of compound BnH12047**

(3R,6S)-6-methyl-1-(2-(4-(pyrazin-2-yl)phenyl)acetyl)piperidine-3-carboxylic acid

Step 1: Synthesis of compound BnH12047

A solution of compound 3-1 (235 mg, 0.586 mmol), 2-bromopyrazine (102 mg, 0.644 mmol) and $Na_2CO_3$ (124 mg, 1.17 mmol) in MeCN/$H_2O$ (2:1, 7.55 mL) was stirred vigorously and bubbled with $N_2$ gas for 5 minutes and the Pd(dppf)$Cl_2$ (42.9 mg, 0.059 mmol) was added. The mixture was bubbled with $N_2$ gas for 5 minutes and refluxed at 90 °C for 3 hours. Then, the mixture was cooled to room temperature, 1N NaOH (1.17 mL, 1.17 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 1-2 and the mixture was extracted with EtOAc (3.00 mL × 3). The combined organic layer was dried over $MgSO_4$, filtered, and concentrated under reduced pressure. The residue was purified by column chromatography on $SiO_2$ (3% to 5 % MeOH in DCM) to afford compound BnH12047 (76.0 mg, 38 %) as a white solid.

$^1$H NMR (400 MHz, DMSO-d6) δ 12.41 (s, 1H), 9.25 (d, J = 1.5 Hz, 1H), 8.71 (t, J = 2.0 Hz, 1H), 8.60 (d, J = 2.5 Hz, 1H), 8.10 (d, J = 7.9 Hz, 2H), 7.39 (dd, J = 18.2, 7.9 Hz, 2H), 4.75 (s, 0.5H), 4.55 (dd, J = 13.6, 4.2 Hz, 0.5H), 4.28 (s, 0.5H), 3.97 - 3.89 (m, 0.5H), 3.89 - 3.74 (m, 2H), 3.04 (t, J = 12.9 Hz, 0.5H), 2.63 (t, J = 12.7 Hz, 0.5H), 2.19 (tt, J = 12.0, 4.2 Hz, 1H), 1.79 (dd, J = 13.5, 3.9 Hz, 1H), 1.66 (h, J = 13.1, 10.8 Hz, 1H), 1.52 (dq, J = 8.8, 4.9, 4.2 Hz, 2H), 1.07 (dd, J = 9.1, 6.8 Hz, 3H).

**(6) Synthesis of compound BnH12048**

(3R,6S)-6-methyl-1-(2-(4-(pyrimidin-2-yl)phenyl)acetyl)piperidine-3-carboxylic acid

Step 1: Synthesis of compound BnH12048

A solution of compound 3-1 (170 mg, 0.424 mmol), 2-bromopyrimidine (67.4 mg, 0.424 mmol) and $Na_2CO_3$ (89.8 mg, 0.847 mmol) in MeCN/$H_2O$ (2:1, 5.47 mL) was stirred vigorously and bubbled with $N_2$ gas for 5 minutes and the Pd(dppf)$Cl_2$ (31.0 mg, 0.042 mmol) was added. The mixture was bubbled with $N_2$ gas for 5 minutes and refluxed at 90 °C for 3 hours. Then, the mixture was cooled to room temperature, 1N NaOH (0.847 mL, 0.847 mmol) was added to the mixture and stirred at room temperature for 3 hours. The mixture was diluted with $H_2O$ (2.00 mL) and washed with DCM (3.00 mL × 3). To the aqueous layer 1N HCl was added to adjust the pH value to pH 1~2 and the mixture was extracted with EtOAc (3.00 mL × 3). The combined organic layer was dried over $MgSO_4$, filtered, and concentrated under reduced pressure. The residue was purified by column chromatography on $SiO_2$ (3% to 5 % MeOH in DCM) to afford compound BnH12048

(34.0 mg, 23 %) as a white solid.

$^1H$ NMR (400 MHz, Chloroform-d) δ 8.83 (t, J = 5.6 Hz, 2H), 8.37 (dd, J = 16.1, 7.9 Hz, 2H), 7.41 (t, J = 6.8 Hz, 2H), 7.20 (t, J = 4.9 Hz, 1H), 4.98 (s, 0.5H), 4.83 (d, J = 13.7 Hz, 0.5H), 4.17 (s, 0.5H), 3.99 - 3.91 (m, 0.5H), 3.84 (s, 2H), 3.13 (t, J = 13.0 Hz, 0.5H), 2.80 (t, J = 12.8 Hz, 0.5H), 2.40 (s, 0.5H), 2.03 (d, J = 7.0 Hz, 0.5H), 1.88 (s, 1H), 1.76 (t, J = 11.8 Hz, 1H), 1.62 - 1.40 (m, 2H), 1.17 - 1.09 (m, 3H).

**(7) Synthesis of compound BnH12049**

(3R,6S)-1-(2-(4-(5-fluoropyrimidin-2-yl)phenyl)acetyl)-6-methylpiperidine-3-carboxylic acid

Step 1: Synthesis of compound BnH12049

A solution of compound 3-1 (170 mg, 0.424 mmol), 2-bromo-5-fluoropyrimidine (82.5 mg, 0.466 mmol) and $Na_2CO_3$ (89.8 mg, 0.847 mmol) in MeCN/$H_2O$ (2:1, 5.47 mL) was stirred vigorously and bubbled with $N_2$ gas for 5 minutes and the Pd(dppf)$Cl_2$ (31.0 mg, 0.042 mmol) was added. The mixture was bubbled with $N_2$ gas for 5 minutes and refluxed at 90 °C for 3 hours. Then, the mixture was cooled to room temperature, 1N NaOH (0.847 mL, 0.847 mmol) was added to the mixture and stirred at room temperature for 3 hours. The mixture was diluted with $H_2O$ (2.00 mL) and washed with DCM (3.00 mL × 3). To the aqueous layer 1N HCl was added to adjust the pH value to pH 1-2 and the mixture was extracted with EtOAc (3.00 mL × 3). The combined organic layer was dried over $MgSO_4$, filtered, and concentrated under reduced pressure. The residue was purified by column chromatography on $SiO_2$ (3% to 5 % MeOH in DCM) to afford compound BnH12049 (42.0 mg, 27 %) as a white solid.

$^1H$ NMR (400 MHz, Chloroform-d) δ 8.66 (s, 2H), 8.33 (s, 2H), 7.38 (d, J = 8.3 Hz, 2H), 4.98 (s, 0.5H), 4.83 (d, J = 13.8 Hz, 0.5H), 4.18 (s, 0.5H), 3.93 (d, J = 13.9 Hz, 0.5H), 3.82 (s, 2H), 3.14 (t, J = 13.0 Hz, 0.5H), 2.79 (t, J = 12.8 Hz, 0.5H), 2.40 (s, 0.5H), 2.10 (s, 0.5H), 1.91 (d, J = 13.2 Hz, 1H), 1.78 (d, J = 13.3 Hz, 1H), 1.56 (d, J = 31.7 Hz, 2H), 1.13 (s, 3H).

**(8) Synthesis of compound BnH12050**

(3R,6S)-6-methyl-1-(2-(4-(2-methylpyrimidin-5-yl)phenyl)acetyl)piperidine-3-carboxylic acid

Step 1: Synthesis of compound BnH12050

A solution of compound 3-1 (233 mg, 0.581 mmol), 5-bromo-2-methylpyrimidine (110 mg, 0.639 mmol) and $Na_2CO_3$ (123 mg, 1.16 mmol) in MeCN/$H_2O$ (2:1, 7.49 mL) was stirred vigorously and bubbled with $N_2$ gas for 5 minutes and the Pd(dppf)$Cl_2$ (42.5 mg, 0.058 mmol) was added. The mixture was bubbled with $N_2$ gas for 5 minutes and refluxed at 90 °C for 3 hours. Then, the mixture was cooled to room temperature, 1N NaOH (1.16 mL, 1.16 mmol) was added to the mixture and stirred at room temperature for 3 hours. The mixture was diluted with $H_2O$ (2.00 mL) and washed with DCM (3.00 mL × 3). To the aqueous layer 1N HCl was added to adjust the pH value to pH 1~2 and the mixture was extracted with EtOAc (3.00 mL × 3). The combined organic layer was dried over $MgSO_4$, filtered, and concentrated under reduced pressure. The residue was purified by column chromatography on $SiO_2$ (3% to 5 % MeOH in DCM) to afford compound BnH12050 (45.0 mg, 21 %) as a white solid.

$^1H$ NMR (400 MHz, Chloroform-d) δ 8.88 (d, J = 8.6 Hz, 2H), 7.53 (d, J = 8.0 Hz, 2H), 7.39 (s, 2H), 4.99 (s, 0.5H), 4.84 (d, J = 13.5 Hz, 0.5H), 4.21 (s, 0.5H), 3.92 (d, J = 13.9 Hz, 0.5H), 3.82 (s, 2H), 3.16 (t, J = 12.9 Hz, 0.5H), 2.79 (s, 3.5H), 2.42 (s, 1H), 2.19 (d, J = 11.2 Hz, 0.5H), 1.96 (s, 0.5H), 1.80 (t, J = 13.5 Hz, 1H), 1.61 (d, J = 16.9 Hz, 2H), 1.18 (q, J = 8.0 Hz, 3H).

**(9) Synthesis of compound BnH12051**

(3R,6S)-6-methyl-1-(2-(4-(2-(trifluoromethyl)pyrimidin-5-yl)phenyl)acetyl)piperidine-3-carboxylic acid

Step 1: Synthesis of compound BnH12051

A solution of compound 3-1 (240 mg, 0.598 mmol), 2-chloro-5-(trifluoromethyl)pyrimidine (120 mg, 0.658 mmol) and $Na_2CO_3$ (127 mg, 1.20 mmol) in MeCN/$H_2O$ (2:1, 7.72 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 1-2 and the mixture was extracted with EtOAc (3.00 mL × 3). The combined organic layer was dried over $MgSO_4$, filtered, and concentrated under

reduced pressure. The residue was purified by column chromatography on $SiO_2$ (5% to 10 % MeOH in DCM) to afford compound BnH12051 (142 mg, 58 %) as a white solid.

$^1$H NMR (400 MHz, DMSO-d6) δ 12.44 (s, 1H), 9.33 (s, 2H), 8.40 (dd, J = 8.3, 2.0 Hz, 2H), 7.43 (dd, J = 17.7, 8.0 Hz, 2H), 4.75 (d, J = 9.4 Hz, 0.5H), 4.55 (dd, J = 13.5, 4.1 Hz, 0.5H), 4.26 (d, J = 8.6 Hz, 0.5H), 3.98 - 3.78 (m, 2.5H), 3.04 (t, J = 12.9 Hz, 0.5H), 2.63 (t, J = 12.8 Hz, 0.5H), 2.25 - 2.11 (m, 1H), 1.78 (dt, J = 12.4, 3.9 Hz, 1H), 1.66 (dq, J = 19.1, 12.9, 10.3 Hz, 1H), 1.52 (dq, J = 11.8, 7.5, 5.7 Hz, 2H), 1.07 (t, J = 7.2 Hz, 3H).

**(10) Synthesis of compound BnH12052**

(3R,6S)-1-(2-(4-(2-cyclopropylpyrimidin-5-yl)phenyl)acetyl)-6-methylpiperidine-3-carboxylic acid

Step 1: Synthesis of compound BnH12052

A solution of compound 3-1 (240 mg, 0.598 mmol), 5-bromo-2-cyclopropylprimidine (131 mg, 0.658 mmol) and $Na_2CO_3$ (127 mg, 1.20 mmol) in MeCN/$H_2O$ (2:1, 7.72 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 1-2 and the mixture was extracted with EtOAc (3.00 mL × 3). The combined organic layer was dried over $MgSO_4$, filtered, and concentrated under reduced pressure. The residue was purified by column chromatography on $SiO_2$ (5% to 10 % MeOH in DCM) to afford compound BnH12052 (84.9 mg, 37 %) as a white solid.

$^1$H NMR (400 MHz, Chloroform-d) δ 8.79 (s, 2H), 7.50 (d, J = 8.0 Hz, 2H), 7.38 (d, J = 8.2 Hz, 2H), 4.99 (s, 0.5H), 4.84 (d, J = 13.8 Hz, 0.5H), 4.21 (s, 0.5H), 3.93 (d, J = 14.0 Hz, 0.5H), 3.80 (s, 2H), 3.17 (t, J = 12.9 Hz, 0.5H), 2.81 (t, J = 12.9 Hz, 0.5H), 2.43 (s, 0.5H), 2.30 (ddd, J = 13.1, 8.2, 4.8 Hz, 1H), 2.20 (s, 0.5H), 1.95 (s, 1H), 1.81 (d, J = 15.7 Hz, 1H), 1.61 (d, J = 16.2 Hz, 2H), 1.15 (dd, J = 16.0, 9.3 Hz, 7H).

**(11) Synthesis of compound BnH12053**

sodium (3R,6S)-6-methyl-1-(2-(4-(1-methyl-1H-imidazol-2-yl)phenyl)acetyl)piperidine-3-carboxylate

Step 1: Synthesis of compound BnH12053

A solution of compound 3-1 (240 mg, 0.598 mmol), 2-bromo-1-methylimidazole (106 mg, 0.658 mmol) and $Na_2CO_3$ (127 mg, 1.20 mmol) in MeCN/$H_2O$ (2:1, 7.72 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 3 hours. The mixture was diluted with $H_2O$ (3.00 mL) and washed with DCM (3.00 mL × 3). To the aqueous layer was concentrated under reduced pressure and purifiyed by column chromatography on $C_{18}$-$SiO_2$ (MeOH:$H_2O$ = 1:1) to afford compound BnH12053 (163 mg, 75 %) as a white solid.

$^1$H NMR (400 MHz, DMSO-d6) δ 7.65 - 7.57 (m, 2H), 7.33 (d, J = 7.9 Hz, 1H), 7.28 (d, J = 7.9 Hz, 1H), 7.23 (s, 1H), 6.95 (s, 1H), 4.72 (s, 0.5H), 4.46 (dd, J = 14.6, 3.8 Hz, 0.5H), 4.20 (d, J = 5.7 Hz, 0.5H), 3.86 - 3.66 (m, 5.5H), 2.96 (t, J = 12.9 Hz, 0.5H), 2.55 (t, J = 12.9 Hz, 0.5H), 1.88 - 1.66 (m, 2H), 1.51 (tq, J = 31.1, 16.4, 13.9 Hz, 3H), 1.05 (t, J = 7.2 Hz, 3H).

**(12) Synthesis of compound BnH12054**

(3R,6S)-6-methyl-1-(2-(4-(pyrimidin-4-yl)phenyl)acetyl)piperidine-3-carboxylic acid

Step 1: Synthesis of compound BnH12054

A solution of compound 3-1 (240 mg, 0.598 mmol), 4-chloropyrimidine (99.3 mg, 0.658 mmol) and $Na_2CO_3$ (190 mg, 1.79 mmol) in MeCN/$H_2O$ (2:1, 7.72 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 1-2 and the mixture was extracted with EtOAc (3.00 mL × 3). The combined organic layer was dried over $MgSO_4$, filtered, and concentrated under reduced pressure. The residue was purified by column chromatography on $SiO_2$ (5% to 10 % MeOH in DCM) to afford compound BnH12054 (83.4 mg, 41 %) as

a white solid.

$^1H$ NMR (400 MHz, DMSO-d6) δ 12.44 (s, 1H), 9.23 (s, 1H), 8.85 (d, J = 5.4 Hz, 1H), 8.17 (d, J = 8.0 Hz, 2H), 8.08 (d, J = 5.4 Hz, 1H), 7.40 (dd, J = 18.3, 7.9 Hz, 2H), 4.75 (s, 0.5H), 4.54 (dd, J = 14.0, 4.2 Hz, 0.5H), 4.28 (d, J = 8.3 Hz, 0.5H), 3.96 - 3.73 (m, 2.5H), 3.03 (t, J = 12.9 Hz, 0.5H), 2.62 (t, J = 12.7 Hz, 0.5H), 2.19 (tt, J = 12.0, 4.1 Hz, 1H), 1.79 (dt, J = 13.4, 4.5 Hz, 1H), 1.67 (tt, J = 17.4, 8.0 Hz, 1H), 1.51 (tq, J = 9.3, 4.6, 4.0 Hz, 2H), 1.08 (q, J = 8.3, 7.8 Hz, 3H).

**(13) Synthesis of compound BnH12055**

(3R,6S)-6-methyl-1-(2-(4-(thiophen-2-yl)phenyl)acetyl)piperidine-3-carboxylic acid

Step 1: Synthesis of compound BnH12055

A solution of compound 3-1 (240 mg, 0.598 mmol), 2-bromothiophene (107 mg, 0.658 mmol) and $Na_2CO_3$ (190 mg, 1.79 mmol) in MeCN/$H_2O$ (2:1, 7.72 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 1-2 and the mixture was extracted with EtOAc (3.00 mL × 3). The combined organic layer was dried over $MgSO_4$, filtered, and concentrated under reduced pressure. The residue was purified by column chromatography on $SiO_2$ (5% MeOH in DCM) to afford compound BnH12055 (30 mg, 14 %) as an ivory solid.

$^1H$ NMR (400 MHz, Chloroform-d) δ 7.57 (d, J = 7.9 Hz, 2H), 7.26 (d, J = 3.4 Hz, 4H), 7.07 (dd, J= 5.1, 3.6 Hz, 1H), 4.98 (s, 0.5H), 4.83 (d, J= 13.6 Hz, 0.5H), 4.18 (s, 0.5H), 3.93 (d, J = 14.1 Hz, 0.5H), 3.75 (s, 2H), 3.15 (t, J = 13.1 Hz, 0.5H), 2.78 (t, J = 12.9 Hz, 0.5H), 2.40 (s, 0.5H), 2.15 (s, 0.5H), 1.92 (d, J = 12.7 Hz, 1H), 1.79 (d, J = 12.3 Hz, 1H), 1.57 (d, J = 27.2 Hz, 2H), 1.14 (d, J = 6.9 Hz, 3H).

**(14) Synthesis of compound BnH12056**

(3R,6S)-1-(2-(4-(furan-2-yl)phenyl)acetyl)-6-methylpiperidine-3-carboxylic acid

Step 1: Synthesis of compound BnH12056

A solution of compound 3-1 (240 mg, 0.598 mmol), 5-bromofuran (96.7 mg, 0.658 mmol) and $Na_2CO_3$ (190 mg, 1.79 mmol) in MeCN/$H_2O$ (2:1, 7.72 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 3 hours. The mixture was diluted with $H_2O$ (3.00 mL) and washed with DCM (3.00 mL × 3). To the aqueous layer 1N HCl was added to adjust the pH value to pH 1-2 and the mixture was extracted with EtOAc (3.00 mL × 3). The combined organic layer was dried over $MgSO_4$, filtered, and concentrated under reduced pressure. The residue was purified by column chromatography on $SiO_2$ (5% MeOH in DCM) to afford compound BnH12056 (109 mg, 55 %) as an ivory solid.

$^1H$ NMR (400 MHz, Chloroform-d) δ 7.63 (d, J = 8.0 Hz, 2H), 7.48 - 7.43 (m, 1H), 7.27 (d, J = 6.0 Hz, 2H), 6.63 (d, J = 3.3 Hz, 1H), 6.46 (dd, J = 3.4, 1.8 Hz, 1H), 4.97 (s, 0.5H), 4.82 (d, J = 13.6 Hz, 0.5H), 4.17 (s, 0.5H), 3.92 (d, J = 13.7 Hz, 0.5H), 3.76 (s, 2H), 3.13 (t, J = 13.0 Hz, 0.5H), 2.78 (t, J = 12.8 Hz, 0.5H), 2.39 (s, 0.5H), 2.11 (s, 0.5H), 1.91 (d, J = 12.7 Hz, 1H), 1.77 (d, J = 13.0 Hz, 1H), 1.67 - 1.40 (m, 2H), 1.13 (d, J = 8.7 Hz, 3H).

**(15) Synthesis of compound BnH12072**

(3R,6S)-6-methyl-1-(2-(3'-nitro-[1,1'-biphenyl]-4-yl)acetyl)piperidine-3-carboxylic acid

Step 1: Synthesis of compound BnH12072

A solution of compound 3-1 (150 mg, 0.374 mmol), 3-bromonitrobenzene (75.5 mg, 0.374 mmol) and $Na_2CO_3$ (79.2 mg, 0.4748 mmol) in MeCN/$H_2O$ (2:1, 4.82 mL) was stirred vigorously and bubbled with $N_2$ gas for 5 minutes and the Pd(dppf)$Cl_2$ (27.4 mg, 0.037 mmol) was added. The mixture was bubbled with $N_2$ gas for 5 minutes and refluxed at 90 °C for 3 hours. Then, the mixture was cooled to room temperature, 1N NaOH (0.748 mL, 0.748 mmol) was added to the mixture and stirred at room temperature for 17 hours. The mixture was diluted with $H_2O$ (4.00 mL) and washed with EtOAc (4.00 mL × 3). To the aqueous layer 1N HCl was added to adjust the pH value to pH 3~4 and the mixture was extracted with EtOAc

(4.00 mL × 3). The combined organic layer was dried over $MgSO_4$, filtered, and concentrated under reduced pressure. The residue was purified by column chromatography on $SiO_2$ (3% to 5% MeOH in DCM) to afford compound BnH12072 (120 mg, 84 %) as an off-white solid.

$^1H$ NMR (400 MHz, DMSO-d6) δ 8.44 (t, J = 2.0 Hz, 1H), 8.25 - 8.13 (m, 2H), 7.77 (t, J = 8.1 Hz, 3H), 7.38 (dd, J = 18.2, 7.9 Hz, 2H), 4.75 (s, 0.5H), 4.55 (d, J = 13.3 Hz, 0.5H), 4.29 (s, 0.5H), 3.94 (s, 0.5H), 3.85 - 3.73 (m, 2H), 3.05 (t, J = 12.8 Hz, 0.5H), 2.67 - 2.57 (t, J = 12.8 Hz, 0.5H), 2.20 (s, 1H), 1.80 (dd, J = 18.3, 6.8 Hz, 1H), 1.67 (d, J = 13.3 Hz, 1H), 1.54 (s, 2H), 1.08 (s, 3H).

**(16) Synthesis of compound BnH12077**

(3R,6S)-6-methyl-1-(2-(4'-nitro-[1,1'-biphenyl]-4-yl)acetyl)piperidine-3-carboxylic acid

Step 1: Synthesis of compound BnH12077

A solution of compound 3-1 (150 mg, 0.374 mmol), 4-bromonitrobenzene (75.5 mg, 0.374 mmol) and $Na_2CO_3$ (79.2 mg, 0.748 mmol) in MeCN/$H_2O$ (2:1, 4.82 mL) was stirred vigorously and bubbled with $N_2$ gas for 5 minutes and the Pd(dppf)$Cl_2$ (27.4 mg, 0.037 mmol) was added. The mixture was bubbled with $N_2$ gas for 5 minutes and refluxed at 90 °C for 3 hours. Then, the mixture was cooled to room temperature, 1N NaOH (0.748 mL, 0.748 mmol) was added to the mixture and stirred at room temperature for 17 hours. The mixture was diluted with $H_2O$ (4.00 mL) and washed with EtOAc (4.00 mL × 3). To the aqueous layer 1N HCl was added to adjust the pH value to pH 3-4 and the mixture was extracted with EtOAc (4.00 mL × 3). The combined organic layer was dried over $MgSO_4$, filtered, and concentrated under reduced pressure. The residue was purified by column chromatography on $SiO_2$ (5% to 10% MeOH in DCM) to afford compound BnH12077 (107 mg, 75 %) as a brown solid.

$^1H$ NMR (400 MHz, DMSO-d6) δ 8.30 (d, J = 8.3 Hz, 2H), 7.97 (d, J = 8.4 Hz, 2H), 7.75 (d, J = 7.8 Hz, 2H), 7.38 (dd, J = 18.5, 7.8 Hz, 2H), 4.75 (s, 0.5H), 4.54 (dd, J = 13.1, 4.0 Hz, 0.5H), 4.29 (s, 0.5H), 3.92 (d, J = 12.9 Hz, 0.5H), 3.82 (q, J = 11.9, 8.6 Hz, 2H), 3.04 (t, J = 12.9 Hz, 0.5H), 2.62 (t, J = 12.8 Hz, 0.5H), 2.19 (d, J = 10.1 Hz, 1H), 1.79 (q, J = 9.5, 6.7 Hz, 1H), 1.66 (d, J = 13.2 Hz, 1H), 1.57 - 1.49 (m, 2H), 1.15 - 1.08 (m, 3H).

**(17) Synthesis of compound BnH12081**

(3R,6S)-1-(2-(4-(6-methoxypyridin-3-yl)phenyl)acetyl)-6-methylpiperidine-3-carboxylic acid

Step 1: Synthesis of compound BnH12081

A solution of compound 3-1 (220 mg, 0.548 mmol), 5-bromo-2-methoxypyridine (0.071 mL, 0.548 mmol) and $Na_2CO_3$ (116 mg, 1.10 mmol) in MeCN/$H_2O$ (2:1, 7.07 mL) was stirred vigorously and bubbled with $N_2$ gas for 5 minutes and the Pd(dppf)$Cl_2$ (40.1 mg, 0.055 mmol) was added. The mixture was bubbled with $N_2$ gas for 5 minutes and refluxed at 90 °C for 3 hours. Then, the mixture was cooled to room temperature, 1N NaOH (1.10 mL, 1.10 mmol) was added to the mixture and stirred at room temperature for 17 hours. The mixture was diluted with $H_2O$ (7.00 mL) and washed with EtOAc (5.00 mL × 3). To the aqueous layer 1N HCl was added to adjust the pH value to pH 3~4 and the mixture was extracted with EtOAc (5.00 mL × 3). The combined organic layer was dried over $MgSO_4$, filtered, and concentrated under reduced pressure. The residue was purified by column chromatography on $SiO_2$ (10% MeOH in DCM) to afford compound BnH12081 (107 mg, 75 %) as a white solid.

$^1H$ NMR (400 MHz, DMSO-d6) δ 12.37 (s, 1H), 8.47 (d, J = 2.5 Hz, 1H), 8.00 (dd, J = 8.6, 2.6 Hz, 1H), 7.60 (d, J = 7.9 Hz, 2H), 7.30 (dd, J = 18.4, 7.8 Hz, 2H), 6.90 (d, J = 8.6 Hz, 1H), 4.75 (s, 0.5H), 4.54 (d, J = 13.5 Hz, 0.5H), 4.28 (s, 0.5H), 3.89 (s, 0.5H), 3.84 - 3.68 (m, 2H), 3.03 (t, J = 12.9 Hz, 0.5H), 2.67 - 2.56 (m, 0.5H), 2.19 (s, 1H), 1.85 - 1.74 (m, 1H), 1.66 (d, J = 13.1 Hz, 1H), 1.52 (s, 2H), 1.10 - 1.04 (m, 3H).

**(18) Synthesis of compound BnH12082**

(3R,6S)-1-(2-(4-(6-cyclopropylpyridin-3-yl)phenyl)acetyl)-6-methylpiperidine-3-carboxylic acid

Step 1: Synthesis of compound BnH12082

A solution of compound 3-1 (200 mg, 0.498 mmol), 3-bromo-6-(cyclopropyl)pyridine (0.060 mL, 0.498 mmol) and $Na_2CO_3$ (106 mg, 0.997 mmol) in MeCN/$H_2O$ (2:1, 6.42 mL) was stirred vigorously and bubbled with $N_2$ gas for 5 minutes and the Pd(dppf)$Cl_2$ (36.5 mg, 0.050 mmol) was added. The mixture was bubbled with $N_2$ gas for 5 minutes and refluxed at 90 °C for 2 hours. Then, the mixture was cooled to room temperature, 1N NaOH (1.10 mL, 1.10 mmol) was added to the

mixture and stirred at room temperature for 17 hours. The mixture was diluted with $H_2O$ (6.00 mL) and washed with EtOAc (4.00 mL × 3). To the aqueous layer 1N HCl was added to adjust the pH value to pH 1-2 and the mixture was washed with EtOAc (4.00 mL × 2). To the aqueous layer 1N NaOH was added to adjust the pH value to pH 4-5 and the mixture was extracted with EtOAc (4.00 mL × 3). The combined organic layer was dried over $MgSO_4$, filtered, and concentrated under reduced pressure to afford compound BnH12082 (120 mg, 64 %) as a white solid.

$^1$H NMR (400 MHz, DMSO-d6) δ 12.30 (s, 1H), 8.70 (d, J = 2.4 Hz, 1H), 7.92 (dd, J = 8.1, 2.4 Hz, 1H), 7.63 (d, J = 7.8 Hz, 2H), 7.40 - 7.26 (m, 3H), 4.75 (s, 0.5H), 4.59 - 4.50 (m, 1=0.5H), 4.29 (s, 0.5H), 3.93 (d, J = 11.1 Hz, 0.5H), 3.85 - 3.69 (m, 2H), 3.04 (t, J = 12.8 Hz, 0.5H), 2.68 - 2.57 (t, J = 12.8 Hz, 0.5H), 2.31 - 2.04 (m, 2H), 1.80 (dd, J = 13.2, 3.6 Hz, 1H), 1.66 (d, J = 12.5 Hz, 1H), 1.58 - 1.43 (m, 2H), 1.14 - 1.03 (m, 3H), 0.96 (td, J = 7.3, 6.5, 2.5 Hz, 4H).

**(19) Synthesis of compound BnH12083**

(3R,6S)-6-methyl-1-(2-(4-(6-(trifluoromethyl)pyridin-3-yl)phenyl)acetyl)piperidine-3-carboxylic acid

Step 1: Synthesis of compound BnH12083

A solution of compound 3-1 (240 mg, 0.598 mmol), 5-bromo-2-trifluoromethylpyridine (135 mg, 0.598 mmol) and $Na_2CO_3$ (127 mg, 1.20 mmol) in MeCN/$H_2O$ (2:1, 7.71 mL) was stirred vigorously and bubbled with $N_2$ gas for 5 minutes and the Pd(dppf)$Cl_2$ (43.8 mg, 0.060 mmol) was added. The mixture was bubbled with $N_2$ gas for 5 minutes and refluxed at 90 °C for 3 hours. Then, the mixture was cooled to room temperature, 1N NaOH (1.20 mL, 1.20 mmol) was added to the mixture and stirred at room temperature for 17 hours. The mixture was diluted with $H_2O$ (7.00 mL) and washed with EtOAc (5.00 mL × 3). To the aqueous layer 1N HCl was added to adjust the pH value to pH 3~4 and the mixture was extracted with EtOAc (5.00 mL × 3). The combined organic layer was dried over $MgSO_4$, filtered, and concentrated under reduced pressure. The residue was purified by column chromatography on $SiO_2$ (10% MeOH in DCM) to afford compound BnH12083 (167 mg, 69 %) as an off-white solid.

$^1$H NMR (400 MHz, DMSO-d6) δ 12.41 (s, 1H), 9.10 (d, J = 2.3 Hz, 1H), 8.37 (dd, J = 8.2, 2.3 Hz, 1H), 7.98 (d, J = 8.2 Hz, 1H), 7.79 (d, J = 8.0 Hz, 2H), 7.40 (dd, J = 18.3, 7.9 Hz, 2H), 4.76 (s, 0.5H), 4.59 - 4.51 (d, J = 13.3 Hz, 0.5H), 4.34 - 4.25 (m, 0.5H), 3.94 (d, J = 13.3 Hz, 0.5H), 3.82 (qd, J = 15.4, 5.8 Hz, 2H), 3.06 (t, J = 12.8 Hz, 0.5H), 2.69 - 2.58 (m, 0.5H), 2.31 - 2.13 (m, 1H), 1.87 - 1.77 (m, 1H), 1.67 (d, J = 11.6 Hz, 1H), 1.54 (dt, J = 6.7, 3.8 Hz, 2H), 1.14 - 1.02 (m, 3H).

**(20) Synthesis of compound BnH12084**

(3R,6S)-6-methyl-1-(2-(4-(6-methylpyridin-3-yl)phenyl)acetyl)piperidine-3-carboxylic acid

Step 1: Synthesis of compound BnH12084

A solution of compound 3-1 (200 mg, 0.498 mmol), 5-bromo-2-methylpyridine (85.7 mg, 0.498 mmol) and $Na_2CO_3$ (106 mg, 0.997 mmol) in MeCN/$H_2O$ (2:1, 6.42 mL) was stirred vigorously and bubbled with $N_2$ gas for 5 minutes and the Pd(dppf)$Cl_2$ (36.5 mg, 0.050 mmol) was added. The mixture was bubbled with $N_2$ gas for 5 minutes and refluxed at 90 °C for 2.5 hours. Then, the mixture was cooled to room temperature, 1N NaOH (0.997 mL, 0.997 mmol) was added to the mixture and stirred at room temperature for 3 hours. The mixture was diluted with $H_2O$ (6.00 mL) and washed with EtOAc (4.00 mL × 3). To the aqueous layer 1N HCl was added to adjust the pH value to pH 3-4 and the mixture was extracted with EtOAc (4.00 mL × 3). The combined organic layer was dried over $MgSO_4$, filtered, and concentrated under reduced pressure. To the residue was added $Et_2O$ (2.00 mL) and stirred at room temperature for 30 minutes. The precipitate was filtered and washed with $Et_2O$ (3.00 mL) to afford compound BnH12084 (92.0 mg, 52 %) as a white solid.

$^1$H NMR (400 MHz, Chloroform-d) δ 8.82 (s, 1H), 8.02 (d, J = 7.9 Hz, 1H), 7.56 (d, J = 7.6 Hz, 2H), 7.41 (d, J = 7.5 Hz, 3H), 5.00 (s, 0.5H), 4.86 (s, 0.5H), 4.23 (s, 0.5H), 3.95 (d, J = 13.6 Hz, 0.5H), 3.84 (s, 2H), 3.18 (t, J = 12.8 Hz, 0.5H), 2.84 (s, 0.5H), 2.74 (s, 3H), 2.44 (s, 0.5H), 2.19 (s, 0.5H), 1.99 (d, J = 13.4 Hz, 1H), 1.82 (s, 1H), 1.64 (s, 2H), 1.17 (d, J = 7.9 Hz, 3H).

**(21) Synthesis of compound BnH12085**

(3R,6S)-1-(2-(4'-cyclohexyl-[1,1'-biphenyl]-4-yl)acetyl)-6-methylpiperidine-3-carboxylic acid

Step 1: Synthesis of compound BnH12085

A solution of compound 3-1 (191 mg, 0.476 mmol), 1-bromo-4-cyclohexylbenzene (0.088 mL, 0.476 mmol) and $Na_2CO_3$ (101 mg, 0.952 mmol) in MeCN/$H_2O$ (2:1, 6.14 mL) was stirred vigorously and bubbled with $N_2$ gas for 5 minutes

and the Pd(dppf)$Cl_2$ (34.8 mg, 0.048 mmol) was added. The mixture was bubbled with $N_2$ gas for 5 minutes and refluxed at 90 °C for 2.5 hours. Then, the mixture was cooled to room temperature, 1N NaOH (1.20 mL, 1.20 mmol) was added to the mixture and stirred at room temperature for 2 hours. The mixture was diluted with $H_2O$ (6.00 mL) and washed with EtOAc (4.00 mL × 3). To the aqueous layer 1N HCl was added to adjust the pH value to pH 2-3 and the mixture was extracted with EtOAc (4.00 mL × 3). The combined organic layer was dried over $MgSO_4$, filtered, and concentrated under reduced pressure. The residue was purified by column chromatography on $SiO_2$ (10% MeOH in DCM). To the residue was added $Et_2O$ (3.00 mL) and stirred at room temperature for 30 minutes. The precipitate was filtered and washed with $Et_2O$ (4.00 mL) to afford compound BnH12085 (127 mg, 64 %) as an off-white solid.

$^1$H NMR (400 MHz, DMSO-d6) δ 12.44 (s, 1H), 7.57 (t, J = 7.0 Hz, 4H), 7.28 (dd, J = 16.4, 7.9 Hz, 4H), 4.75 (s, 0.5H), 4.58 - 4.50 (m, 0.5H), 4.28 (s, 0.5H), 3.92 (d, J = 13.6 Hz, 0.5H), 3.86 - 3.67 (m, 2H), 3.04 (t, J = 12.8 Hz, 0.5H), 2.63 ((t, J = 12.8 Hz, 0.5H), 2.27 - 2.12 (m, 1H), 1.91-1.15 (m, 15H), 1.07 (t, J = 6.4 Hz, 3H).

**(22) Synthesis of compound BnH12086**

(3R,6S)-1-(2-(4'-amino-[1,1'-biphenyl]-4-yl)acetyl)-6-methylpiperidine-3-carboxylic acid

Step 3: Synthesis of compound BnH12086

A solution of compound 3-1 (225 mg, 0.561 mmol), 4-bromoaniline (96.5 mg, 0.561 mmol) and $Na_2CO_3$ (119 mg, 1.12 mmol) in MeCN/$H_2O$ (2:1, 7.01 mL) was stirred vigorously and bubbled with $N_2$ gas for 5 minutes and the Pd(dppf)$Cl_2$ (41.0 mg, 0.06 mmol) was added. The mixture was bubbled with $N_2$ gas for 5 minutes and refluxed at 90 °C for 2 hours. Then, the mixture was cooled to room temperature, 1N NaOH (1.12 mL, 1.12 mmol) was added to the mixture and stirred at room temperature for 4 hours. The mixture was diluted with $H_2O$ (5.00 mL) and washed with EtOAc (5.00 mL × 3). To the aqueous layer 1N HCl was added to adjust the pH value to pH 1-2 and the mixture was washed with EtOAc (5.00 mL × 2). To the aqueous layer 1N NaOH was added to adjust the pH value to pH 5-6 and the mixture was extracted with EtOAc (5.00 mL × 3). The combined organic layer was dried over $MgSO_4$, filtered, and concentrated under reduced pressure. The residue was purified by column chromatography on $SiO_2$ (0 to 10% MeOH in DCM). To the residue was added $Et_2O$ (2.00 mL) and stirred at room temperature for 30 minutes. The precipitate was filtered and washed with $Et_2O$ (2.00 mL) to afford compound BnH12086 (70.0 mg, 35 %) as a beige solid.

$^1$H NMR (400 MHz, DMSO-$d_6$) δ 12.44 (s, 1H), 7.49-7.46 (m, 2H), 7.36-7.34 (d, *J* = 8.2 Hz, 2H), 7.24-7.17 (dd, *J* = 18.4, 7.8 Hz, 2H), 6.64-6.62 (d, *J* = 8.2 Hz, 2H), 5.20 (s, 2H), 4.75 (s, 0.5H), 4.56-4.52 (m, 0.5H), 4.26 (s, 0.5H), 3.92-3.89 (d, *J* = 13.7 Hz, 0.5H), 3.77-3.66 (m, 2H), 3.04-2.98 (t, *J* = 12.8 Hz, 0.5H), 2.64-2.57 (t, *J* = 12.9 Hz, 0.5H), 2.16-2.12 (d, *J* = 13.6 Hz, 1H), 1.80-1.77 (d, *J* = 12.9 Hz, 1H), 1.66-1.63 (d, *J* = 13.0 Hz, 1H), 1.53-1.50 (d, *J* = 10.9 Hz, 2H), 1.07-1.05 (dd, *J* = 6.9, 2.3 Hz, 3H).

**(23) Synthesis of compound BnH12087**

(3R,6S)-6-methyl-1-(2-(4-(6-phenylpyridin-3-yl)phenyl)acetyl)piperidine-3-carboxylic acid

Step 3: Synthesis of compound BnH12087

A solution of compound 3-1 (194 mg, 0.483 mmol), 5-bromo-2-phenylpyridine (113 mg, 0.483 mmol) and $Na_2CO_3$ (102 mg, 0.967 mmol) in MeCN/$H_2O$ (2:1, 6.04 mL) was stirred vigorously and bubbled with $N_2$ gas for 5 minutes and the Pd(dppf)$Cl_2$ (35.4 mg, 0.05 mmol) was added. The mixture was bubbled with $N_2$ gas for 5 minutes and refluxed at 90 °C for 2 hours. Then, the mixture was cooled to room temperature, 1N NaOH (0.967 mL, 0.967 mmol) was added to the mixture and stirred at room temperature for 4 hours. The mixture was diluted with $H_2O$ (5.00 mL) and washed with EtOAc (5.00 mL × 3). To the aqueous layer 1N HCl was added to adjust the pH value to pH 1~2 and the mixture was extracted with EtOAc (4.00 mL × 3). The combined organic layer was dried over $MgSO_4$, filtered, and concentrated under reduced pressure. The residue was purified by column chromatography on $SiO_2$ (0 to 4% MeOH in DCM). To the residue was added $Et_2O$ (2.00 mL) and stirred at room temperature for 30 minutes. The precipitate was filtered and washed with $Et_2O$ (2.00 mL) to afford compound BnH12087 (70.1 mg, 35 %) as an off-white solid.

$^1$H NMR (400 MHz, DMSO-$d_6$) δ 8.99 (s, 1H), 8.18-8.13 (t, *J* = 8.7 Hz, 3H), 8.06-8.04 (d, *J* = 8.4 Hz, 1H), 7.76-7.73 (dd, *J* = 8.0, 4.4 Hz, 2H), 7.53-7.49 (t, *J* = 7.5 Hz, 2H), 7.47-7.43 (t, *J* = 7.2 Hz, 1H), 7.39-7.37 (d, *J* = 7.8 Hz, 1H), 7.34-7.32 (d, *J* = 7.9 Hz, 1H), 4.74 (s, 0.5H), 4.55-4.50 (m, 0.5H), 4.28 (s, 0.5H), 3.92-3.89 (d, *J* = 13.8 Hz, 0.5H), 3.83-3.76 (m, 2H), 3.04-2.98 (t, *J* = 12.9 Hz, 0.5H), 2.63-2.56 (d, *J* = 12.6 Hz, 0.5H), 2.11-2.08 (d, *J* = 10.2 Hz, 1H), 1.80-1.76 (d, *J* = 12.8 Hz, 1H), 1.64-1.61 (m, 1H), 1.51 (s, 2H), 1.10-1.05 (dd, *J* = 14.4, 6.8 Hz, 3H).

**(24) Synthesis of compound BnH12088**

(3R,6S)-1-(2-(3'-amino-[1,1'-biphenyl]-4-yl)acetyl)-6-methylpiperidine-3-carboxylic acid

Step 1: Synthesis of compound BnH12088

A solution of compound 3-1 (210 mg, 0.523 mmol), 3-bromoaniline (0.057 mL, 0.523 mmol) and $Na_2CO_3$ (111 mg, 1.05 mmol) in MeCN/$H_2O$ (2:1, 6.75 mL) was stirred vigorously and bubbled with $N_2$ gas for 5 minutes and the Pd(dppf)$Cl_2$ (38.3 mg, 0.052 mmol) was added. The mixture was bubbled with $N_2$ gas for 5 minutes and refluxed at 90 °C for 2.5 hours. Then, the mixture was cooled to room temperature, 1N NaOH (1.05 mL, 1.05 mmol) was added to the mixture and stirred at room temperature for 3 hours. The mixture was diluted with $H_2O$ (6.00 mL) and washed with EtOAc (4.00 mL × 3). To the aqueous layer 1N HCl was added to adjust the pH value to pH 1-2 and the mixture was washed with EtOAc (4.00 mL × 2). To the aqueous layer 1N NaOH was added to adjust the pH value to pH 5~6 and the mixture was extracted with EtOAc (4.00 mL × 3). The combined organic layer was dried over $MgSO_4$, filtered, and concentrated under reduced pressure. To the residue was added $Et_2O$/n-Hexane (2:1, 2.00 mL) and stirred at room temperature for 30 minutes. The precipitate was filtered and washed with $Et_2O$/n-Hexane (2:1, 3.00 mL) to afford compound BnH12088 (115 mg, 62 %) as a beige solid.

$^1$H NMR (400 MHz, DMSO-d6) δ 12.43 (s, 1H), 7.49 (d, J = 7.7 Hz, 2H), 7.26 (dd, J = 18.0, 7.8 Hz, 2H), 7.08 (t, J = 7.8 Hz, 1H), 6.83 (d, J = 2.1 Hz, 1H), 6.76 (d, J = 7.7 Hz, 1H), 6.54 (dd, J = 7.9, 2.2 Hz, 1H), 5.13 (s, 2H), 4.75 (s, 0.5H), 4.55 (d, J = 13.6 Hz, 0.5H), 4.27 (s, 0.5H), 3.90 (s, 0.5H), 3.84 - 3.61 (m, 2H), 3.03 (t, J = 12.8 Hz, 0.5H), 2.66 (t, J = 12.8 Hz, 0.5H), 2.18 (s, 1H), 1.79 (d, J = 12.7 Hz, 1H), 1.66 (d, J = 12.6 Hz, 1H), 1.52 (d, J = 8.8 Hz, 2H), 1.08 (q, J = 6.5, 6.1 Hz, 3H).

**(25) Synthesis of compound BnH12089**

(3R,6S)-6-methyl-1-(2-(4'-(pyridin-3-yl)-[1,1'-biphenyl]-4-yl)acetyl)piperidine-3-carboxylic acid

Step 1: Synthesis of compound BnH12089

A solution of compound 3-1 (201 mg, 0.501 mmol), 3-(4-Bromophenyl)pyridine (117 mg, 0.501 mmol) and $Na_2CO_3$ (106 mg, 1.00 mmol) in MeCN/$H_2O$ (2:1, 6.46 mL) was stirred vigorously and bubbled with $N_2$ gas for 5 minutes and the Pd(dppf)$Cl_2$ (36.7 mg, 0.050 mmol) was added. The mixture was bubbled with $N_2$ gas for 5 minutes and refluxed at 90 °C for 2 hours. Then, the mixture was cooled to room temperature, 1N NaOH (1.00 mL, 1.00 mmol) was added to the mixture and stirred at room temperature for 3 hours. The mixture was diluted with $H_2O$ (6.00 mL) and washed with EtOAc (4.00 mL × 3). To the aqueous layer 1N HCl was added to adjust the pH value to pH 1~2 and the mixture was washed with EtOAc (4.00 mL × 2). To the aqueous layer 1N NaOH was added to adjust the pH value to pH 4~5 and the mixture was extracted with EtOAc (4.00 mL × 3). The combined organic layer was dried over $MgSO_4$, filtered, and concentrated under reduced pressure. To the residue was added $Et_2O$ (3.00 mL) and stirred at room temperature for 30 minutes. The precipitate was filtered and washed with $Et_2O$ (4.00 mL) to afford compound BnH12089 (127 mg, 61 %) as a white solid.

$^1$H NMR (400 MHz, DMSO-d6) δ 12.43 (s, 1H), 8.96 (d, J = 2.4 Hz, 1H), 8.59 (dd, J = 4.8, 1.6 Hz, 1H), 8.14 (dt, J = 8.0, 2.0 Hz, 1H), 7.84 (t, J = 6.4 Hz, 4H), 7.70 (d, J = 7.8 Hz, 2H), 7.51 (dd, J = 7.9, 4.7 Hz, 1H), 7.34 (dd, J = 18.2, 7.8 Hz, 2H), 4.76 (s, 0.5H), 4.56 (d, J = 13.8 Hz, 0.5H), 4.30 (s, 0.5H), 4.00 - 3.90 (d, J = 13.8 Hz, 0.5H), 3.87 - 3.71 (m, 2H), 3.05 (t, J = 12.9 Hz, 0.5H), 2.68 - 2.58 (t, J = 12.9 Hz, 0.5H), 2.20 (d, J = 3.9 Hz, 1H), 1.81 (d, J = 12.7 Hz, 1H), 1.75 - 1.60 (m, 1H), 1.54 (s, 2H), 1.09 (dd, J = 10.5, 6.8 Hz, 3H).

**(26) Synthesis of compound BnH12090**

(3R,6S)-1-(2-(4'-(diethylamino)-[1,1'-biphenyl]-4-yl)acetyl)-6-methylpiperidine-3-carboxylic acid

Step 1: Synthesis of compound BnH12090

A solution of compound 3-1 (252 mg, 0.628 mmol), 4-bromo-N,N-diethylaniline (143 mg, 0.628 mmol) and $Na_2CO_3$ (106 mg, 1.00 mmol) in MeCN/$H_2O$ (2:1, 7.85 mL) was stirred vigorously and bubbled with $N_2$ gas for 5 minutes and the Pd(dppf)$Cl_2$ (46.0 mg, 0.06 mmol) was added. The mixture was bubbled with $N_2$ gas for 5 minutes and refluxed at 90 °C for 2 hours. Then, the mixture was cooled to room temperature, 1N NaOH (1.00 mL, 1.00 mmol) was added to the mixture and stirred at room temperature for 4 hours. The mixture was diluted with $H_2O$ (6.00 mL) and washed with EtOAc (4.00 mL × 3). To the aqueous layer 1N HCl was added to adjust the pH value to pH 4~5 and the mixture was extracted with EtOAc (4.00 mL × 3). The combined organic layer was dried over $MgSO_4$, filtered, and concentrated under reduced pressure. The residue was purified by column chromatography on $SiO_2$ (5 to 10 % MeOH in DCM). To the residue was added $Et_2O$ (2.00 mL) and stirred at room temperature for 30 minutes. The precipitate was filtered and washed with $Et_2O$

(3.00 mL) to afford compound BnH12090 (55.0 mg, 21 %) as an off-white solid.

$^1$H NMR (400 MHz, DMSO-$d_6$) δ 12.47 (s, 1H), 7.52-7.47 (dd, *J* = 13.4, 7.9 Hz, 4H), 7.26-7.19 (dd, *J* = 18.3, 7.8 Hz, 2H), 6.74-6.72 (d, *J* = 8.6 Hz, 2H), 4.75 (s, 0.5H), 4.57-4.53 (d, *J* = 13.9 Hz, 0.5H), 4.28 (s, 0.5H), 3.94-3.90 (d, *J* = 14.1 Hz, 0.5H), 3.78-3.67 (m, 2H), 3.48-3.34 (m, 4H), 3.05-2.99 (t, *J* = 12.8 Hz, 0.5H), 2.64-2.58 (t, *J* = 12.8 Hz, 0.5H), 2.20-2.14 (m, 1H), 1.81-1.77 (m, 1H), 1.70-1.64 (d, *J* = 12.6 Hz, 1H), 1.53-1.51 (d, *J* = 9.3 Hz, 2H), 1.13-1.10 (t, *J* = 6.9 Hz, 6H), 1.08-1.05 (dd, *J* = 7.1, 2.9 Hz, 3H).

**Preparation Example 3. Preparation of active compounds according to representative reaction scheme 4**

[Reaction scheme 4]

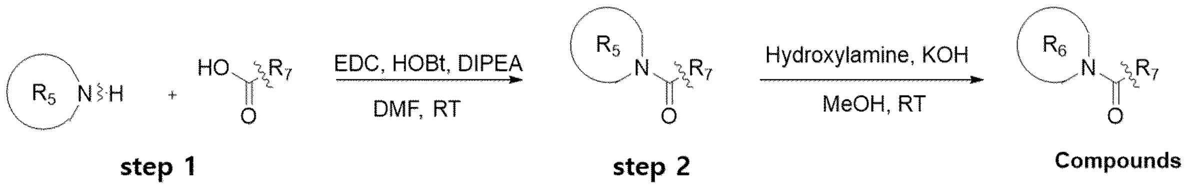


| Compound | R5 | R6 | R7 |
|---|---|---|---|
| BnH12015 | 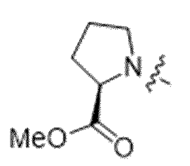 | 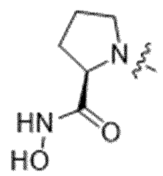 | 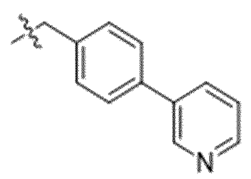 |
| BnH12017 | 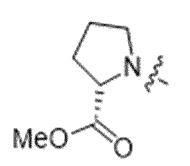 | 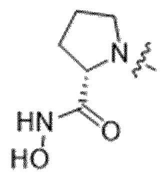 | 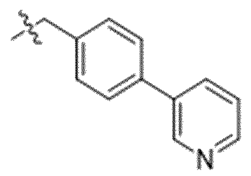 |

**(1) Synthesis of compound BnH12015**

(R)-N-hydroxy-1-(2-(4-(pyrimidin-5-yl)phenyl)acetyl)pyrrolidine-2-carboxamide

Step 1: Synthesis of 2-(4-(pyrimidin-5-yl)phenyl)acetic acid

A suspension of 4-bromophenylacetic acid (1.00 g, 4.65 mmol), 5-Pyrimidineboronic acid pinacol ester (910 mg, 4.42 mmol) and $Na_2CO_3$ (986 mg, 9.30 mmol) in MeCN/$H_2O$ (2:1, 60.0 mL) was stirred vigorously and bubbled with nitrogen gas for 5 minutes then Pd(dppf)$Cl_2$ (340 mg, 0.465 mmol) was added. The mixture was bubbled with nitrogen gas for 5 minutes and refluxed at 90 °C for 2 hours. After completion of reaction, the reaction mixture was filtered through a celite pad and washed with EtOAc (10.0 mL × 2). The filtrate was diluted with $H_2O$ (40.0 mL) and washed with EtOAc (40.0 mL× 2). The aqueous layer was acidified with 1N HCl until pH 1~2 and stirred for 30 minutes. The precipitation was filtered and washed with $H_2O$ (40.0 mL). The filter cake was dried under reduced pressure to afford desired compound (542 mg, 54 %) as a white solid.

Step 2: Synthesis of methyl (2-(4-(pyrimidin-5-yl)phenyl)acetyl)-D-prolinate

To a 2-(4-(pyrimidin-5-yl)phenyl)acetic acid (110 mg, 0.513 mmol) in DMF (1.71 mL) were added methyl D-prolinate (10.2 mg, 0.616 mmol), DIPEA(0.135 mL, 0.770 mmol), HOBt (83.3 mg, 0.616 mmol) and EDC·HCl (95.7 mg, 0.616 mmol). The mixture was stirred at room temperature for 3 hours 3 minutes. The mixture was diluted with EtOAc (5.00 mL), washed with sat. aq. $NH_4Cl$ (5.00 mL) and brine (5.00 mL). The organic layer was dried over $MgSO_4$, filtered, and

concentrated under reduced pressure. The residue was purified by column chromatography on $SiO_2$ (*n*-Hexane : EtOAc = 2 : 1) to afford desired compound (145 mg, 79 %) as a colorless oil.

Step 3: Synthesis of compound BnH12015

To a solution of methyl (2-(4-(pyrimidin-5-yl)phenyl)acetyl)-D-prolinate (83.0 mg, 0.255 mmol) in THF/MeOH (1:1, 3.00 mL) were added hydroxylamine hydrochloride (169 mg, 2.55 mmol) and KOH (28.6 mg, 0.510 mmol). The mixture was stirred at room temperature for 2 hours. The mixture was diluted with $H_2O$ (2.00 mL) and washed two times with DCM (3.00 mL × 2). The aqueous layer was acidified with 1N HCl until pH 1~2 at 0 °C and extracted with EtOAc (3.00 mL). The organic layer was washed with brine (3.00 mL), dried over $MgSO_4$, filtered, and concentrated under reduced pressure. The residue was purified by column chromatography on $SiO_2$ (DCM : MeOH = 9 : 1) to afford desired compound BnH12015 (48.0 mg, 57 %) as a white solid.

$^1H$ NMR (400 MHz, Chloroform-d) δ 9.90 (s, 1H), 9.21 (s, 1H), 8.94 (s, 2H), 7.60 - 7.53 (m, 2H), 7.40 (d, J = 8.0 Hz, 2H), 4.55 (d, J = 8.0 Hz, 1H), 3.75 (s, 2H), 3.65 - 3.59 (m, 1H), 3.52 (q, J = 8.7 Hz, 1H), 2.49 (s, 1H), 2.25 - 2.12 (m, 1H), 2.00 (d, J = 38.2 Hz, 2H).

**(2) Synthesis of compound BnH12017**

Step 1: Synthesis of methyl (2-(4-(pyrimidin-5-yl)phenyl)acetyl)-D-prolinate

To a 2-(4-(pyrimidin-5-yl)phenyl)acetic acid (110 mg, 0.513 mmol) in DMF (1.71 mL) were added methyl L-prolinate (10.2 mg, 0.616 mmol), DIPEA (0.135 mL, 0.770 mmol), HOBt (83.3 mg, 0.616 mmol) and EDC·HCl (95.7 mg, 0.616 mmol). The mixture was stirred at room temperature for 3 hours 3 minutes. The mixture was diluted with EtOAc (5.00 mL), washed with sat. aq. $NH_4Cl$ (5.00 mL) and brine (5.00 mL). The organic layer was dried over $MgSO_4$, filtered, and concentrated under reduced pressure. The residue was purified by column chromatography on $SiO_2$ (*n*-Hexane : EtOAc = 2 : 1) to afford desired compound (83 mg, 49 %) as a colorless oil.

Step 2: Synthesis of compound BnH12017

To a solution of methyl (2-(4-(pyrimidin-5-yl)phenyl)acetyl)-D-prolinate (80.0 mg, 0.246 mmol) in THF/MeOH (1:1, 3.00 mL) were added hydroxylamine hydrochloride (162 mg, 2.46 mmol) and KOH (27.6 mg, 0.492 mmol). The mixture was stirred at room temperature for 2 hours. The mixture was diluted with $H_2O$ (2.00 mL) and washed two times with DCM (3.00 mL × 2). The aqueous layer was acidified with 1N HCl until pH 1~2 at 0 °C and extracted with EtOAc (3.00 mL). The organic layer was washed with brine (3.00 mL), dried over $MgSO_4$, filtered, and concentrated under reduced pressure. The residue was purified by column chromatography on $SiO_2$ (DCM : MeOH = 9 : 1) to afford desired compound BnH12017 (52.0 mg, 64 %) as a white solid.

$^1H$ NMR (400 MHz, Chloroform-d) δ 9.87 (s, 1H), 7.35 - 7.27 (m, 2H), 7.15 (dd, J = 8.4, 2.6 Hz, 2H), 6.99 (td, J = 8.3, 2.7 Hz, 2H), 4.54 (d, J = 8.1 Hz, 1H), 3.73 (d, J = 2.6 Hz, 2H), 3.66 - 3.49 (m, 1H), 2.46 (s, 1H), 2.17 (dd, J = 17.6, 8.5 Hz, 1H), 2.09 - 1.89 (m, 2H).

**Preparation Example 4. Preparation of active compounds according to representative reaction scheme 5**

[Reaction scheme 5]

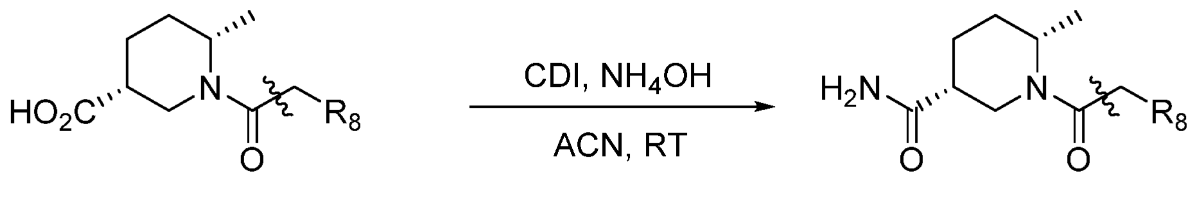


Compounds

Compounds

| Compound | $R_8$ | Compound | $R_8$ |
|---|---|---|---|
| BnH12058 | 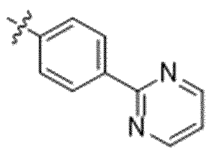 | BnH12059 | 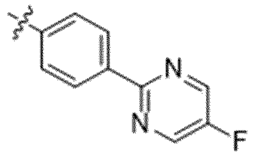 |

**(1) Synthesis of compound BnH12058**

(3R,6S)-6-methyl-1-(2-(4-(pyrimidin-2-yl)phenyl)acetyl)piperidine-3-carboxamide

Step 1: Synthesis of compound BnH12058

To a suspension of compound BnH12048 (100 mg, 0.295 mmol) in MeCN (3.17 mL) was added CDI (71.7 mg, 0.442 mmol) and stirred at room temperature for 20 minutes. To the mixture $NH_4OH$ (0.172 mL, 4.42 mmol) was added and the mixture was stirred at room temperature for 17 hours. The mixture was diluted with EtOAc (4.00 mL) and washed with sat. $NH_4Cl$ (4.00 mL), 10% citric acid (4.00 mL) and brine (4.00 mL). The organic layer was dried over $MgSO_4$, filtered and concentrated. The residue was purified by column chromatography (5% MeOH in DCM) to afford compound BnH12058 (48 mg, 48%) as a white solid.

$^1H$ NMR (400 MHz, DMSO-d6) δ 8.97 (d, J = 0.8 Hz, 2H), 8.31 - 8.24 (m, 2H), 7.43 - 7.29 (m, 3H), 6.86 (d, J = 9.7 Hz, 1H), 4.74 (d, J = 6.7 Hz, 0.5H), 4.38 (dd, J = 13.3, 4.2 Hz, 0.5H), 4.30 - 4.24 (m, 0.5H), 3.87 - 3.75 (m, 2.5H), 3.02 (dd, J = 13.7, 11.9 Hz, 0.5H), 2.61 (q, J = 14.0, 13.0 Hz, 0.5H), 2.08 (ddd, J = 16.4, 10.5, 3.1 Hz, 1H), 1.68 (td, J = 13.0, 11.4, 4.6 Hz, 2H), 1.56 - 1.39 (m, 2H), 1.12 - 1.02 (m, 3H).

**(2) Synthesis of compound BnH12059**

(3R,6S)-1-(2-(4-(5-fluoropyrimidin-2-yl)phenyl)acetyl)-6-methylpiperidine-3-carboxamide

Step 1: Synthesis of compound BnH12059

To a suspension of compound BnH12049 (100 mg, 0.280 mmol) in MeCN (3.01 mL) was added CDI (68.1 mg, 0.420 mmol) and stirred at room temperature for 20 minutes. To the mixture $NH_4OH$ (0.163 mL, 4.20 mmol) was added and the mixture was stirred at room temperature for 17 hours. The mixture was diluted with EtOAc (4.00 mL) and washed with sat. $NH_4Cl$ (4.00 mL), 10% citric acid (4.00 mL) and brine (4.00 mL). The organic layer was dried over $MgSO_4$, filtered and concentrated. The residue was purified by column chromatography (5% MeOH in DCM) to afford compound BnH12059 (52 mg, 52%) as a white solid.

$^1H$ NMR (400 MHz, DMSO-d6) δ 8.89 (d, J = 4.9 Hz, 2H), 8.34 (dd, J = 8.4, 2.3 Hz, 2H), 7.47 - 7.29 (m, 4H), 6.86 (d, J = 9.5 Hz, 1H), 4.75 (s, 0.5H), 4.38 (dd, J = 13.0, 4.2 Hz, 0.5H), 4.25 (d, J = 6.6 Hz, 0.5H), 3.95 - 3.72 (m, 2.5H), 3.02 (t, J = 12.8 Hz, 0.5H), 2.63 (t, J = 12.7 Hz, 0.5H), 2.09 (dd, J = 13.6, 9.5 Hz, 1H), 1.66 (d, J = 14.3 Hz, 2H), 1.58 - 1.36 (m, 2H), 1.13 - 1.02 (m, 3H).

**Preparation Example 5. Preparation of active compounds according to representative reaction scheme 6**

[Reaction scheme 6]

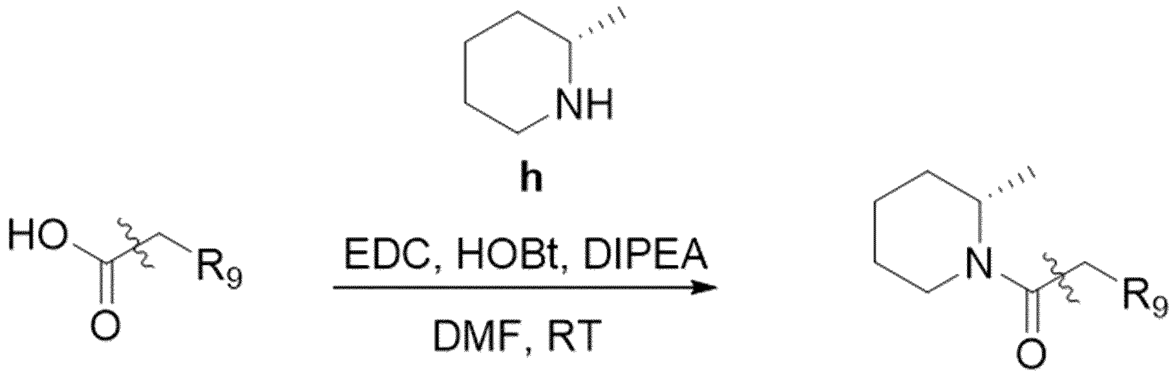


step 1 compounds

| Compound | $R_9$ |
| --- | --- |
| BnH12064 | 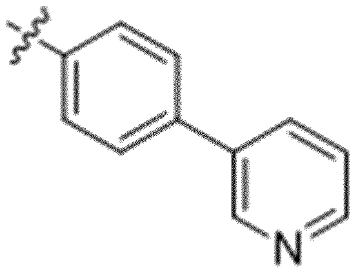 |

**(1) Synthesis of compound BnH12064**

(S)-1-(2-methylpiperidin-1-yl)-2-(4-(pyridin-3-yl)phenyl)ethan-1-one

Step 1: Synthesis of 2-(4-(4,4,5,5-tetramethyl-1,3,2-dioxaborolan-2-yl)phenyl)acetic acid

A suspension of compound 2-(4-bromophenyl)acetic acid (700 mg, 3.26 mmol), Bis(pinacolato)diborane (911 mg, 3.59 mmol) and KOAc (958.4 mg, 9.77 mmol) in 1,4-dioxane (11.2 mL) was stirred vigorously and bubbled with $N_2$ gas for 5 minutes then Pd(dppf)$Cl_2$ (238 mg, 0.326 mmol) was added. The mixture was bubbled with $N_2$ gas for 5 minutes and refluxed at 100 °C for 17 hours. After completion of reaction, the reaction mixture was filtered through a celite pad and washed with DCM (5.00 mL × 3). The combined organic layer was washed with $H_2O$ (20.0 mL), dried over $MgSO_4$, filtered and concentrated under reduced pressure. The residue was purified by column chromatography on $SiO_2$ (n-Hexane : EtOAc = 3 : 1 to 2 : 1) to afford compound 6-1-BnH12064-1 (552 mg, 65 %) as a yellow solid.

Step 2: Synthesis of 2-(4-(pyridin-3-yl)phenyl)acetic acid (6-1-BnH12064)

A solution of compound 6-1-BnH12064-1 (450 mg, 1.72 mmol), 3-bromopyridine (0.182 mL, 1.89 mmol) and $Na_2CO_3$ (546 mg, 5.15mmol) in MeCN/$H_2O$ (2:1, 21.4 mL) was stirred vigorously and bubbled with $N_2$ gas for 5 minutes and the Pd(dppf)$Cl_2$ (126 mg, 0.172 mmol) was added. The mixture was bubbled with $N_2$ gas for 5 minutes and refluxed at 90 °C for 3 hours. Then, the mixture was cooled to room temperature. The mixture was diluted with $H_2O$ (10.0 mL) and washed with DCM (10.0 mL × 3). To the aqueous layer 1N HCl was added to adjust the pH value to pH 1-2 and the mixture was extracted with EtOAc (15.0 mL × 3). The combined organic layer was dried over $MgSO_4$, filtered, and concentrated

under reduced pressure and used without further purification.

Step 3: Synthesis of compound BnH12064

To a solution of 2-(4-(pyridin-3-yl)phenyl)acetic acid (6-1-BnH12064, 90 mg, 0.422 mmol) in DMF (1.41 mL) were added (S)-2-methylpiperidine (44.0 mg, 0.443 mmol), DIPEA (0.110 mL, 0.633 mmol), HOBt (68.4 mg, 0.506 mmol) and EDC·HCl (78.6 mg, 0.506 mmol). The mixture was stirred at room temperature for 3 hours. The mixture was diluted with EtOAc (2.00 mL), washed with sat. aq. $NH_4Cl$ (2.00 mL) and brine (2.00 mL). The organic layer was dried over $MgSO_4$, filtered, and concentrated under reduced pressure. The residue was purified by column chromatography on SiO2 (n-Hexane: EtOAc = 1 : 1 to 0 : 1) to afford compound BnH12064 (46.6 mg, 38 %) as a yellowish oil.

$^1$H NMR (400 MHz, Chloroform-d) δ 8.88 (d, J = 2.3 Hz, 1H), 8.62 (dd, J = 5.0, 1.6 Hz, 1H), 8.00 (dt, J = 8.0, 2.0 Hz, 1H), 7.57 (d, J = 7.9 Hz, 2H), 7.47 (dd, J = 8.0, 4.9 Hz, 1H), 7.39 (d, J = 8.9 Hz, 2H), 4.99 (s, 0.5H), 4.58 (d, J = 13.7 Hz, 0.5H), 4.22 (s, 0.5H), 3.80 (s, 2H), 3.68 (d, J = 13.8 Hz, 0.5H), 3.11 (t, J = 13.5 Hz, 0.5H), 2.72 (t, J = 13.6 Hz, 0.5H), 1.58 (d, J = 31.7 Hz, 6H), 1.18 (d, J = 6.9 Hz, 3H).

**Preparation Example 6. Preparation of active compounds according to representative reaction scheme 7 (absolute form)**

[Reaction scheme 7]

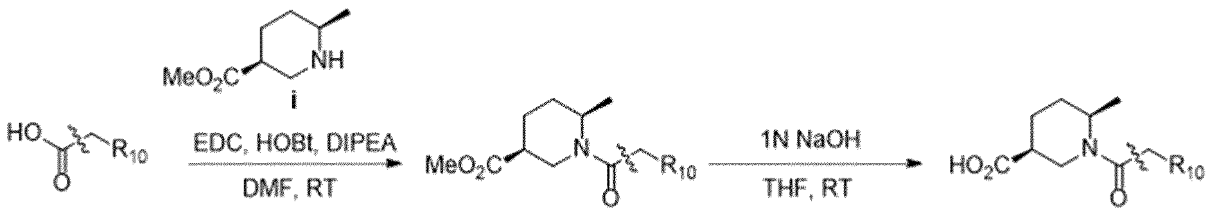


Step 1 Step 2 Compounds

| Compound | $R_{10}$ |
|---|---|
| BnH12066 | 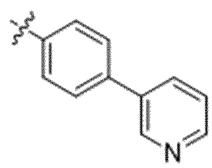 |
| BnH12071 | 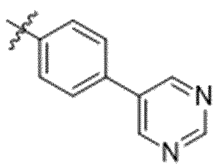 |

**(1) Synthesis of compound BnH12066**

(3S,6R)-6-methyl-1-(2-(4-(pyridin-3-yl)phenyl)acetyl)piperidine-3-carboxylic acid

Step 1: Synthesis of methyl (3S,6R)-6-methyl-1-(2-(4-(pyridin-3-yl)phenyl)acetyl)piperidine-3-carboxylate

To a solution of 2-(4-(pyridin-3-yl)phenyl)acetic acid (6-1-BnH12064, 90 mg, 0.422 mmol) in DMF (1.41 mL) were added methyl (3 S,6R)-6-methylpiperidine-3-carboxylate (absolute form) (69.7 mg, 0.443 mmol), DIPEA (0.110 mL, 0.633

mmol), HOBt (68.4 mg, 0.506 mmol) and EDC·HCl (78.6 mg, 0.506 mmol). The mixture was stirred at room temperature for 3 hours. The mixture was diluted with EtOAc (2.00 mL), washed with sat. aq. $NH_4Cl$ (2.00 mL) and brine (2.00 mL). The organic layer was dried over $MgSO_4$, filtered, and concentrated under reduced pressure. The residue was purified by column chromatography on $SiO_2$ (5% MeOH in DCM) to afford desired compound (37.0 mg, 25 %) as a yellowish oil.

Step 2: Synthesis of compound BnH12066

To a solution of methyl (3S,6R)-6-methyl-1-(2-(4-(pyridin-3-yl)phenyl)acetyl)piperidine-3-carboxylate (37.0 mg, 0.108 mmol) in THF (0.373 mL) was added 1N NaOH (0.162 mL, 0.162 mmol). The mixture was stirred at room temperature for 2 hours. The mixture was diluted with $H_2O$ (2.00 mL) and washed two times with DCM (2.00 mL × 2). The aqueous layer was acidified with 1N HCl until pH 1~2 at 0 °C and extracted with EtOAc (3.00 mL). The organic layer was washed with brine (3.00 mL), dried over $MgSO_4$, filtered, and concentrated under reduced pressure to afford compound BnH12066 (30.0 mg, 82 %) as a white solid.

$^1H$ NMR (400 MHz, DMSO-$d_6$) δ 9.08 (d, *J* = 2.2 Hz, 1H), 8.72 (d, *J* = 5.2 Hz, 1H), 8.50 (d, *J* = 7.9 Hz, 1H), 7.80 (dd, *J* = 19.9, 8.1 Hz, 3H), 7.40 (dd, *J* = 18.3, 7.9 Hz, 2H), 4.75 (s, 0.5H), 4.58 - 4.51 (m, 0.5H), 4.30 (s, 0.5H), 3.93 (d, *J* = 13.6 Hz, 0.5H), 3.89 - 3.74 (m, 2H), 3.05 (t, *J* = 12.8 Hz, 0.5H), 2.61 (t, *J* = 12.8 Hz, 0.5H), 2.22 (d, *J* = 13.8 Hz, 1H), 1.81 (d, *J* = 12.7 Hz, 1H), 1.67 (d, *J* = 12.6 Hz, 1H), 1.58 - 1.46 (m, 2H), 1.09 (dd, *J* = 16.1, 6.9 Hz, 3H).

**(2) Synthesis of compound BnH12071**

(3S,6R)-6-methyl-1-(2-(4-(pyrimidin-5-yl)phenyl)acetyl)piperidine-3-carboxylic acid

Step 1: Synthesis of methyl (3S,6R)-6-methyl-1-(2-(pyridin-3-yl)acetyl)piperidine-3-carboxylate

To a solution of 2-(4-(pyrimidin-5-yl)phenyl)acetic acid (100 mg, 0.467 mmol) in DMF (2.00 mL) were added methyl (3S,6R)-6-methylpiperidine-3-carboxylate (absolute form) (77.1 mg, 0.490 mmol), DIPEA (0.122 mL, 0.700 mmol), HOBt (75.7 mg, 0.560 mmol) and EDC·HCl (107 mg, 0.560 mmol). The mixture was stirred at room temperature for 2 hours. The mixture was diluted with EtOAc (3.00 mL), washed with sat. aq. $NH_4Cl$ (3.00 mL) and brine (3.00 mL). The organic layer was dried over $MgSO_4$, filtered, and concentrated under reduced pressure. The residue was purified by column chromatography on $SiO_2$ (EtOAc only) to afford desired compound (93.5 mg, 57 %) as a pale yellow oil.

Step 2: Synthesis of compound BnH12071

To a solution of methyl (3S,6R)-6-methyl-1-(2-(4-(pyrimidin-5-yl)phenyl)acetyl)piperidine-3-carboxylate (92.0 mg, 0.260 mmol) in THF (1.00 mg, 0.260 mmol) was added 1N NaOH (0.390 mL, 0.390 mmol). The mixture was stirred at room temperature for 2 hours. The mixture was diluted with $H_2O$ (2.00 mL) and washed two times with DCM (2.00 mL × 2). The aqueous layer was acidified with 1N HCl until pH 1~2 at 0 °C and extracted with EtOAc (3.00 mL). The organic layer was washed with brine (3.00 mL), dried over $MgSO_4$, filtered, and concentrated under reduced pressure. The residue was purified by column chromatography on $SiO_2$ (10% MeOH in DCM) to afford compound BnH12071 (35.0 mg, 44 %) as a white solid.

$^1H$ NMR (400 MHz, DMSO-d6) δ 9.16 (d, J = 12.7 Hz, 2H), 7.77 (d, J = 7.9 Hz, 2H), 7.38 (dd, J = 19.1, 7.8 Hz, 2H), 4.75 (s, 0.5H), 4.58 - 4.51 (d, J = 13.8 Hz, 0.5H), 4.29 (s, 0.5H), 3.92 (d, J = 13.8 Hz, 0.5H), 3.80 (tt, J = 15.5, 7.8 Hz, 2H), 3.05 (t, J = 12.8 Hz, 1H), 2.60 (t, J = 12.9 Hz, 1H), 2.24 - 2.11 (m, 1H), 1.86 - 1.77 (m, 1H), 1.66 (d, J = 13.3 Hz, 1H), 1.53 (q, J = 5.7, 4.6 Hz, 2H), 1.08 (dd, J = 16.6, 6.9 Hz, 3H).

**Preparation Example 7. Preparation of active compounds according to representative reaction scheme 8**

[Reaction scheme 8]

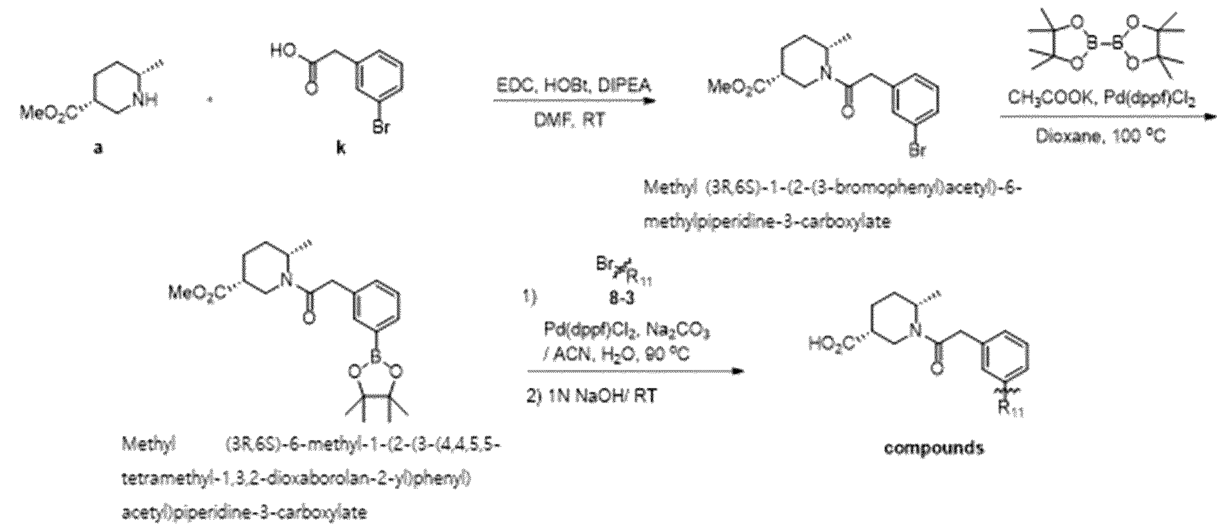


| Compound | $R_{11}$ |
|---|---|
| BnH12091 | 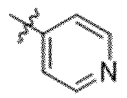 |
| BnH12092 | 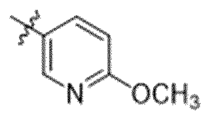 |
| BnH12093 | 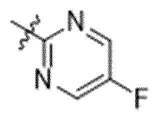 |

**(1) Synthesis of compound BnH12091**

(3R,6S)-6-methyl-1-(2-(3-(pyridin-4-yl)phenyl)acetyl)piperidine-3-carboxylic acid

Step 1: Synthesis of methyl (3R,6S)-1-(2-(3-bromophenyl)acetyl)-6-methylpiperidine-3-carboxylate

To a solution of 3-bromophenylacetic acid (800 mg, 3.72 mmol) in DMF (12.4 mL) were added methyl (3R,6S)-rel-6-methyl-3-piperidinecarboxylate (614 mg, 3.91 mmol), DIPEA (0.972 mL, 5.58 mmol), HOBt (603 mg, 4.46 mmol) and EDC·HCl (692 mg, 4.46 mmol). The mixture was stirred at room temperature for 4 hours. The mixture was diluted with EtOAc (15.0 mL), washed with sat. aq. $NH_4Cl$ (15.0 mL) and brine (15.0 ml). The organic layer was dried over $MgSO_4$, filtered, and concentrated under reduced pressure. The residue was purified by column chromatography on $SiO_2$ (*n*-Hexane : EtOAc = 2 : 1 to 1 : 1) to afford desired compound (990 mg, 75 %) as a colorless oil.

Step 2: Synthesis of methyl (3R,6S)-6-methyl-1-(2-(3-(4,4,5,5-tetramethyl-1,3,2-dioxaborolan-2-yl)phenyl)acetyl)piperidine-3-carboxylate **(8-2)**

A suspension of methyl (3R,6S)-1-(2-(3-bromophenyl)acetyl)-6-methylpiperidine-3-carboxylate (990 mg, 2.80 mmol), Bis(pinacolato)diborane (501 mg, 1.97 mmol) and KOAc (823 mg, 3.07 mmol) in 1,4-dioxane (9.70 mL) was stirred vigorously and bubbled with $N_2$ gas for 5 minutes then $Pd(dppf)Cl_2$ (205 mg, 0.279 mmol) was added. The mixture was bubbled with $N_2$ gas for 5 minutes and refluxed at 100 °C for 14 hours. After completion of reaction, the reaction mixture was filtered through a celite pad and washed with DCM (10.0 mL × 3). The combined organic layer was washed with $H_2O$ (30.0 mL), dried over $MgSO_4$, filtered and concentrated under reduced pressure. The residue was purified by column chromatography on $SiO_2$ (n-Hexane : EtOAc = 2 : 1 to 1 : 1) to afford desired compound (840 mg, 75 %) as a yellowish oil.

Step 3: Synthesis of compound BnH12091

A solution of methyl (3R,6S)-6-methyl-1-(2-(3-(4,4,5,5-tetramethyl-1,3,2-dioxaborolan-2-yl)phenyl)acetyl)piperidine-3-carboxylate (8-2, 215 mg, 0.536 mmol), 4-bromopyridine hydrochloride (104 mg, 0.536 mmol) and $Na_2CO_3$ (170 mg, 1.61 mmol) in MeCN/$H_2O$ (2:1, 6.70 mL) was stirred vigorously and bubbled with $N_2$ gas for 5 minutes. To the mixture Pd(dppf)$Cl_2$ (31.0 mg, 0.042 mmol) was added. The mixture was bubbled with $N_2$ gas for 5 minutes and refluxed at 90 °C for 2 hours. The reaction mixture was cooled to room temperature and added 1N NaOH (2.14 mL, 2.14 mmol). The mixture was stirred at room temperature for 2 hours. The mixture was diluted with $H_2O$ (7.00 mL) and washed with EtOAc (7.00 mL × 3). To the aqueous layer 1N HCl was added to adjust the pH value to pH 4-5 and the mixture was extracted with EtOAc (7.00 mL × 3). The combined organic layer was dried over $MgSO_4$, filtered, and concentrated under reduced pressure. To the residue was added $Et_2O$ (5.00 mL) and stirred at room temperature for 30 minutes. The precipitate was filtered and washed with $Et_2O$ (3.0 mL) to afford compound BnH12091 (112 mg, 62%) as an off-white solid.

$^1$H NMR (400 MHz, DMSO-$d_6$) δ 12.43 (s, 1H), 8.64-8.63 (d, *J* = 5.1 Hz, 2H), 7.69-7.63 (d, *J* = 7.3 Hz, 4H), 7.48-7.44 (t, *J* = 7.7 Hz, 1H), 7.35-7.29 (dd, *J* = 15.8, 7.6 Hz, 1H), 4.74 (s, 0.5H), 4.56-4.52 (m, 0.5H), 4.32 (s, 0.5H), 3.98-3.94 (d, *J* = 14.0 Hz, 0.5H), 3.89-3.77 (m, 2H), 3.07-3.01 (t, *J* = 12.9 Hz, 0.5H), 2.62-2.58 (d, *J* = 12.9 Hz, 0.5H), 2.18-2.14 (d, *J* = 14.5 Hz, 1H), 1.77 (s, 1H), 1.70-1.64 (m, 1H), 1.52 (s, 2H), 1.09-1.04 (m, 3H).

**(2) Synthesis of compound BnH12092**

(3R,6S)-1-(2-(3-(6-methoxypyridin-3-yl)phenyl)acetyl)-6-methylpiperidine-3-carboxylic acid

Step 1: Synthesis of compound BnH12092

A solution of methyl (3R,6S)-6-methyl-1-(2-(3-(4,4,5,5-tetramethyl-1,3,2-dioxaborolan-2-yl)phenyl)acetyl)piperidine-3-carboxylate (8-2, 225 mg, 0.561 mmol), 5-bromo-2-methoxypyridine (105 mg, 0.561 mmol) and $Na_2CO_3$ (119 mg, 1.12 mmol) in MeCN/$H_2O$ (2:1, 7.50 mL) was stirred vigorously and bubbled with $N_2$ gas for 5 minutes. To the mixture Pd(dppf)$Cl_2$ (41.0 mg, 0.056 mmol) was added. The mixture was bubbled with $N_2$ gas for 5 minutes and refluxed at 90 °C for 2 hours. The reaction mixture was cooled to room temperature and added 1N NaOH (1.12 mL, 1.12 mmol). The mixture was stirred at room temperature for 14 hours. The mixture was diluted with $H_2O$ (7.00 mL) and washed with EtOAc (7.00 mL × 3). To the aqueous layer 1N HCl was added to adjust the pH value to pH 4-5 and the mixture was extracted with EtOAc (7.00 mL × 3). The combined organic layer was dried over $MgSO_4$, filtered, and concentrated under reduced pressure. The residue was purified by column chromatography on $SiO_2$ (DCM : MeOH = 20 : 1 to 10 : 1). To the residue was added $Et_2O$ (4.00 mL) and stirred at room temperature for 30 minutes. The precipitate was filtered and washed with $Et_2O$ (2.0 mL) to afford compound BnH12092 (106 mg, 52 %) as an off-white solid.

$^1$H NMR (400 MHz, DMSO-$d_6$) δ 12.43 (s, 1H), 8.45 (dd, *J* = 7.3, 2.7 Hz, 1H), 7.97 (dd, *J* = 8.4, 2.5 Hz, 1H), 7.56 - 7.46 (m, 2H), 7.41 (t, *J* = 7.6 Hz, 1H), 7.22 (dd, *J* = 16.2, 7.6 Hz, 1H), 6.92 (d, *J* = 8.6 Hz, 1H), 4.75 (s, 0.5H), 4.59 - 4.50 (m, 0.5H), 4.32 (s, 0.5H), 3.90 (s, 3.5H), 3.87 - 3.72 (m, 2H), 3.03 (t, *J* = 12.9 Hz, 0.5H), 2.62 (t, *J* = 12.7 Hz, 0.5H), 2.17 (q, *J* = 10.2, 8.7 Hz, 1H), 1.79 (d, *J* = 12.0 Hz, 1H), 1.67 (dd, *J* = 12.5, 6.2 Hz, 1H), 1.51 (d, *J* = 12.4 Hz, 2H), 1.08 (dt, *J* = 12.6, 4.7 Hz, 3H).

**(3) Synthesis of compound BnH12093**

(3R,6S)-1-(2-(3-(5-fluoropyrimidin-2-yl)phenyl)acetyl)-6-methylpiperidine-3-carboxylic acid

Step 1: Synthesis of compound BnH12093

A solution of methyl (3R,6S)-6-methyl-1-(2-(3-(4,4,5,5-tetramethyl-1,3,2-dioxaborolan-2-yl)phenyl)acetyl)piperidine-3-carboxylate (8-2, 213 mg, 0.531 mmol), 2-bromo-5-fluoropyrimidine (93.9 mg, 0.531 mmol) and $Na_2CO_3$ (113 mg, 1.06 mmol) in MeCN/$H_2O$ (2:1, 6.84 mL) was stirred vigorously and bubbled with $N_2$ gas for 5 minutes. To the mixture Pd(dppf)$Cl_2$ (38.8 mg, 0.053 mmol) was added. The mixture was bubbled with $N_2$ gas for 5 minutes and refluxed at 90 °C for 2 hours. The reaction mixture was cooled to room temperature and added 1N NaOH (1.12 mL, 1.12 mmol). The mixture was stirred at room temperature for 17 hours. The mixture was diluted with $H_2O$ (7.00 mL) and washed with EtOAc (7.00 mL × 3). To the aqueous layer 1N HCl was added to adjust the pH value to pH 3-4 and the mixture was extracted with EtOAc (7.00 mL × 3). The combined organic layer was dried over $MgSO_4$, filtered, and concentrated under reduced pressure. The residue was purified by column chromatography on $SiO_2$ (3 to 5% MeOH in DCM). To the residue was added $Et_2O$ (5.00 mL) and stirred at room temperature for 30 minutes. The precipitate was filtered and washed with $Et_2O$ (3.0 mL) to afford compound BnH12093 (122 mg, 64 %) as a beige solid.

$^1$H NMR (400 MHz, DMSO-$d_6$) δ 12.39 (s, 1H), 8.98 (s, 2H), 8.22 (t, *J* = 10.5 Hz, 2H), 7.53 - 7.34 (m, 2H), 4.76 (s,

0.5H), 4.55 (dd, $J$ = 13.2, 4.0 Hz, 0.5H), 4.37 - 4.22 (m, 0.5H), 3.98 - 3.76 (m, 2.5H), 3.03 (t, $J$ = 12.8 Hz, 0.5H), 2.62 (t, $J$ = 12.7 Hz, 0.5H), 2.18 (q, $J$ = 10.8, 9.2 Hz, 1H), 1.79 (d, $J$ = 13.0 Hz, 1H), 1.65 (td, $J$ = 12.5, 7.6 Hz, 1H), 1.51 (d, $J$ = 9.5 Hz, 2H), 1.08 (dt, $J$ = 7.1, 4.4 Hz, 3H).

**Preparation Example 8. Preparation of active compounds according to methods other than representative reaction schemes**

**(1) Synthesis of BnH12002**

(3R,6S)-1-(2-(4'-fluoro-[1,1'-biphenyl]-4-yl)acetyl)-6-methylpiperidine-3-carboxylic acid

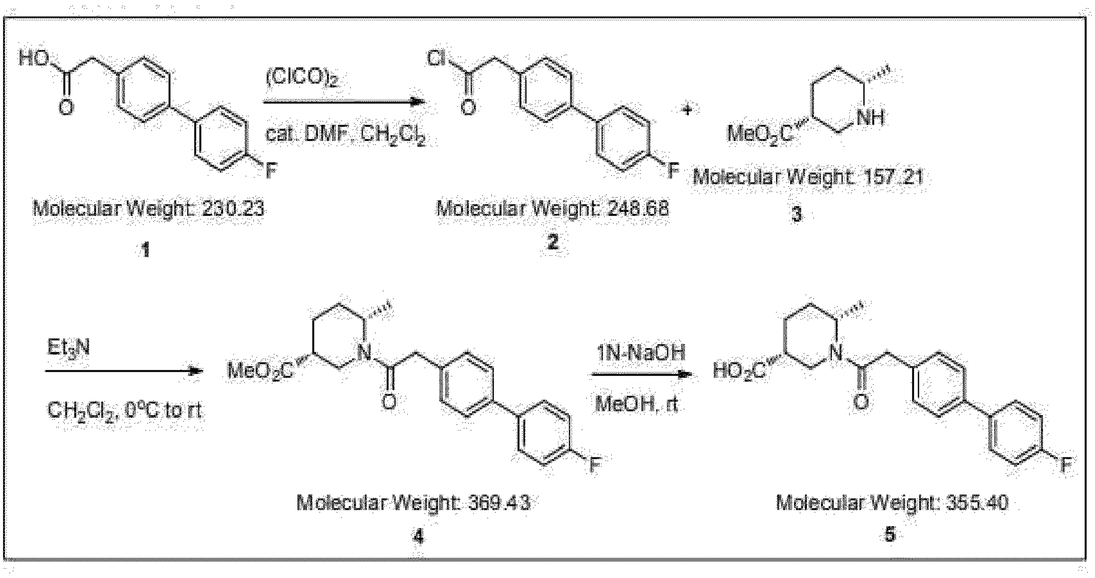


**Step 1:** Compound 1 (293 mg, 1.27 mmol) was dissolved in 5 mL of DCM, and oxalyl chloride (163 μL, 1.91 mmol) was added dropwise at 0 °C for 5 minutes, and then DMF (1 drop) was added together with a catalyst. After stirring at room temperature for 6 hours, the mixture was concentrated under reduced pressure, which resulted in 316 mg of compound 2. The reaction proceeded to the next step without further purification.

**Step 2:** Compound 3 (200 mg, 1.27 mmol) was dissolved in 10 mL of DCM, and triethyl amine (354 μL, 2.54 mmol) was added at 0 °C. Compound 2 (316 mg, 1.27 mmol) was dissolved in 5 mL of DCM and added dropwise at 0 °C, followed by stirring at room temperature for 2 hours. After work-up of the reaction mixture with DCM and water, 213 mg of compound 4 was obtained using a column (yield 45%).

**Step 3:** Compound 4 (100 mg, 473 μmol) was dissolved in 5 mL of THF/methanol (1:1), 1N-NaOH (947 μL, 947 μmol) was added, and it was stirred at room temperature for 12 hours. After completion of the reaction, the pH was adjusted to 3 using 1N-HCl, and after extracting with ethyl acetate, it was concentrated under reduced pressure. 75 mg of target compound 5 was obtained from the obtained reaction mixture using prep TLC (yield 45%).

Identity was confirmed by NMR spectra: 1H NMR (CDCl3, 400 MHz): δ 7.22 (1H, m), 7.08 (1H, m), 6.91 (1H, bs), 4.75(1H, m), 3.86 (1H, m), 3.71(2H, m), 3.01 (1H, m), 2.28 (1H, m), 1.91 (1H, bs), 1.74(1H, m), 1.58 (1H, bs), 1.13(3H, m).

**(2) Synthesis of BnH12003**

(3R,6S)-6-methyl-1-(2-(4-(pyrimidin-5-yl)phenyl)acetyl)piperidine-3-carboxylic acid

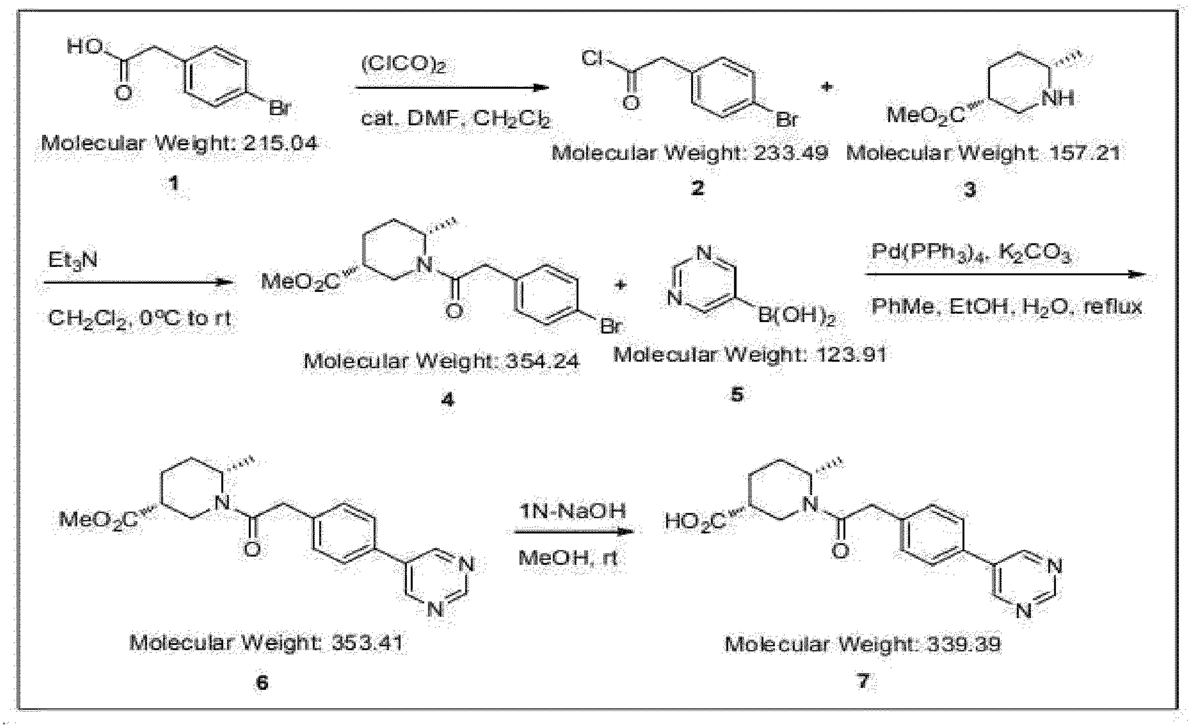


**Step 1:** Compound 1 (547 mg, 2.54 mmol) was dissolved in 10 mL of DCM, and oxalyl chloride (327 μL, 3.81 mmol) was added dropwise at 0 °C for 5 minutes, and then DMF (1 drop) was added together with a catalyst. After stirring at room temperature for 6 hours, the mixture was concentrated under reduced pressure to obtain 593 mg of compound 2, and the reaction proceeded to the next step without further purification.

**Step 2:** Compound 3 (400 mg, 2.54 mmol) was dissolved in 10 mL of DCM, and triethyl amine (708 μL, 5.08 mmol) was added at 0 °C. Compound 2 (593 mg, 2.54 mmol) was dissolved in 5 mL of DCM and added dropwise at 0 °C, followed by stirring at room temperature for 2 hours. After work-up of the reaction mixture with DCM and water, 347 mg of compound 4 was obtained using a column (yield 38%).

**Step 3:** Compound 4 (300 mg, 846 μmol), compound 5 (104 mg, 846 μmol), Pd(PPh3)4 (49 mg, 43 μmol) and $K_2CO_3$ (234 mg, 1.69 mmol) were dissolved in toluene 3 mL/ethanol 1 mL/$H_2O$ 1 mL, degassed under nitrogen and refluxed for 12 hours. After completion of the reaction, it was extracted with EA and concentrated under reduced pressure to obtain 38 mg of compound 6 using prep TLC (yield 13%).

**Step 4:** Compound 6 (38 mg, 107 μmol) was dissolved in 3 mL of THF/methanol (1:1), 1N-NaOH (430 μL, 430 μmol) was added, and it was stirred at room temperature for 12 hours. After completion of the reaction, the pH was adjusted to 3 using 1N-Hcl and after extracting with ethyl acetate, it was concentrated under reduced pressure. 12 mg of target compound 7 was obtained from the obtained reaction mixture using prep TLC (yield 45%).

Identity was confirmed by NMR spectra: 1H NMR (CDCl3, 400 MHz): δ 9.20 (1H, s), 8.97 (2H, m), 7.55 (2H, d, J=8Hz), 7.42 (2H, d, J=8Hz), 4.88(1H, m), 4.15 (1H, m), 3.82(2H, s), 3.01 (1H, m), 2.28 (1H, m), 1.98 (1H, m), 1.80(1H, m), 1.60 (2H, m), 1.17(3H, m).

**(3) Synthesis of compound BnH12057**

(3R,6S)-6-methyl-1-(2-(4-(oxazol-5-yl)phenyl)acetyl)piperidine-3-carboxylic acid

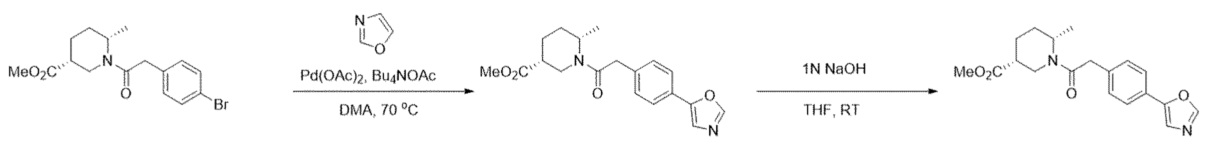


1-2-BnH12032 g BnH12057

Step 1: Synthesis of methyl (3R,6S)-6-methyl-1-(2-(4-(oxazol-5-yl)phenyl)acetyl)piperidine-3-carboxylate (g)

A solution of compound 1-2-BnH12032 (500 mg, 1.41 mmol), oxazole (97.5 mg, 1.41 mmol) and $Bu_4NOAc$ (553 mg, 1.84 mmol) in DMA (4.71 mL) was stirred vigorously and bubbled with $N_2$ gas for 5 minutes and the $Pd(OAc)_2$ (95.1 mg, 0.423 mmol) was added. The mixture was bubbled with $N_2$ gas for 5 minutes and refluxed at 70 °C for 17 hours. Then, the mixture was cooled to room temperature and concentrated. The residue was purified by column chromatography on SiO2 (*n*-Hexane : EtOAc = 1 : 1) to afford desired compound (110 mg, 22 %) as a brown oil.

Step 2: Synthesis of compound BnH12057

To a solution of methyl (3R,6S)-6-methyl-1-(2-(4-(oxazol-5-yl)phenyl)acetyl)piperidine-3-carboxylate (25.0 mg, 0.073 mmol) in THF (0.252 mL) was added 1N NaOH (0.110 mL, 0.110 mmol). The mixture was stirred at room temperature for 2 hours. The mixture was diluted with $H_2O$ (2.00 mL) and washed with DCM (2.00 × 2). The aqueous layer was acidified with 1N HCl until pH 1~2 at 0 °C and extracted with EtOAc (3.00 mL). The organic layer was washed with brine (3.00 mL), dried over $MgSO_4$, filtered and concentrated under reduced pressure to afford compound BnH12057 (10.0 mg, 41 %) as a white solid.

$^1H$ NMR (400 MHz, Chloroform-d) δ 7.93 (s, 1H), 7.62 (d, J = 8.1 Hz, 2H), 7.37 - 7.29 (m, 3H), 4.98 (s, 0.5H), 4.83 (d, J = 13.9 Hz, 0.5H), 4.15 (d, J = 16.6 Hz, 0.5H), 3.92 (d, J = 13.9 Hz, 0.5H), 3.78 (s, 2H), 3.16 (t, J = 12.9 Hz, 0.5H), 2.79 (t, J = 12.9 Hz, 0.5H), 2.40 (s, 0.5H), 2.16 (d, J = 7.3 Hz, 0.5H), 1.94 (d, J = 14.3 Hz, 1H), 1.80 (q, J = 12.2, 11.3 Hz, 1H), 1.67 - 1.44 (m, 2H), 1.14 (d, J = 6.9 Hz, 3H).

**(4) Synthesis of compound BnH12067 (abosolute form)**

(3S,6R)-6-methyl-1-(2-(4-(pyrazin-2-yl)phenyl)acetyl)piperidine-3-carboxylic acid

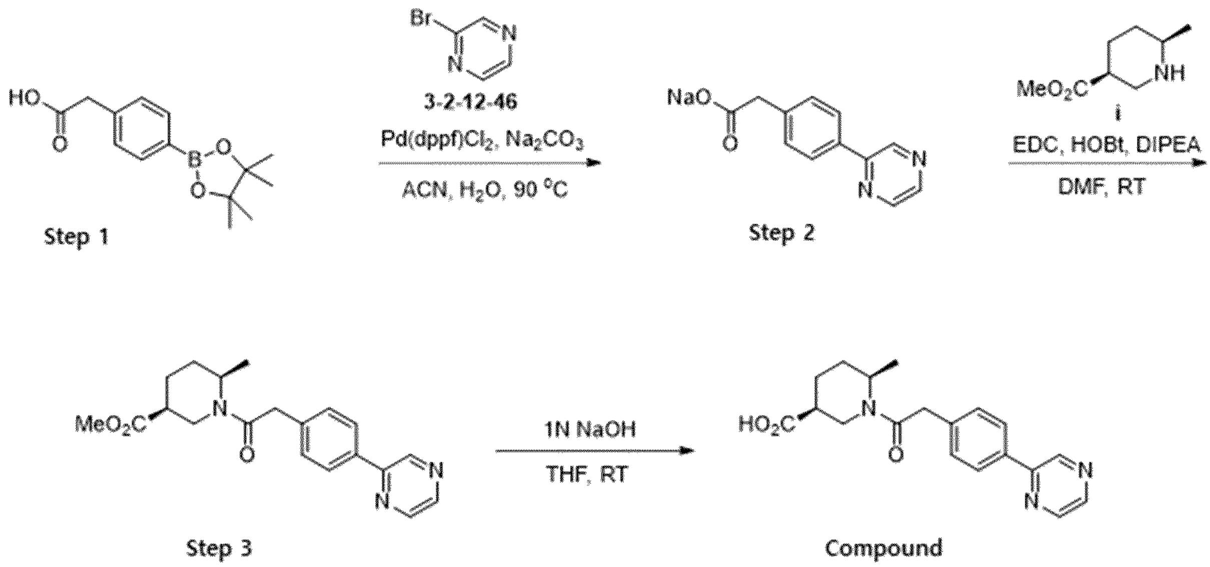

Step 1 Step 2 Step 3 Compound

Step 1: Synthesis of sodium 2-(4-(pyrazin-2-yl)phenyl)acetate **(BnH12067-2)**

A solution of 2-(4-(4,4,5,5-tetramethyl-1,3,2-dioxaborolan-2-yl)phenyl)acetic acid (6-1-BnH12064-1, 100 mg, 0.382 mmol), 2-bromopyrazine (66.3 mg, 0.420 mmol) and $Na_2CO_3$ (121 mg, 1.15 mmol) in MeCN/$H_2O$ (2:1, 4.76 mL) was stirred vigorously and bubbled with $N_2$ gas for 5 minutes and the $Pd(dppf)Cl_2$ (27.9 mg, 0.038 mmol) was added. The mixture was bubbled with $N_2$ gas for 5 minutes and refluxed at 90 °C for 3 hours. Then, the mixture was cooled to room temperature. The mixture was diluted with $H_2O$ (10.0 mL) and washed with DCM (5.00 mL × 3). To the aqueous layer was concentrated under reduced pressure to afford desired compound (90.0 mg, quant.) as a yellow solid. The desired compound was used without further purification.

Step 2: Synthesis of methyl (3S,6R)-6-methyl-1-(2-(4-(pyrazin-2-yl)phenyl)acetyl)piperidine-3-carboxylate **(BnH12067-1)**

To a solution of sodium 2-(4-(pyrazin-2-yl)phenyl)acetate (BnH12067-2, 90 mg, 0.381 mmol) in DMF (1.27 mL) were added methyl (3R,6S)-rel-6-methyl-3-piperidinecarboxylate (abosolute form) (62.9 mg, 0.400 mmol), DIPEA (0.100 mL, 0.572 mmol), HOBt (61.8 mg, 0.457 mmol) and EDC·HCl (71.0 mg, 0.457 mmol). The mixture was stirred at room temperature for 6 hours. The mixture was diluted with EtOAc (2.00 mL), washed with sat. aq. $NH_4Cl$ (2.00 mL) and brine (2.00 mL). The organic layer was dried over $MgSO_4$, filtered, and concentrated under reduced pressure. The residue was purified by column chromatography on $SiO_2$ (5% MeOH in DCM) to afford desired compound (67.0 mg, 50 %) as a brown oil.

Step 3: Synthesis of compound BnH12067

To a solution of methyl (3S,6R)-6-methyl-1-(2-(4-(pyrazin-2-yl)phenyl)acetyl)piperidine-3-carboxylate (67.0 mg, 0.190 mmol) in THF (1.00 mL) was added 1N NaOH (0.284 mL, 0.284 mmol). The mixture was stirred at room temperature for 2 hours. The mixture was diluted with $H_2O$ (2.00 mL) and washed two times with DCM (2.00 mL × 2). The aqueous layer was acidified with 1N HCl until pH 1~2 at 0 °C and extracted with EtOAc (2.00 mL). The organic layer was washed with brine (3.00 mL), dried over $MgSO_4$, filtered, and concentrated under reduced pressure to afford compound BnH12067 (49.9 mg, 88 %) as an off-white solid.

$^1H$ NMR (400 MHz, DMSO-$d_6$) δ 9.25 (s, 1H), 8.71 (t, *J* = 2.0 Hz, 1H), 8.60 (d, *J* = 2.5 Hz, 1H), 8.10 (d, *J* = 7.9 Hz, 2H), 7.39 (dd, *J* = 18.5, 7.9 Hz, 2H), 4.75 (s, 0.5H), 4.55 (d, *J* = 13.7 Hz, 0.5H), 4.28 (s, 0.5H), 3.92 (d, *J* = 13.8 Hz, 0.5H), 3.88 - 3.73 (m, 2H), 3.04 (t, *J* = 12.9 Hz, 0.5H), 2.62 (t, *J* = 12.8 Hz, 0.5H), 2.19 (ddt, *J* = 11.7, 8.0, 4.0 Hz, 1H), 1.79 (d, *J* = 11.9 Hz, 1H), 1.66 (d, *J* = 13.7 Hz, 1H), 1.52 (s, 2H), 1.07 (dd, *J* = 9.2, 7.0 Hz, 3H).

**(5) Synthesis of compound BnH12095**

(3R,6S)-6-methyl-1-(2-(3-(pyrimidin-5-yl)phenyl)acetyl)piperidine-3-carboxylic acid

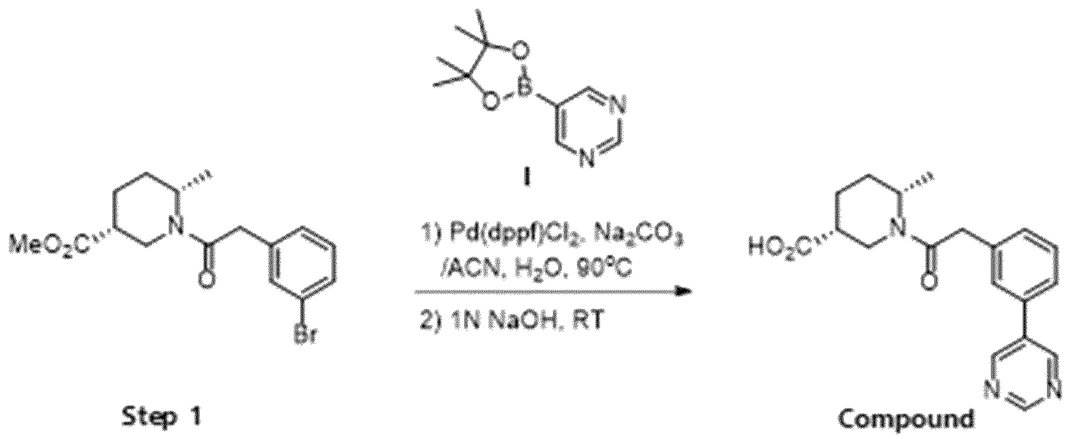


Step 1: Synthesis of compound BnH12095

A solution of methyl (3R,6S)-1-(2-(3-bromophenyl)acetyl)-6-methylpiperidine-3-carboxylate (8-1, 170 mg, 0.424 mmol), 5-(4,4,5,5-tetramethyl-1,3,2-dioxaborolan-2-yl)pyrimidine (87.3 mg, 0.424 mmol) and $Na_2CO_3$ (89.8 mg, 0.847mmol) in MeCN/$H_2O$ (2:1, 5.46 mL) was stirred vigorously and bubbled with $N_2$ gas for 5 minutes. To the mixture Pd(dppf)$Cl_2$ (31.0 mg, 0.042 mmol) was added. The mixture was bubbled with $N_2$ gas for 5 minutes and refluxed at 90 °C for 3 hours. The reaction mixture was cooled to room temperature and added 1N NaOH (0.847 mL, 0.847 mmol). The mixture was stirred at room temperature for 17 hours. The mixture was diluted with $H_2O$ (5.00 mL) and washed with EtOAc (5.00 mL × 3). To the aqueous layer 1N HCl was added to adjust the pH value to pH 2-3 and the mixture was extracted with EtOAc (5.00 mL × 3). The combined organic layer was dried over $MgSO_4$, filtered, and concentrated under reduced pressure. The residue was purified by column chromatography on $SiO_2$ (3 to 5% MeOH in DCM) to afford compound BnH12095 (95.0 mg, 66 %) as an off-white solid.

$^1H$ NMR (400 MHz, DMSO-$d_6$) δ 12.40 (s, 1H), 9.20 (s, 1H), 9.12 (d, *J* = 8.6 Hz, 2H), 7.68 (d, *J* = 9.7 Hz, 2H), 7.49 (t,

$J$ = 7.7 Hz, 1H), 7.33 (dd, $J$ = 15.5, 7.7 Hz, 1H), 4.74 (s, 0.5H), 4.61 - 4.49 d, $J$ = 13.6 Hz, 0.5H), 4.33 (s, 0.5H), 3.98 (d, $J$ = 13.6 Hz, 0.5H), 3.91 - 3.74 (m, 2H), 3.06 (t, $J$ = 12.9 Hz, 0.5H), 2.62 (t, $J$ = 12.7 Hz, 0.5H), 2.15 (dd, $J$ = 25.2, 12.4 Hz, 1H), 1.76 (s, 1H), 1.66 (dq, $J$ = 12.1, 6.6 Hz, 1H), 1.54 (d, $J$ = 9.8 Hz, 2H), 1.14 - 1.03 (m, 3H).

**(6) Synthesis of compound BnH12097**

6-Ethyl-1-(2-(4-(pyrimidin-5-yl)phenyl)acetyl)piperidine-3-carboxylic acid

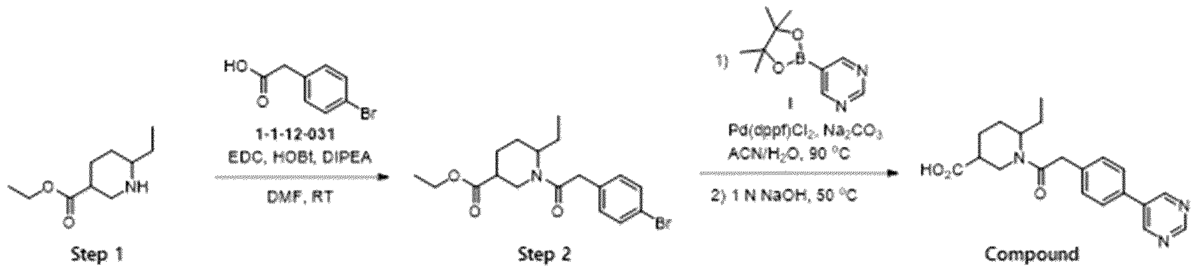


Step 1: Synthesis of ethyl 1-(2-(4-bromophenyl)acetyl)-6-ethylpiperidine-3-carboxylate (BnH12097-1)

To a solution of 2-(4-bromophenyl)acetic acid (700 mg, 3.26 mmol) in DMF (9.00 mL) were added ethyl 6-ethylpiperidine-3-carboxylate (p, 633 mg, 3.42 mmol), DIPEA (1.70 mL, 9.77 mmol), HOBt (528 mg, 3.91 mmol) and EDC·HCl (749 mg, 3.91 mmol). The mixture was stirred at room temperature for 17 hours. The reaction mixture was diluted with EtOAc (25.0 mL), washed with sat. aq. $NH_4Cl$ (25.0 mL) and brine (25.0 mL). The organic layer was dried over $Na_2SO_4$, filtered, and concentrated under reduced pressure. The residue was purified by column chromatography on $SiO_2$ (*n*-Hexane : EtOAc = 1 : 0 to 2 : 1) to afford desired compound (302 mg, 36 %, for 2 steps) as a yellow oil.

Step 2: Synthesis of compound BnH12097

A solution of ethyl 1-(2-(4-bromophenyl)acetyl)-6-ethylpiperidine-3-carboxylate (150 mg, 0.392 mmol), 5-(4,4,5,5-tetramethyl-1,3,2-dioxaborolan-2-yl)pyrimidine (80.9 mg, 0.392 mmol), and $Na_2CO_3$ (83.2 mg, 0.785 mmol) in MeCN:$H_2O$ (2:1, 4.80 mL) was stirred vigorously and bubbled with $N_2$ gas for 5 minutes and Pd(dppf)$Cl_2$ (28.7 mg, 0.039 mmol) was added. The mixture was bubbled with $N_2$ gas for 5 minutes and refluxed at 90 °C for 3 hours. After stirring, the mixture was cooled to room temperature, 1 NNaOH (0.970 mL, 0.970 mmol) was added, and stirred at 50 °C for 17 hours. The reaction mixture was diluted with $H_2O$ (5.00 mL) and washed two times with DCM (5.00 mL × 2). The aqueous layer was acidified with 1 N HCl until pH 1~2 at 0 °C and extracted with EtOAc (5.00 mL). The organic layer was dried over $Na_2SO_4$, filtered, and concentrated under reduced pressure. The residue was purified by column chromatography on $SiO_2$ (DCM : MeOH = 1 : 0 to 4 : 1) to afford compound BnH12097 (35.3 mg, 26 %) as a pale brown solid.

$^1H$ NMR (400 MHz, DMSO-$d_6$) δ 9.18 - 9.14 (m, 3H), 7.77 (d, J = 7.8 Hz, 2H), 7.39 (dd, J = 14.3, 7.9 Hz, 2H), 4.58 - 4.54 (m, 1H), 3.99 - 3.71 (m, 3.5H), 2.96 (t, J = 12.9 Hz, 0.5H), 2.19 - 2.09 (m, 1H), 1.79 (s, 1H), 1.65 - 1.39 (m, 5H), 0.77 (dt, J = 14.0, 7.3 Hz, 3H).

**(7) Synthesis of compound BnH12098**

6-isopropyl-1-(2-(4-(pyrimidin-5-yl)phenyl)acetyl)piperidine-3-carboxylic acid

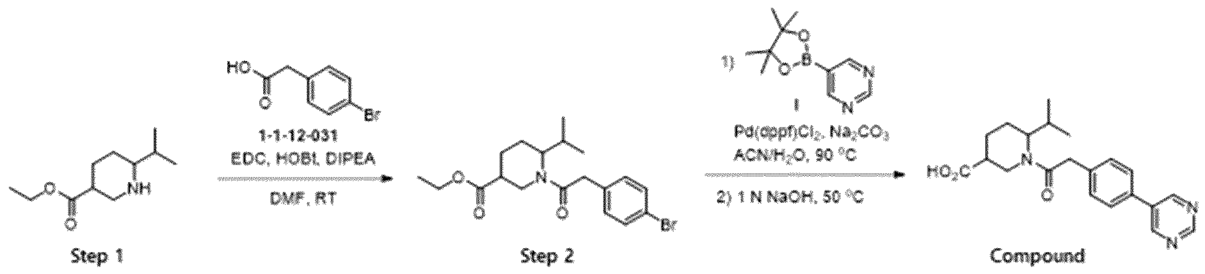


Step 1: Synthesis of ethyl 1-(2-(4-bromophenyl)acetyl)-6-isopropylpiperidine-3-carboxylate (BnH12098-1)

To a solution of 2-(4-bromophenyl)acetic acid (620 mg, 2.91 mmol) in DMF (9.69 mL) were added ethyl 6-isopropylpiperidine-3-carboxylate (s, 608 mg, 3.05 mmol), DIPEA (1.52 mL, 8.72 mmol), HOBt (471 mg, 3.49 mmol) and EDC·HCl (542 mg, 3.49 mmol). The mixture was stirred at room temperature for 17 hours. The reaction mixture was diluted with EtOAc (20.0 mL), washed with sat. aq. $NH_4Cl$ (20.0 mL) and brine (20.0 mL). The organic layer was dried over $MgSO_4$, filtered, and concentrated under reduced pressure. The residue was purified by column chromatography on $SiO_2$ (*n*-Hexane : EtOAc = 3 : 1 to 2 : 1) to afford desired compound (618 mg, 54 %, for 2 steps) as a yellow oil.

Step 2: Synthesis of compound BnH12098

A solution of ethyl 1-(2-(4-bromophenyl)acetyl)-6-isopropylpiperidine-3-carboxylate (300 mg, 0.748 mmol), 5-(4,4,5,5-tetramethyl-1,3,2-dioxaborolan-2-yl)pyrimidine (154 mg, 0.748 mmol), and $Na_2CO_3$ (158 mg, 1.50 mmol) in MeCN:$H_2O$ (2:1, 9.64 mL) was stirred vigorously and bubbled with $N_2$ gas for 5 minutes and Pd(dppf)$Cl_2$ (54.7 mg, 0.075 mmol) was added. The mixture was bubbled with $N_2$ gas for 5 minutes and refluxed at 90 °C for 2 hours. After stirring, the mixture was cooled to room temperature, 1 N NaOH (1.50 mL, 1.50 mmol) was added, and stirred at 50 °C for 17 hours. The reaction mixture was diluted with $H_2O$ (5.00 mL) and washed two times with DCM (5.00 mL × 2). The aqueous layer was acidified with 1 N HCl until pH 1~2 at 0 °C and extracted with EtOAc (10.0 mL). The organic layer was dried over $MgSO_4$, filtered, and concentrated under reduced pressure. The residue was purified by column chromatography on $SiO_2$ (5 to 10% MeOH in DCM) to afford compound BnH12098 (90 mg, 33 %) as an off-white solid.

$^1$H NMR (400 MHz, DMSO-$d_6$) δ 12.39 (s, 1H), 9.20 - 9.12 (m, 3H), 7.78 (d, *J* = 7.9 Hz, 2H), 7.40 (dd, *J* = 10.7, 7.9 Hz, 2H), 4.62 - 4.54 (m, 0.5H), 4.25 - 4.13 (m, 0.5H), 3.97 (dd, *J* = 14.9, 4.1 Hz, 0.5H), 3.93 - 3.68 (m, 2H), 3.60 (d, *J* = 10.2 Hz, 0.5H), 2.96 (t, *J* = 13.0 Hz, 1H), 2.29 - 1.96 (m, 2H), 1.82 (d, *J* = 14.5 Hz, 1.5H), 1.70 - 1.55 (m, 1H), 1.32 (dt, *J* = 28.1, 13.4 Hz, 1H), 0.92 (dd, *J* = 17.3, 6.5 Hz, 3H), 0.75 (dd, *J* = 25.8, 6.6 Hz, 3H).

The following biological assays were performed using the compounds obtained in the above preparation examples.

## Examples

### Example 1. Cell culture and maintenance

Human undifferentiated neuroblastoma cell line SH-SY5Y and human embryonic kidney cell line (HEK)-293T were purchased from Korean Cell Line Bank (Korea) and Takara (Japan), respectively. Cells were grown in HyClone Minimal Essential Medium (MEM) and Dulbecco's Modified Eagle Medium (DMEM), respectively, supplemented with 10% (v/v) fetal bovine serum (FBS), penicillin (100 U/mL), and streptomycin (100 mg/mL). Cells were maintained in a humidified atmosphere of 95% air and 5% $CO_2$ at 37°C. Cells were passaged 10 times at maximum. Cells were dissociated using 0.05% trypsin-EDTA solution and the cells were seeded in new culture dishes for maintenance.

Meanwhile, primary cortical neuron cells were isolated from cerebral cortices of 18-day-old mouse fetuses, incubated in prep media (composition (mM): NaCl 107.5, $NaH_2PO_4$ 1, $NaHCO_3$ 33.5, glucose 11, HEPS 1%, penicillin-streptomycin 1%) containing 0.25% trypsin and DNaseI for 30 minutes, dissociated into single cells, and seeded. Primary cortical neuron cells were grown in Neurobasal medium supplemented with 1% (v/v) B27, 1% penicillin (100 U/mL), streptomycin (100 mg/mL), and 1% Glutamax. Cells were maintained in a humidified atmosphere of 95% air and 5% $CO_2$ at 37°C. Drug efficacy was evaluated after culturing primary cortical neuron cells for 14 days.

### Example 2. Cytotoxicity test

To examine the cytotoxicity of compounds described herein in human cell lines, SH-SY5Y and 293T cells were used. Approximately 8,000 cells (for 293T) and 15,000 cells (for SH-SY5Y) were plated per well of a 96-well plate (SPL Life

Sciences Co., Ltd., Pocheon, Korea). After incubating at 37°C for 24 hours in a humidified atmosphere containing 5% $CO_2$, the culture medium was replaced by a series of concentrations of test compounds diluted in the corresponding culture medium. The time of co-incubation was 24 hours and six replicates were made for each measurement. Finally, 10 μL of CCK-8 reagent (Dojindo Laboratories, Rockville, MD) was added to each well, and after incubation at 37°C for 3 minutes, the OD at 450 nm was measured using a multifunction microplate reader (Tecan Infinite M200 Pro). These measurements were compared to that of a control and expressed as a percentage of the control to calculate cell viability. The $IC_{50}$ value was calculated using SPSS software. To do this, the absorbance of the blank wells with only growth medium is subtracted from the values or the wells with cells. The readout value of background (vehicle) was set as a 100% viability, and then the percent viability for each test solution was calculated.

$$\text{Cell viability (\%)} = [(\text{As-Ab}) / (\text{Ac-Ab})] \times 100$$

The data was presented as the mean value and standard error of the mean (S.E.M.). Statistical analysis was performed with the one-way ANOVA with Tukey's post hoc test (GraphPad Prism 5 software package, version 5.02, GraphPad Software Inc. USA). Significance was accepted at $p < 0.05$.

**Example 3. Measurement of expression levels of BDNF and/or GluN2B**

**Example 3.1. RT-PCR**

RT-PCR was used to measure the amount of BDNF and GluN2B in response to compounds of the present disclosure. 293T cells were dissociated using 0.05% Trypsin-EDTA solution and seeded in 24 well or 6 well dishes for RNA work. After incubation at 37°C in a humidified atmosphere with 5% $CO_2$ for 24 hours, the culture medium was replaced by a medium containing a series of concentrations of tested compounds diluted with the DMEM without FBS. Time course experiments were performed. The same volume of DMSO was used for negative control.

RNA was isolated from the cells after the compound treatment. After removing growth media, 0.3 to 0.4 mL of TRIzol™ per $1 \times 10^5$ to $10^7$ cells were added directly to the culture dish to lyse the cells. After incubating for 10 minutes at 4°C, the sample was centrifuged for 10 minutes at 12,000 × g at 4°C. Total RNA precipitate formed a white gel-like pellet at the bottom of the tube. After discarding the supernatant, the pellet was resuspended in 1 mL of 75% ethanol per 1 mL of TRIzol™ used for lysis. After vortexing the sample briefly, the sample was centrifuged for 5 minutes at 7500 × g at 4°C. The supernatant was discarded with a micropipettor and vacuum or air dried for 5 to 10 minutes. The pellet was resuspended in 20-50 μL of RNase-free water. RNA concentration was determined with the Thermo Scientific™ μDrop™ plate. RNA quality was determined by measuring A230/260 and A260/280 ratio.

On ice, the experimental RNA (up to 1 μg in 4 ul) and the oligo dT primer 1 ul (100 pmol) in Nuclease-Free Water were combined for a final volume of 5 μl per RT reaction. After closing each tube of RNA tightly, the tubes were placed into a 70°C for 5 minutes (in PCR machine). After taking the tubes out, the tubes were immediately chilled in ice-water for at least 5 minutes. Each tube was centrifuged for 10 seconds in a microcentrifuge to collect the condensate and maintain the original volume. The tubes were kept closed and on ice until the reverse transcription reaction mix was added. The reverse transcription reaction mix was prepared as follows.

| | |
|---|---|
| DW | 4.9 ul |
| 5x RT buffer | 4 ul |
| 25 mM $MgCl_2$ | 1.6 ul (final 2.6 mM) |
| 10 mM dNTP (2.5 mM each) | 3 ul (final 0.5 mM each dNTP) |
| RNasin (40 u/ul) | 0.5 ul (final 20 u) |
| RT | 1 ul |
| Total | 15 ul/tube |

Reverse transcription was performed using Improm-II Reverse Transcriptase (A3803) (Promega) and Recombinant RNasin Ribonuclease Inhibitor (N2515) (Promega) according to the manufacturer's instruction. 15 μl aliquots of the reverse transcription reaction mix were added to each reaction tube on ice and placed the tubes back in a PCR machine. PCR condition was as follows:

25°C, 5 min (annealing) → 42°C, 60 min (extension) → 70°C, 15 min (inactivation of RT) → holding at 4°C

The PCR product was analyzed by agarose gel electrophoresis. Housekeeping genes (ACTIN) were used as a PCR control. Replicate samples confirmed the validity of observed changes in RNA levels. Primer sequences used are as follows:

| | |
|---|---|
| h-GluN2B-Forward | CTTCCATTTACCCTGCCTCA |
| h-GluN2B-Reverse | CATTGCCTCTAGCTCCCTTG |
| h-BDNF-Forward | CATCCGAGGACAAGGTGGCTTG |
| h-BDNF-Reverse | GCCGAACTTTCTGGTCCTCATC |
| h-ActinB-Forward | CACCAACTGGGACGACAT |
| h-ActinB-Reverse | ACAGCCTGGATAGCAACG |

**Example 3.2. Western blotting**

The amount of GluN2B reacted with the compound of the present disclosure was measured using Western blot. The fetal cerebral cortex was extracted from mice at 17 days of gestation, dissociated into single cells, cultured for 13 days, and treated with BnH drugs for 24 hours. The drug-treated cells were subjected to protein lysate preparation using RIPA protein lysis buffer containing protease and phosphatase inhibitors. The protein concentration was determined using a BCA protein assay kit (Pierce Biotechnology) using bovine serum albumin as a standard. Equal amounts of protein (20 μg per lane) were separated by electrophoresis on SDS-polyacrylamide gels at 90 V for 2 hours, then transferred from the SDS-polyacrylamide gel to PVDF membrane at 100 V for 1 hour. Then, primary reaction with GluN2B antibody (ab65783, Abcam) was performed at 4°C for 1 day, washed three times at 10-minutes intervals with 1X PBT, and secondary reaction was performed with anti-mouse antibody (sc-2357, Santa Cruz) for 1 hour, followed by washing three times at 10-minutes intervals with 1X PBT. Western blotting was performed as described above to confirm GluN2B protein expression. Subsequently, each blot was re-probed using anti-β-actin antibody (sc-47778, Santa Cruz). Housekeeping protein β-actin was detected as a normalization control.

As a result, the protein changes of GluN2B were confirmed in various BnH12 series compounds as shown in Table 6.

Data analysis

The increase and decrease of BnH12 series were confirmed by setting the vehicle to 1 (N for each group was 3 or more).

**Example 4. Synaptic function test**

**Example 4.1. Os pump transplantation**

Eight weeks old male C57BL/6J mice were anesthetized with isoflurane during surgery. The mice were then be placed in a stereotaxic frame (RWD Life Science, China). The parietal and the frontal bones were exposed by a sagittal incision of the scalp at the midline from the level of the eyes to the occipital protuberance. A small burr hole was drilled after exposing the skull. The injection cannula was placed at the following coordinates: (Mouse: Bregma - 3.30, lateral -1.50, and ventral 0.7 mm). Sterile saline solution (+/- a compound of the present disclosure) was perfused at a constant flow rate of 0.5 μL/h through the injection cannula using a mini-Os pump (DURECT Corporation ALZET Osmotic Pumps, CA). Implantation was done in layer 4 of the barrel cortex ipsilateral. To estimate extent of the diffusion of the injected compound in layer 4 barrel cortex during pump injection, methylene blue was first injected to stain the diffusion area. On average methylene blue diffused to 2000.0 μm away from the injection site (the total area of the barrel cortex region is about 1500 μm). Based on this analysis, the present inventors recorded from barrel cortex area.

**Example 4.2. Oral administration of compounds**

BnH12003 was dissolved using DMSO:PEG400:saline (5:55:40) and administered at a dose of 25 mg/kg (200 μl/animal). For the control group, DMSO:PEG400:saline (5:55:40) (200 μl/animal) was administered. Starting from 8 weeks of age, the compound was administered once a day for a total of 22 times from day 10 to day 31.

**Example 4.3. Intraperitoneal injection of compounds**

The compound was diluted to 25 mg/kg using DMSO:PEG400:Saline(5:55:40). The compound was administered intraperitoneal at 10:00 a.m. daily (*i.p.* volume was 200 μl) for about 11 to 12 days. Control group was also administered by *i.p.* injection in the same manner using the vehicle (DMSO:PEG400:Saline(5:55:40)). 5 mice were used for the compound administration group and the control group, respectively.

**Example 4.4. Preparation of thalamocortical (TC) or hippocampus slice**

For TC or hippocampus slice preparation, 9 to 10 weeks-old male C57BL/6J mice were anesthetized with isoflurane. Then, the brain was rapidly cooled via transcardial perfusion with ice-cold sucrose-artificial cerebrospinal fluid (aCSF). The brain was removed and placed in ice-cold sucrose aCSF. Paracoronal slices were prepared at an angle of 50° relative to the midline on a ramp at an angle of 10°. Then, slices were incubated in aCSF at 35°C for 30 minutes to recover. Slices were then incubated in aCSF at room temperature (23 to 25°C) for 1 to 4 hours before being placed in the recording chamber for experiments. The standard aCSF contains saturated with 95% $O_2$/5% $CO_2$. The brain of the mouse was removed and cut into 400 μm at a 50° angle (mouse at a 55° angle) to attach the cut portion to the vibratome disc to be the bottom. Brain slice was incubated in 35°C in aCSF solution for 30 minutes, or at RT in aCSF solution for 1 hour.

Composition of sucrose based aCSF

| Cutting aCSF (4°C) | | | | | | |
|---|---|---|---|---|---|---|
| Chemical | Sigma # | F.W | Conc (mM) | g/1L | g/2L | g/4L |
| Sucrose | S9378 | 342.3 | 195.5 | 66.931 | 133.862 | 267.725 |
| KCl | P9333 | 74.56 | 2.5 | 0.186 | 0.373 | 0.746 |
| $NaH_2PO_4$ | S8282 | 119.98 | 1 | 0.BnH120 | 0.240 | 0.480 |
| $NaHCO_3$ | S6297 | 84.01 | 32.5 | 2.730 | 5.461 | 10.921 |
| Glucose | G7528 | 180.16 | 11 | 1.982 | 3.964 | 7.927 |
| Na pyruvate | P5280 | 110.04 | 2 | 0.220 | 0.440 | 0.880 |
| Na L-ascorbate | A4034 | 198.11 | 1 | 0.198 | 0.396 | 0.792 |
| Daily addition to the cutting aCSF (4°C) | | | | | | |
| Stock solution | Conc (mM) | ml/600mL | ml/1L | ml/2L | ml/4L | ml/500ml |
| 1M $MgSO_4$ | 5 | 3 | 5 | 10 | 20 | 2.5 |
| 1M $CaCl_2$ | 0.5 | 0.3 | 0.5 | 1 | 2 | 0.25 |

Composition of recording aCSF (room temperature: RT)

| Recording aCSF (22°C) | | | | | | |
|---|---|---|---|---|---|---|
| Chemical | Sigma # | F.W | Conc (mM) | g/1L | g/2L | g/4L |
| Sucrose | S7653 | 58.44 | 124 | 7.247 | 14.493 | 28.986 |
| KCl | P9333 | 74.56 | 2.5 | 0.186 | 0.373 | 0.746 |
| $NaH_2PO_4$ | S8282 | 119.98 | 1 | 0.BnH120 | 0.240 | 0.480 |
| $NaHCO_3$ | S6297 | 84.01 | 26.2 | 2.201 | 4.402 | 8.804 |
| Glucose | G7528 | 180.16 | 11 | 1.982 | 3.964 | 7.927 |
| Na pyruvate | P5280 | 110.04 | 2 | 0.220 | 0.440 | 0.880 |

(continued)

| Recording aCSF (22°C) | | | | | | |
|---|---|---|---|---|---|---|
| Chemical | Sigma # | F.W | Conc (mM) | g/1L | g/2L | g/4L |
| Na L-ascorbate | A4034 | 198.11 | 1 | 0.198 | 0.396 | 0.792 |
| Daily addition to the recording aCSF (4°C) | | | | | | |
| Stock solution | Conc (mM) | ml/600mL | ml/1L | ml/2L | ml/4L | ml/3.5L |
| 1M $MgSO_4$ | 1.3 | 0.65 | 1.3 | 2.6 | 5.2 | 4.55 |
| 1M $CaCl_2$ | 2.5 | 1.25 | 2.5 | 5 | 10 | 8.75 |

**Example 4.5. Electrophysiological experiment for barrel cortex**

All electrophysiological experiments were conducted at 27 to 29°C. For electrophysiological experiments, electrodes with 3.5-4.2 mQ resistance were used and stimuli were applied to the ventral posteromedial nucleus (VPM) using a concentric bipolar electrode (FHC, Bowdoin, ME). The somatosensory cortex was identified by the presence of barrels under 4X magnification and differential interference contrast (DIC) optics, and by the ability to evoke short and constant latency field excitatory postsynaptic potentials (fEPSPs) by VPM stimulation. Whole-cell voltage-clamp recordings were made from stellate cells (SC) in layer IV of the somatosensory barrel cortex using infrared illumination and differential interference contrast (DIC) optics. The composition of whole cell recording solution is shown as follows:

The whole cell recording internal solution

| Cs methane sulfonate-based pipet solution | | | | |
|---|---|---|---|---|
| Chemical | Sigma # | F.W | Conc (mM) | g/100mL |
| Cs-me$SO_4$ | 19823 (A) | 228 | 125 | 2.8500 |
| NaCl | S-7653 | 58.44 | 8 | 0.0468 |
| Phosphocreatine di (Tris) | P-1937 | 453.4 | 5 | 0.2267 |
| MgATP | A-9187 | 507.2 | 4 | 0.2029 |
| $Na_2$GTP | G-8877 | 523.2 | 0.4 | 0.0209 |
| EGTA | E-4378 | 380.4 | 0.5 | 0.0190 |
| HEPES | H-3375 | 238.3 | 10 | 0.2383 |
| QX314-Cl | Q-150 | 289.85 | 5 | 0.1449 |
| pH=7.35 (with CsOH at RT), QX314-Cl (purchased from Alomone Labs)<br>mOsm = ~ 285 mOsm | | | | |

Whole cell recordings were made using a multiclamp 700A (Molecular devices, Sunnyvale, CA) digitized at 10 kHz and filtered at 2 kHz. Input resistance and series resistance were monitored continuously during recordings, as previously described (see Isaac, Crair, Nicoll, & Malenka, 1997, Silent synapses during development of thalamocortical inputs, DOI: 10.1016/s0896-6273(00)80267-6). TC EPSCs were evoked at 0.1 Hz by VB stimulation and were accepted as monosynaptic if they exhibited a short and constant latency that did not change with increasing stimulus intensity as previously described (see Feldman, Nicoll, Malenka, & Isaac, 1998, Synaptic plasticity at thalamocortical synapses in developing rat somatosensory cortex: LTP, LTD, and silent synapses, https://doi.org/10.1002/(SICI)1097-4695(199910)41:1<92::AID-NEU12>3.0.CO;2U; and see Isaac et al., 1997, Silent Synapses during Development of Thalamocortical Inputs, https://doi.org/10.1016/S08966273(00)80267-6).

For the minimal stimulation protocol (Potency), VB thalamic stimulation intensity was adjusted to find the lowest intensity that elicited a mixture of synaptic responses and failures. Failure rate was calculated as the number of failures/total number of trials. Potency was calculated as the mean EPSC peak amplitude excluding failures (see Chittajallu & Isaac, 2010, Emergence of cortical inhibition by coordinated sensory-driven plasticity at distinct synaptic loci, DOI: 10.1038/nn.2639; see Isaac et al., 1997, Silent Synapses during Development of Thalamocortical Inputs, https://doi.org/10.1016/S08966273(00)80267-6; and see Stevens & Wang, 1995, Facilitation and depression at single central synapses, DOI: 10.1016/08966273(95)90223-6).

The criteria for single-axon stimulation were all or no synaptic events and no change in the mean amplitude of the EPSC evoked by small increases in stimulus intensity, as previously reported (see Chittajallu and Isaac, 2010, Emergence of cortical inhibition by coordinated sensory-driven plasticity at distinct synaptic loci, DOI: 10.1038/nn.2639; see Gil et al., 1999, Efficacy of Thalamocortical and Intracortical Synaptic Connections: Quanta, Innervation, and Reliability, https://doi.org/10.1016/50896-6273(00)80788-6). Measurement parameters are as follows:

Measurement parameters

| Measurement | Holding voltage | Record method |
|---|---|---|
| Minimal stimulation (Potency) | -70 mV | 50% on/off of 20 or 50 trials |
| GABA AMPA ratio | 0 mV/-70 mV | Average after 5 trials each |

Excitatory postsynaptic potential (EPSC) was recorded by the whole-cell patch clamp method using a patch clamp amplifier (molecular devices, USA). The measuring electrode was a borosilicate glass capillary, and a glass capillary having a resistance of 3.5 MΩ to 4.2 MΩ when the solution inside the electrode was filled was used. The brain slice in the chamber was placed on the upright microscope and fixed with an anchor to flow the recording aCSF solution at a rate of 5 to 10 mL/min by gravity. The electrode of the stimulator was placed on the thalamus fiber and connections in the S1 barrel cortex (layer 4) area and the cell in the area was found and recorded. After the whole cell patch, the membrane voltage was fixed at -70 mV (measure the AMPA current), and the stimulus was applied to check the EPSC, and the stimulator was manipulated to find the minimum stimulus intensity at which the current on/off becomes 50%. Amplitude was measured with the trace that Recording and Current for 3 minutes and 20 seconds, or 8 minutes and 20 seconds (total 20 or 50 traces). For potency, the EPSC size was set to 50 to 100 pA at -70 mV and 5 traces were measured at 0 mV.

**Example 4.6. Electrophysiological experiment for hippocampus**

Field recordings were made with a concentric bipolar electrode positioned in the stratum radiatum of the CA1 region using an extracellular glass pipette (3.5 MΩ) filled with aCSF. Specifically, fEPSPs were evoked by Schaffer collateral (SC)/commissural fibers with a concentric bipolar electrode placed 200 to 300 μm from the recording pipette. Stimulation was delivered through a bipolar electrode (FHC, Bowdoin, ME, USA) placed in the Schaffer collateral-CA1 (SC). Te SC circuit was visualized using differential interference contrast (DIC) microscopy at 4X magnification and identified by the ability to evoke short and constant latency field excitatory postsynaptic potentials (fEPSPs) at CA1 synapses by SC input stimulation. In all fEPSP experiments, the test stimulation were measured prior to the beginning of all the experiments (30 μA) and a test-pulse stimulation strength that evokes 50% of the maximum fEPSP was used. Baseline synaptic responses was recorded for 30 minutes, and then long-term potentiation (LTP) was induced by high-frequency stimulation (HFS; 100 Hz, 4 trains, 1 s duration, 20 s inter-train interval). Recordings were made every 10 seconds for 1 hour using multiclamp 700A amplifier (Molecular Devices, San Jose, CA, USA) digitized at 10 kHz, and filtered at 2 kHz with Digidata 1440 and pClamp 10.0 software (Molecular Devise, San Jose, CA, USA). Measurement parameters are as follows:

Measurement parameters

| Measurement | Clamp mode | Record method |
|---|---|---|
| Input/Output Curve | Current clamp | Input (fiber volley-amplitude)/Output fEPSP (20 to 80% fEPSP slope) slope |
| Long-term potentiation | Current clamp | Time course analysis of the % EPSP slope before (20 min) and after (60 min) TBS with the value of 50% fEPSP |

Excitatory postsynaptic potential (EPSP) was recorded by the whole-cell patch clamp method using a patch clamp amplifier (molecular devices, USA). The measuring electrode was a borosilicate glass capillary, and a glass capillary having a resistance of 3.5 MΩ -4.2 MΩ when the solution inside the electrode was filled was used. The brain slice

in the chamber was placed on the upright microscope and fixed with an anchor to flow the recording aCSF solution at a rate of 5 to 10 mL/min by gravity. The electrode of the stimulator was placed on the SC area and the connection in the CA1 area was found and recorded. As the stimulus intensity increased, the point was recorded until fEPSP did not increase or a pop spike occurred (I/O curve). Baseline synaptic responses were recorded for 20 minutes and then long-term potentiation (LTP) was induced by high-frequency stimulation (HFS; 100 Hz, 4 trains, 1 s duration, 20 s inter-train interval / TBS; 100 Hz, 10 trains, 50 ms duration, 200 ms inter-train interval). It was then recorded for 60 minutes (LTP).

**Example 4.7. Data analysis**

To perform potency analysis, the Clampfit program (Axon Instruments) was used. The baseline was set by specifying an interval between 0 and 100 ms. The trace was averaged by selecting 'On' current that comes out within 110 to 200 ms. The negative current amplitude (pA) of the average trace was checked.

Clampfit was used to analyze GABA/AMPA ratio. For AMPA current, the baseline was set by specifying an interval between 0 and 100 ms. Traces of the data within 110 to 200 ms was averaged. The negative current amplitude (pA) of the average trace was checked. For GABA current, the baseline was set by specifying an interval between 0 and 110 ms. Traces of the data within 110 to 300 ms was averaged. The positive current amplitude (pA) of the average trace was checked

Clampfit was used to calculate I/O curve. The baseline was set by specifying an interval between 0 and 6 ms. The traces recorded (in each of 5) from the same stimulus were averaged. The amplitude of fiber volley and the 20 to 80% rise slope of fEPSP were analyzed. For LTP, the baseline was set by specifying an interval between 0 and 6 ms. The 20 to 80% rise slope of fEPSP before and after LTP induction with HFS (or TBS) was analyzed.

**Example 4.8. Statistical analysis**

All data were presented as mean ± SEM, and n represents the number of cells used in each experiment. Typically, one slice was used from each animal; one recording was made from each slice. Unpaired Student's t test was used to compare mean values from the two groups and one-way ANOVA was used from three or more groups. p values < 0.05 were considered statistically significant. These statistical analyses were performed using Prism 9.0 (GraphPad Prism 9.1.2, GraphPad Software Inc. USA).

Dose response curve was drawn using Graphpad, Prism 9 according to the manufacturer's instructions.

**Example 4.9. Western blotting**

For preparation of thalamocortical (TC) slices, 9- to 10-week-old male C57BL/6J rodents (mice) were anesthetized with isoflurane. Then, the brains were rapidly cooled by transcardial perfusion with ice-cold sucrose-artificial cerebrospinal fluid (aCSF). The brains were removed and placed in ice-cold sucrose aCSF. Paracoronal slices were prepared at a 50° angle to the midline on a ramp at an angle of 10°. The slices were then incubated in aCSF for 30 minutes at 35°C to recover.

Subsequently, the slices were incubated in aCSF for 1 to 4 hours at room temperature (23 to 25°C) before being placed in the recording chamber for experiment. Standard aCSF contains saturated with 95% $O_2$/5% $CO_2$.

Composition of sucrose based aCSF

| Cutting aCSF (4°C) | | | | | | |
|---|---|---|---|---|---|---|
| Chemical | Sigma # | F.W | Conc (mM) | g/1L | g/2L | g/4L |
| Sucrose | S9378 | 342.3 | 195.5 | 66.931 | 133.862 | 267.725 |
| KCl | P9333 | 74.56 | 2.5 | 0.186 | 0.373 | 0.746 |
| $NaH_2PO_4$ | S8282 | 119.98 | 1 | 0.120 | 0.240 | 0.480 |
| $NaHCO_3$ | S6297 | 84.01 | 32.5 | 2.730 | 5.461 | 10.921 |
| Glucose | G7528 | 180.16 | 11 | 1.982 | 3.964 | 7.927 |
| Na pyruvate | P5280 | 110.04 | 2 | 0.220 | 0.440 | 0.880 |
| Na L-ascorbate | A4034 | 198.11 | 1 | 0.198 | 0.396 | 0.792 |

(continued)

| Daily addition to the cutting aCSF (4°C) | | | | | | |
|---|---|---|---|---|---|---|
| Stock solution | Conc (mM) | ml/600mL | ml/1L | ml/2L | ml/4L | ml/500ml |
| 1M $MgSO_4$ | 5 | 3 | 5 | 10 | 20 | 2.5 |
| 1M $CaCl_2$ | 0.5 | 0.3 | 0.5 | 1 | 2 | 0.25 |

Composition of recording aCSF (room temperature: RT)

| Recording aCSF (22°C) | | | | | | |
|---|---|---|---|---|---|---|
| Chemical | Sigma # | F.W | Conc (mM) | g/1L | g/2L | g/4L |
| NaCl | S7653 | 58.44 | 124 | 7.247 | 14.493 | 28.986 |
| KCl | P9333 | 74.56 | 2.5 | 0.186 | 0.373 | 0.746 |
| $NaH_2PO_4$ | S8282 | 119.98 | 1 | 0.120 | 0.240 | 0.480 |
| $NaHCO_3$ | S6297 | 84.01 | 26.2 | 2.201 | 4.402 | 8.804 |
| Glucose | G7528 | 180.16 | 11 | 1.982 | 3.964 | 7.927 |
| Na pyruvate | P5280 | 110.04 | 2 | 0.220 | 0.440 | 0.880 |
| Na L-ascorbate | A4034 | 198.11 | 1 | 0.198 | 0.396 | 0.792 |
| Daily addition to the recording aCSF (4°C) | | | | | | |
| Stock solution | Conc (mM) | ml/600mL | ml/1L | ml/2L | ml/4L | ml/3.5L |
| 1M $MgSO_4$ | 1.3 | 0.65 | 1.3 | 2.6 | 5.2 | 4.55 |
| 1M $CaCl_2$ | 2.5 | 1.25 | 2.5 | 5 | 10 | 8.75 |

All electrophysiological experiments were conducted at 27 to 29°C. Rodent (mice) was perfused with flowing blood from a sucrose-based aCSF solution (4°C) using gravity. The brain of the rodent (mice) was removed and cut into 400 um at a 50° angle (mouse at a 55° angle) to attach the cut portion to the vibratome disc to be the bottom. Brain slice was incubated in Incubate aCSF solution at 35°C for 30 minutes or Incubate aCSF solution at RT for 1 hour. Cortical tissue from mice was prepared according to protocol above then was homogenized and lysed for protein extraction. The protein lysate was prepared from the tissue with NP40(FNN0021, Invitrogen) protein lysis buffer containing protease & phosphate inhibitor according to the manufacturer's instruction. Protein concentrations were determined using the BCA Protein Assay Kit (Pierce Biotechnology) with bovine serum albumin as a standard. Equal amounts of proteins (20 $\mu$g per lane) were separated by electrophoresis on 8% or 12% SDS-polyacrylamide gel, followed by blotting onto PVDF membranes (GE Healthcare). To block nonspecific binding, the membranes were incubated for 1 h at room temperature in 5% non-fat dry milk in PBST (150 mM NaCl, 50 mM Tris, pH 7.4, 0.05% Tween 20). The following antibodies were incubated overnight at 4°C in 2% blocking solution: Anti-HDAC4 (SAB4300413, Sigma-Aldrich), Anti-BDNF (ANT-010, Alomone Las), Anti-NMDAR2B (ab65783, abcam plc). Following the incubation with antibodies and several rinses in PBST, the membranes were incubated for 1 h with the mouse anti-rabbit IgG-HRP conjugated secondary anti-bodies (sc-2357, Santa Cruz). Each blot was subsequently re-probed with anti-$\beta$-actin antibody (sc-47778, Santa Cruz). Housekeeping protein $\beta$ actin was detected as a normalization control. Antibodies used for methods described herein are as follows:

| | | |
|---|---|---|
| HDAC4 | $1^{st}$ Ab | SIGMA (SAB4300413) |
| | $2^{nd}$ Ab | Santa Cruz (sc 2357) |
| BDNF | $1^{st}$ Ab | Alomone labs (ANT-010) |
| | $2^{nd}$ Ab | Santa Cruz (sc 2357) |
| GluN2B | $1^{st}$ Ab | Abcam (ab65783) |
| | $2^{nd}$ Ab | Santa Cruz (sc 2357) |

(continued)

| | | |
|---|---|---|
| β-actin | 1st Ab | Santa Cruz (sc 47778) |
| | 2nd Ab | Santa Cruz (sc-020) |

**Example 5. Therapeutic efficacy in PTSD**

**Example 5.1. Animal obtained and management**

The animals were obtained from the DBL. Animals are obtained 7 days before the test start. Twenty male SD-rats aged about 7 weeks were used. Animals were maintained in conventional environmental conditions according to the 12/12 hours light-dark cycle in the colony room, with access to food and water *ad libitum.* The rats were acclimatized to their new housing cage over a period of 7 days. The animals were carried out for all tests. All experiments were performed in accordance with the Guide for the Care and Use of Laboratory Animals, and all efforts were made to minimize animal suffering. Weight was measured at 3-day intervals from Day 7.

**Example 5.2. Rat Modeling of PTSD**

This protocol involves two times exposure to foot shock without an opportunity of escape.

The Context A (Day -1)

The weight was measured three hours before the experiment. The temperature of the laboratory was maintained at 22°C. The animals were placed in the dark laboratory space for 10 minutes to adapt. The animals were each placed in the first room. Two minutes later, a door separating the two compartments was opened, and let the animals move freely on both sides. After 5 minutes, 15 times foot shocks of 1 mA/2 sec were applied at random time (90 to 270 seconds) intervals. After the test was completed, the animal was left in the box for one minute and moved to the colony room.

The Context B (Day 0)

The weight was measured three hours before the experiment. The temperature of the laboratory was maintained at 22°C. The animals were placed in the dark laboratory space for 10 minutes to adapt. The animal was placed in the first compartment with sandpapers attached. At this time, the door remained closed, and thus the animals were not allowed to cross to the second compartment. After 10 minutes, 3 times foot shocks of 2 mA/sec were applied to the animal. After the test was completed, the animal was left in the box for one minute and moved to the colony room.

Compound injection

The compound was diluted to 25 mg/kg using DMSO:PEG400:Saline (5:55:40). From Day 7 for 24 days the compound was administered intraperitoneal at 10:00 a.m. daily (*i.p.* volume was 200 μl). Control group was also administered by *i.p.* injection in the same manner using the vehicle (DMSO:PEG400:Saline (5:55:40)).

Freezing check

For the freezing check, 7 days after the initial foot shock protocol, the rats were placed in the first compartment with sandpaper of the apparatus for 5 minutes (no foot shock) and the freezing behavior was recorded using shutavoid_v1803 program (Panlab, Spain) according to the manufacturer's instruction. At this time, the door remained closed, like the context B. Freezing was defined as a state without skeletal muscle movement of the rat. The animal with a freezing time of 240 seconds or less were excluded. Excluded animals were sacrificed using $CO_2$.

Data analysis

In front of the clear acrylic plate of the electronic box, a camera was placed to check all animal movements. All groups were freezing checked every three days from Day 7. Each animal was film for 5 minutes. Based on the video, the freezing time of each animal was checked. The freezing time for Day 7 was set to 100%, and then the freezing time for each day was converted into a percentage.

**Example 6. Therapeutic efficacy in AD**

**Example 6.1. Animal obtained and management**

APP/PS1 mice, which is an animal model of Alzheimer's disease, were obtained from Taconic Bioscience. Animals are obtained 7 days before the test start. Twenty male SD-rats aged about 15 months were used. Animals were maintained in conventional environmental conditions according to the 12/12 hours light-dark cycle in the colony room, with access to food and water *ad libitum.* The mice were acclimatized to their new housing cage over a period of 7 days. The animals were carried out for all tests. All experiments were performed in accordance with the Guide for the Care and Use of Laboratory Animals, and all efforts were made to minimize animal suffering. Weight was measured at 3-day intervals from Day 7.

**Example 7. Selection of BnH12003**

**Example 7.1. *In vitro* cytotoxicity**

The cytotoxicity of the compound was evaluated by treating cells with BnH12003 and measuring cell viability.
As a result of measuring cell viability in response to BnH12003 (0, 0.1, 1, 10, 100 μM) treatment in human cell lines 293T and SH-SY5Y, no significant change in cell viability was observed in all concentration ranges, confirming the cytotoxic safety of BnH12003 (FIGS. 1A and 1B).

**Example 7.2. Analysis of efficacy in enhancing *ex vivo* synaptic plasticity**

After administering BnH12003, the potency and GABA/AMPA ratio in the barrel cortex were measured to confirm efficacy of the compound.
To this end, 8-week-old C57BL/6J mice were grouped into a control group (vehicle administration group) and an experimental group (BnH12003 administration group) by 5 mice each. After Alzet Os pump injection surgery, BnH12003 was directly injected into layer 4 of the barrel cortex at a total dose of 3 μM continuously for 2 weeks using an Os pump (FIGS. 2A and 2B).
Upon administration of the compound BnH12003, as confirmed by the measurement of the GABA/AMPA ratio in the mouse barrel cortex (layer 4), it was confirmed that while the balance of excitation/inhibition was maintained (FIG. 3A), the excitatory TC synaptic potency (TC potency) was significantly increased (FIG. 3B). These results show that BnH12003 exhibits efficacy of improving cognitive ability by increasing synaptic plasticity. Meanwhile, 8-week-old C57BL/6J mice were grouped into a control group (vehicle administration group) and an experimental group (BnH12003 administration group) by 5 mice each, and administered intraperitoneally at a dose of 25 mg/kg once a day for about 11 or 12 days. As a result, it was confirmed that administration of BnH12003 could increase TC synaptic potency in the sensory cortex while maintaining the balance of excitation/inhibition (FIGS. 4A and 4B).

**Example 7.3. Analysis of protein degradation efficacy of HDAC4**

The BnH12 series according to the present disclosure were synthesized as compounds that target HDAC4, and it was intended to determine whether BnH12003 degrades HDAC4 at a protein level. The barrel cortex was isolated from the mice in Example 7.2 and the HDAC4 protein level was determined according to the presence or absence of BnH12003 treatment. As a result, it was confirmed that the HDAC4 protein level decreased by 60% to 80% in the mice of all experimental groups compared to the mice of the control group (FIG. 5). From these results, it was confirmed that BnH12003 regulates the HDAC4 protein level and increases synaptic plasticity, thereby improving cognitive function.

**Example 7.4. Analysis of efficacy of treating post-traumatic stress disorder using animal model of post-traumatic stress disorder**

Since the HDAC4 gene has been studied to be closely associated with the fear response in the brain, it was intended to observe the therapeutic efficacy of BnH12003 administration using an animal model of post-traumatic stress disorder (PTSD).
To this end, SD rodents (rats) (300 g) were acclimated for 3 days. Before entering the experiment, the experimental rats were placed in a chamber for 5 minutes to test for freezing behavior, during which photographs were taken. In a chamber surrounded by coarse sandpaper, electrical stimulation was randomly applied to the sole of the rat's paw with 15 shocks at intervals of 90 to 270 seconds at an intensity of 1 mA and a duration of 2 seconds. The next day, in a chamber surrounded by coarse sandpaper, electrical stimulation was applied to the sole of the rat's paw with 3 shocks at

intervals of 3 seconds at an intensity of 2 mA and a duration of 2 seconds. PTSD rodents (rats) were selected by checking the freezing time on day 7. The selected rodents (rats) were grouped into a control group and an experimental group by 12 mice each, and BnH12003 at 1 μM was administered into the ventricles of the rodents (rats) using an Os pump. On day 10 after the production of the rodent (rat) PTSD model, saline was administered intraperitoneally to the control group, and BnH12003 (25 mg/kg) was administered intraperitoneally to the experimental group, and then the freezing time was checked at 3-day intervals. The freezing time was checked a total of 8 times, and the decrease in freezing time was calculated as a percentage (%) based on day 7 after the production of the rodent (rat) PTSD model.

As a result, it was confirmed that the experimental group administered with BnH12003 showed a significant decrease in freezing time compared to the control group (FIG. 6), and it was confirmed that when BnH12003 was administered to rats with traumatic memory (fear memory), it exhibited an effect of decreasing or eliminating traumatic memory (fear memory). Therefore, it was confirmed that when the compound of the present disclosure was used, the learning effect of erasing traumatic memory (fear memory) or new memory (a place that is no longer dangerous) was enhanced.

**Example 7.5. Analysis of efficacy of enhancing *ex vivo* synaptic plasticity in Alzheimer's animal model**

After administration of BnH12003 to APP/PS1 mice (Taconic Bioscience), which are an animal model of Alzheimer's disease, it was attempted to determine efficacy of the compound against Alzheimer's disease by measuring the potency and GABA/AMPA ratio in the hippocampus, and in particular, to compare the effects obtained by single and combinational administration of BnH12003 and solanezumab. To this end, BnH12003 and solanezumab (Creative Biolabs, USA) were administered to the hippocampus at doses of 3 μM and 200 μg, respectively, by Os pump ventricular infusion as follows: As a result of observing changes in the I/O curve of field excitatory postsynaptic potential (EPSP) in the CA1 region of the Schaffer collateral circuit in the hippocampus, it was confirmed that synaptic efficacy was increased by *in vivo* administration of BnH12003 (FIG. 7A). In addition, as a result of measuring synaptic long-term potentiation (LTP) of EPSP in the CA1 region of the Schaffer collateral circuit in the hippocampus, it was also confirmed that LTP of the excitatory SC pathway was also significantly increased (FIG. 7B).

Since increases in I/O curve and LTP indicate that synaptic plasticity is increased, it was found from these results that the combinational administration of BnH12003 and solanezumab increased the SC pathway synaptic potency in the hippocampus, which indicates that BnH12003 is effective in treating Alzheimer's disease. In this case, the combinational administration of BnH12003 and solanezumab showed a better effect than the single administration of either.

Meanwhile, after administering BnH12003 to APP/PS1 mice, it was attempted to confirm the compound efficacy for Alzheimer's disease by measuring the potency and GABA/AMPA ratio in the barrel cortex, and in particular, it was attempted to compare the effects of the single administration and combinational administration of BnH12003 and solanezumab.

When BnH12003 was administered to APP/PS1 mice, *in vivo* administration of BnH12003 increased the excitatory TC synaptic potential (TC potential) (FIG. 8B) while maintaining the balance of excitation/inhibition (FIG. 8A), as confirmed by the measurement of the GABA/AMPA ratio. Since an increase in TC potency indicates that synaptic plasticity is increased, it was found from these results that the administration of the compound BnH12003 increased the TC synaptic potency in the sensory cortex, while maintaining the balance of excitation/inhibition, which indicates that BnH12003 is effective in treating Alzheimer's disease. In this case, a significantly excellent effect was exhibited when BnH12003 and solanezumab were co-administered compared to when BnH12003 and solanezumab were administered alone, respectively.

**Example 8. Surface plasmon resonance assay**

Compounds of the present disclosure were selected for their ability to interact with HDAC4. To conduct the test, surface plasmon resonance assay was used. The samples were prepared as follows and loaded onto Biacore T200.

| Type | Sample | Stock | Solvent | M.W. |
|---|---|---|---|---|
| Ligand 1 | HDAC4 | 20 ug | 25 mM Tris-HCl, pH 7.3, 100 mM glycine, 10% glycerol | 118.9 kDa |
| Analyte | BnHR12003 | 3.2 mg | DMSO | 339.39 Da |

The samples were immobilized under the following conditions. The protein stock was diluted with 10 mM sodium acetate. 1 M ethanolamine (pH 8.5) was used as a blocking agent.

| Chip No | Ligand | Cone. | Immobilization Buffer | Immobilization level |
|---|---|---|---|---|
| #2 | HDAC4 | 50 ug/mL | Sodium acetate, pH 5.0 | 1489.5 RU |

FIG. 9 illustrates the binding kinetics of BnH12003 to HDAC4.

**Example 9. Effect of compounds on HDAC catalytic activities**

The compound was resuspended in 100 mM DMSO and tested in a 10-dose $IC_{50}$ with 3-fold serial dilution starting at 100 μM against HDACs 1-9, and 11. As reference compounds, Trichostatin A (TSA) and TMP269 were tested in a 10-dose $IC_{50}$ with 3-fold serial dilution starting at 10 μM or 1 μM.

10 μM Fluorogenic peptide from p53 residues 379-382 (RHKK(Ac)AMC) was used for the substrate for HDAC 1, 2, 3, and 6. 50 μM Fluorogenic HDAC Class2a Substrate (Trifluoroacetyl Lysine) was used for the substrate for HDAC 4, 5, 7, 9, and 11. 100 μM Fluorogenic peptide from p53 residues 379-382 (RHK(Ac)K(Ac)AMC) was used for the substrate for HDAC8.

$IC_{50}$ values were calculated using the GraphPad Prism 4 program based on a sigmoidal dose-response equation and the blank (DMSO) value concentration was entered as 1.00E-12 M for curve fitting. Curve fits were performed for compound data sets that displayed enzyme activity less than 65% at the highest concentration of compound tested.

The experiments were performed in duplicates. BnH12003 did not show any inhibitory effect on HDAC 1 to 9, and 11's catalytic activities.

**Example 10**. **Effects on expressions of BDNF and GluN2B, and thalamocortical EPSC potency**

When HDAC4 protein expression was suppressed by transfecting lentiviral HDAC4 shRNA, BDNF and GluN2B expression was significantly increased in SH-SY5Y cells (FIGS. 10A and 10B). Similarly, BnH12003 (1 μM) augmented BDNF and GluN2B expression by application for 6 and 24 hours in SH-SY5Y cells (FIGS. 11A and 11B). BnH12003 significantly suppressed HDAC4 protein expression by oral and intraperitoneal administration, respectively, in mice barrel cortex and this inhibitory effect was maintained at least for 10 days (FIGS. 12A and 12B).

**Example 11. *In vitro* cytotoxicity of various BnH12 series compounds**

By treating cells with various BnH12 series compounds and measuring cell viability using the experimental procedure described in Example 2, the cytotoxicity of the compounds was evaluated. As a result, the cells showed a cell viability of 85% or higher for almost all compounds at a concentration of 10 μM, and showed a cell viability of 80% or higher even when the concentration was increased to 100 μM.

[Table 4]

| Compound name | Cell viability test results (CCK) | |
|---|---|---|
| | 10uM | 100uM |
| BnH12002 | 90% < | 95% < |
| BnH12003 | 99% < | 90% < |
| BnH12015 | 99% < | 99% < |
| BnH12017 | 95% < | 80% < |
| BnH12036 | 95% < | 95% < |
| BnH12037 | 99% < | 99% < |
| BnH12042 | 95% < | 90% < |
| BnH12043 | 99% < | 99% < |
| BnH12044 | 80% < | 75% < |
| BnH12045 | 99% < | 99% < |
| BnH12046 | 95% < | 90% < |
| BnH12047 | 99% < | 99% < |

(continued)

| Compound name | Cell viability test results (CCK) | |
|---|---|---|
| | 10uM | 100uM |
| BnH12048 | 95% < | 80% < |
| BnH12049 | 90% < | 85% < |
| BnH12051 | 99% < | 99% < |
| BnH12052 | 80% < | 75% < |
| BnH12053 | 95% < | 95% < |
| BnH12064 | 99% < | 99% < |
| BnH12055 | 90% < | 60% < |
| BnH12056 | 95% < | 90% < |
| BnH12057 | 95% < | 55% < |
| BnH12058 | 99% < | 90% < |
| BnH12059 | 90% < | 70% < |
| BnH12064 | 99% < | 30% < |
| BnH12066 | 99% < | 90% < |
| BnH12067 | 99% < | 85% < |
| BnH12071 | 80% < | 75% < |
| BnH12072 | 90% < | 80% < |
| BnH12077 | 99% < | 60% < |
| BnH12078 | 90% < | 75% < |
| BnH12080 | 85% < | 85% < |
| BnH12081 | 85% < | 70% < |
| BnH12082 | 90% < | 90% < |
| BnH12083 | 95% < | 75% < |
| BnH12084 | 80% < | 70% < |
| BnH12085 | 30% < | 0% < |
| BnH12086 | 80% < | 70% < |
| BnH12087 | 85% < | 45% < |
| BnH12088 | 90% < | 90% < |
| BnH12089 | 65% < | 55% < |
| BnH12090 | 85% < | 30% < |
| BnH12091 | 95% < | 85% < |
| BnH12092 | 90% < | 75% < |
| BnH12093 | 85% < | 80% < |
| BnH12095 | 80% < | 75% < |
| BnH12097 | 90% < | 85% < |
| BnH12098 | 80% < | 80% < |

As a result of measuring cell viability depending on the compound concentration (10 or 100 μM) in 293T cell line, most compounds showed no significant change in cell viability in both concentration ranges.

**Example 12. Confirmation of efficacy of various BnH12 series compounds**

Using the experimental procedure described in Example 3, it was determined whether various BnH12 series compounds increase GluN2B expression, and the results are shown in Table 5. These *in vitro* experimental results are expressed as relative values to the group (vehicle) that was not treated with the compound, with the effect of the group set to 1.

[Table 5]

| Compound name | Western blot results | |
|---|---|---|
| | Mean | Standard Error |
| BnH12003 | 2.141 | 0.206 |
| BnH12017 | 1.448 | 0.136 |
| BnH12045 | 1.341 | 0.267 |
| BnH12046 | 2.102 | 0.176 |
| BnH12047 | 1.554 | 0.316 |
| BnH12048 | 1.071 | 0.101 |
| BnH12051 | 1.391 | 0.187 |
| BnH12053 | 1.652 | 0.097 |
| BnH12057 | 2.241 | 0.067 |
| BnH12058 | 1.54 | 0.188 |
| BnH12064 | 1.39 | 0.152 |
| BnH12066 | 1.514 | 0.1268 |
| BnH12067 | 2.565 | 0.3455 |
| BnH12071 | 1.799 | 0.236 |
| BnH12072 | 1.822 | 0.2648 |
| BnH12078 | 1.017 | 0.08378 |
| BnH12085 | 1.557 | 0.2962 |
| BnH12086 | 1.753 | 0.6517 |
| BnH12088 | 1.219 | 0.1464 |
| BnH12089 | 1.409 | 0.3554 |
| BnH12090 | 1.698 | 0.2045 |
| BnH12091 | 5.013 | 0.4873 |
| BnH12092 | 3.524 | 0.09348 |
| BnH12093 | 1.611 | 0.3473 |
| BnH12095 | 2.871 | 0.03571 |
| BnH12097 | 2.625 | 0.05012 |
| BnH12098 | 2.863 | 0.4837 |

As can be seen from Table 5, the representative compounds of the present disclosure showed superior effects compared to the vehicle. In particular, BnH12003, BnH12046, BnH12057, BnH12067, BnH12091, BnH12092, BnH12095, BnH12097, and BnH12098 showed excellent effects compared to the other compounds.

## Claims

**1.** A compound of Formula (1), a stereoisomer thereof, or a pharmaceutically acceptable salt thereof:

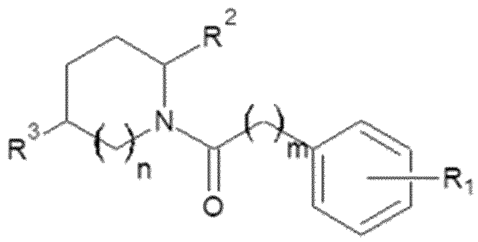

(1)

in the formula, $R^1$ is aryl or heteroaryl, or is combined with an adjacent phenyl group to form a saturated or partially saturated heterocycle, wherein at least one hydrogen atom in the aryl, heteroaryl, or heterocycle is unsubstituted or substituted with a halogen atom, $C_{1-6}$ alkyl, $C_{3-12}$ cycloalkyl, $C_{1-6}$ alkoxy, aryl, heteroaryl, amino, or nitro, and the substituent is unsubstituted or substituted with at least one halogen atom or $C_{1-6}$ alkyl,
$R^2$ is hydrogen, $C_{1-6}$ alkyl, or aryl,
$R^3$ is hydrogen, carboxyl, N-hydroxycarboxamide, or carboxamide, wherein hydrogen in the group may be substituted with $C_{1-6}$ alkyl,
n is 0 or 1, and
m is 0 to 6.

**2.** The compound of claim 1, a stereoisomer thereof, or a pharmaceutically acceptable salt thereof, wherein $R^1$ is phenyl, pyridinyl, pyridazinyl, pyrimidinyl, pyrazinyl, furanyl, thiophenyl, pyrrolyl, imidazolyl, or oxazolyl, or is combined with an adjacent phenyl group to form quinoxalinyl or dihydrobenzodioxynyl.

**3.** The compound of claim 2, a stereoisomer thereof, or a pharmaceutically acceptable salt thereof, wherein at least one hydrogen atom in $R^1$ is substituted with a halogen atom, $C_{1-6}$ alkyl or $C_{3-12}$ cycloalkyl unsubstituted or substituted with at least one halogen atom, $C_{1-6}$ alkoxy, phenyl, pyridinyl, pyridazinyl, pyrimidinyl, amino unsubstituted or substituted with at least one $C_{1-6}$ alkyl, or nitro.

**4.** The compound of claim 1, a stereoisomer thereof, or a pharmaceutically acceptable salt thereof, wherein $R^1$ is phenyl, biphenyl, pyridin-4-yl, pyridin-3-yl, pyridin-2-yl, methyl pyridin-3-yl, 6-methoxy-pyridin-3-yl, 6-cyclopropyl-pyridin-3-yl, trifluoromethylpyridin-3-yl, phenylpyridin-3-yl, pyridazin-4-yl, pyridazin-3-yl, pyridazin-2-yl, pyrimidin-2-yl, pyrimidin-4-yl, pyrimidin-5-yl, methylpyrimidin-5-yl, 5-fluoropyrimidin-2-yl, 2-(trifluoromethyl)pyrimidin-5-yl, 2-cyclopropylpyrimidin-5-yl, pyrazin-2-yl, thiophen-2-yl, furan-2-yl, oxazol-2-yl, 3-nitrophenyl, 4-nitrophenyl, 3-aminophenyl, 4-aminophenyl, or 4-diethylaminophenyl.

**5.** The compound of claim 1, a stereoisomer thereof, or a pharmaceutically acceptable salt thereof, wherein $R^2$ is H or $C_{1-6}$ alkyl.

**6.** The compound of claim 1, a stereoisomer thereof, or a pharmaceutically acceptable salt thereof, wherein $R^3$ is H, carboxyl, methyl carboxyl, ethyl carboxyl, N-hydroxycarboxamide, or carboxamide.

**7.** The compound of claim 1, a stereoisomer thereof, or a pharmaceutically acceptable salt thereof, wherein the stereoisomer is an optical isomer.

**8.** The compound of claim 1, a stereoisomer thereof, or a pharmaceutically acceptable salt thereof, wherein the stereoisomer is (3R,6S)-optical isomer.

**9.** The compound of claim 1, a stereoisomer thereof, or a pharmaceutically acceptable salt thereof, wherein the compound is selected from the following compounds:

| Name | Structure | Name | Structure |
|---|---|---|---|
| (1) BnH 12002 | 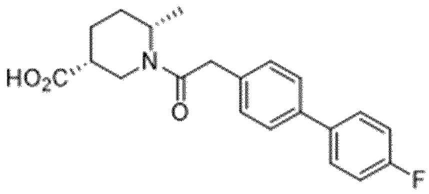 | (2) BnH 12003 | 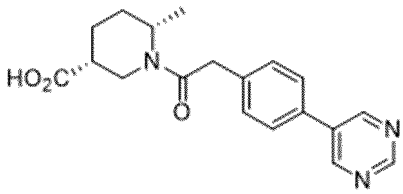 |
| (3) BnH 12015 | 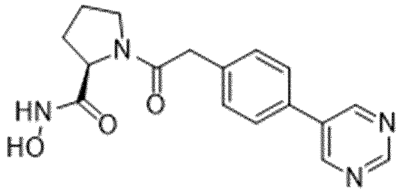 | (4) BnH 12017 | 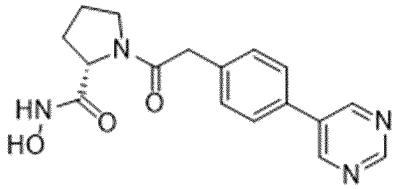 |
| (5) BnH 12036 | 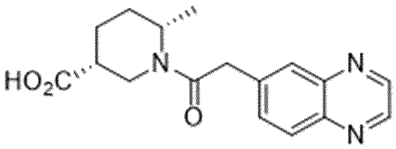 | (6) BnH 12037 | 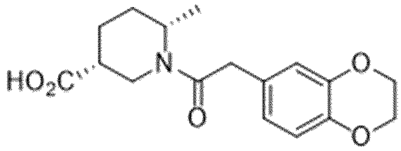 |
| (7) BnH 12042 | 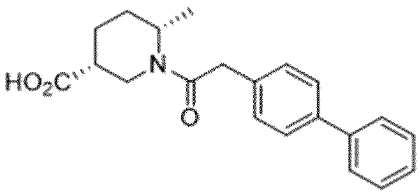 | (8) BnH 12043 | 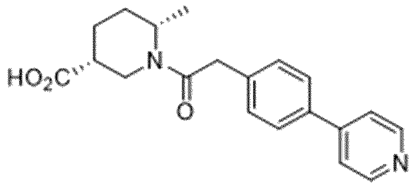 |
| (9) BnH 12044 | 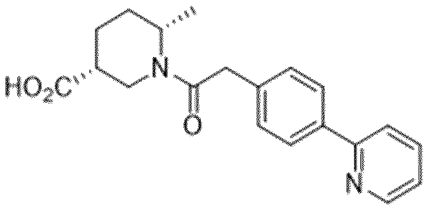 | (10) BnH 12045 | 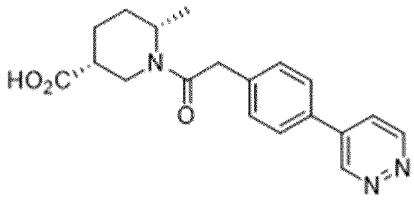 |
| (11) BnH 12046 | 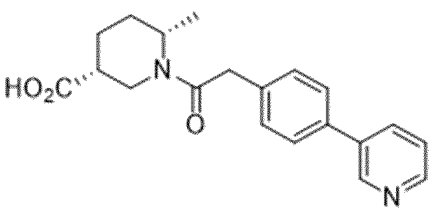 | (12) BnH 12047 | 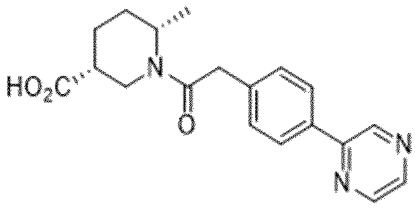 |
| (13) BnH 12048 | 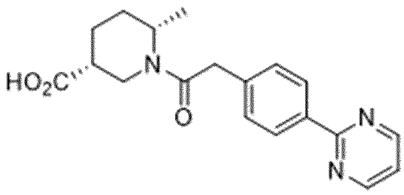 | (14) BnH 12049 | 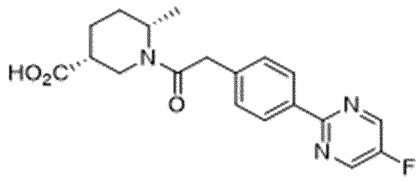 |

(continued)

| Name | Structure | Name | Structure |
|---|---|---|---|
| (15) BnH 12050 | 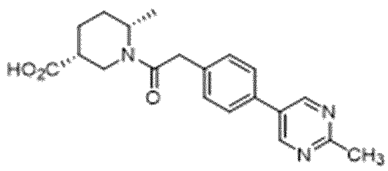 | (16) BnH 12051 | 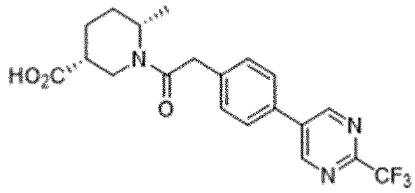 |
| (17) BnH 12052 | 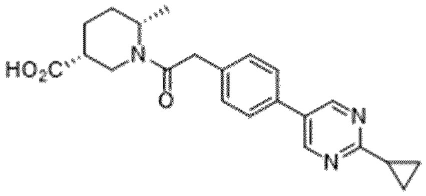 | (18) BnH 12053 | 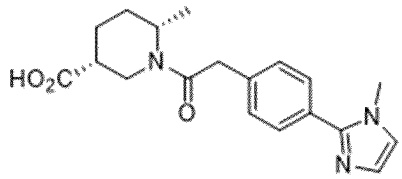 |
| (19) BnH 12054 | 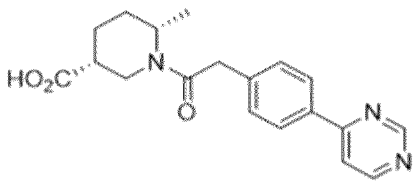 | (20) BnH 12055 | 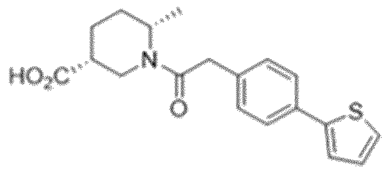 |
| (21) BnH 12056 | 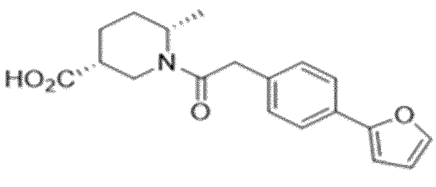 | (22) BnH 12057 | 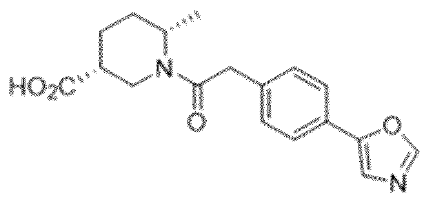 |
| (28) BnH 12058 | 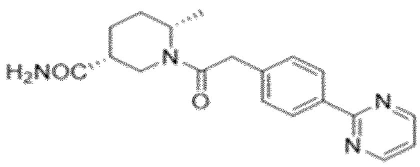 | (24) BnH 12059 | 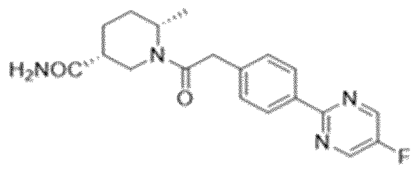 |
| (25) BnH 12064 | 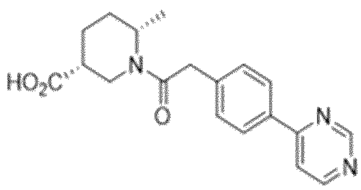 | (26) BnH 12066 | 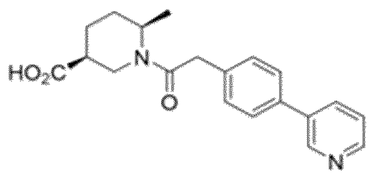 |
| (27) BnH 12067 | 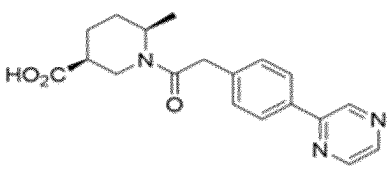 | (28) BnH 12071 | 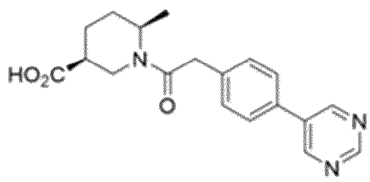 |

(continued)

| Name | Structure | Name | Structure |
|---|---|---|---|
| (29) BnH 12072 | 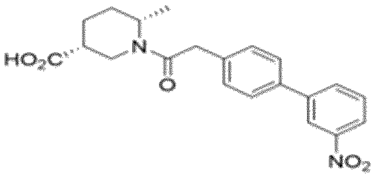 | (30) BnH 12077 | 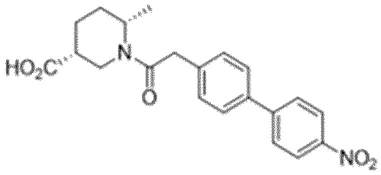 |
| (31) BnH 12078 | 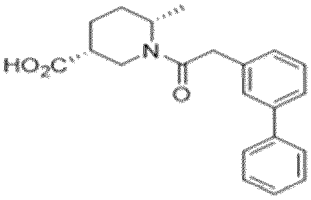 | (32) BnH 12080 | 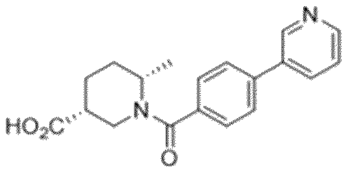 |
| (33) BnH 12081 | 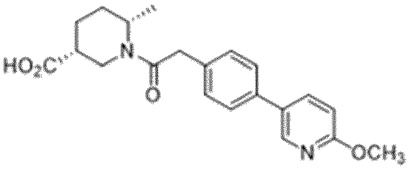 | (34) BnH 12082 | 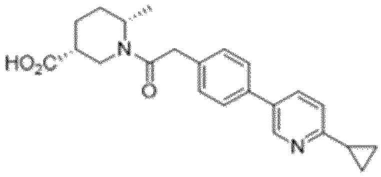 |
| (85) BnH 12083 | 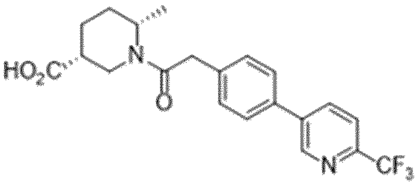 | (36) BnH 12084 | 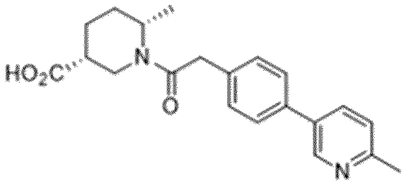 |
| (37) BnH 12085 | 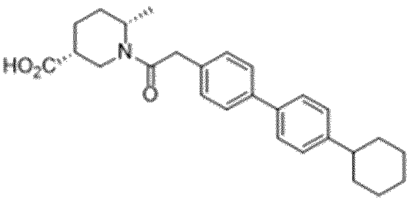 | (38) BnH 12086 | 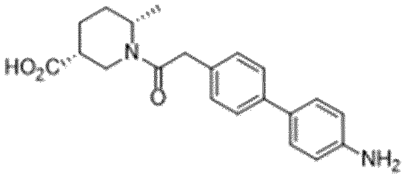 |
| (39) BnH 12087 | 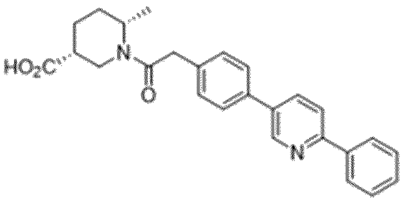 | (40) BnH 12088 | 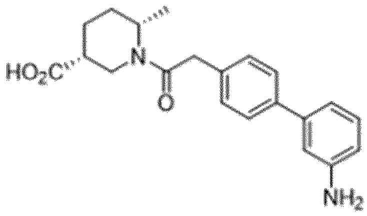 |
| (41) BnH 12089 | 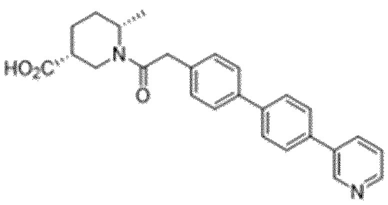 | (42) BnH 12090 | 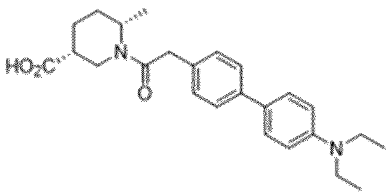 |

(continued)

| Name | Structure | Name | Structure |
|---|---|---|---|
| (43) BnH 12091 | 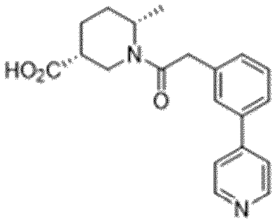 | (44) BnH 12092 | 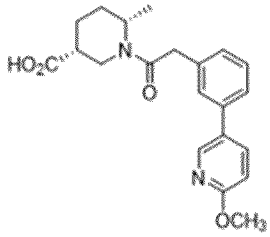 |
| (45) BnH 12093 | 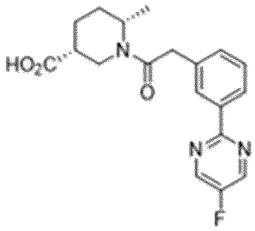 | (45) BnH 12095 | 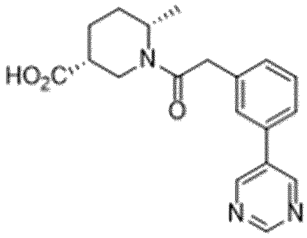 |
| (47) BnH 12097 | 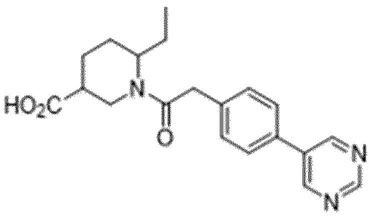 | (48)BnH 12098 | 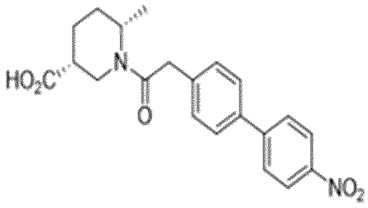 |

**10.** The compound of claim 9, a stereoisomer thereof, or a pharmaceutically acceptable salt thereof, wherein the compound is selected from the group consisting of BnH12003, BnH12017, BnH12045, BnH12046, BnH12047, BnH12048, BnH12051, BnH12053, BnH12057, BnH12058, BnH12064, BnH12066, BnH12067, BnH12071, BnH12072, BnH12078, BnH12085, BnH12086, BnH12088, BnH12089, BnH12090, BnH12091, BnH12092, BnH12093, BnH12095, BnH12097, and BnH12098.

**11.** The compound of claim 1, a stereoisomer thereof, or a pharmaceutically acceptable salt thereof, wherein the compound is an inhibitor of HDAC.

**12.** The compound of claim 11, a stereoisomer thereof, or a pharmaceutically acceptable salt thereof, wherein the HDAC is HDAC4.

**13.** The compound of claim 1, a stereoisomer thereof, or a pharmaceutically acceptable salt thereof, wherein the compound increases synaptic plasticity.

**14.** A pharmaceutical composition comprising, as an active ingredient, the compound of any one of claims 1 to 13, a stereoisomer thereof, or a pharmaceutically acceptable salt thereof.

**15.** The pharmaceutical composition of claim 14, wherein the composition is for prevention or treatment of a neurological disorder or a neurodegenerative disorder.

**16.** The pharmaceutical composition of claim 14, wherein the compound, a stereoisomer thereof, or a pharmaceutically acceptable salt thereof is delivered to the brain at a concentration of about 0.1 to about 3 $\mu$M.

**17.** The pharmaceutical composition of claim 15, wherein the disorder is Alzheimer's disease.

**18.** The pharmaceutical composition of claim 15, wherein the disorder is PTSD.

**19.** The pharmaceutical composition of claim 14, further comprising a second therapeutic agent.

**20.** The pharmaceutical composition of claim 14, wherein the second therapeutic agent is solanezumab.

**21.** A method for preventing or treating a neurological disorder or a neurodegenerative disorder, comprising administering, to a subject in need thereof, the compound according to any one of claims 1 to 13, a stereoisomer thereof, or a pharmaceutically acceptable salt thereof.

[FIG. 1A]

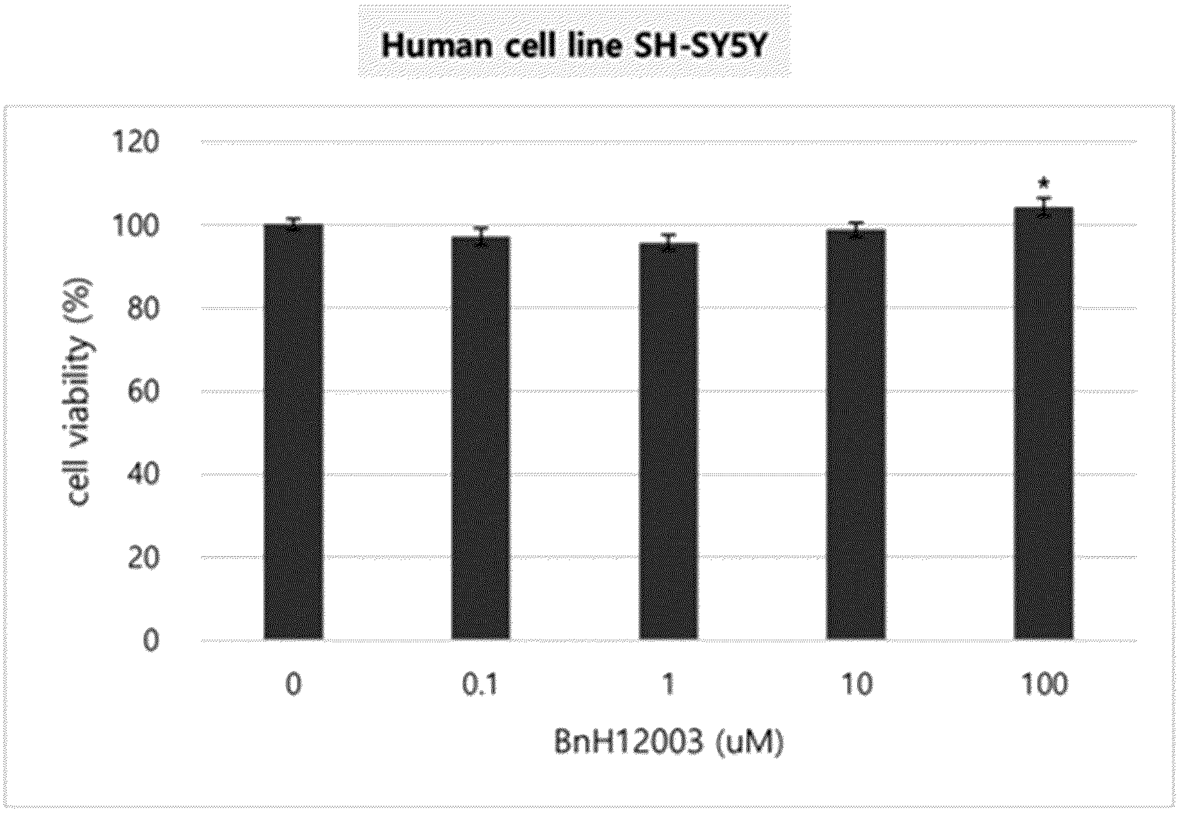
Human cell line SH-SY5Y
120
100
80
60
40
20
0
cell viability (%)
*
0
0.1
1
10
100
BnH12003 (uM)

[FIG. 1B]

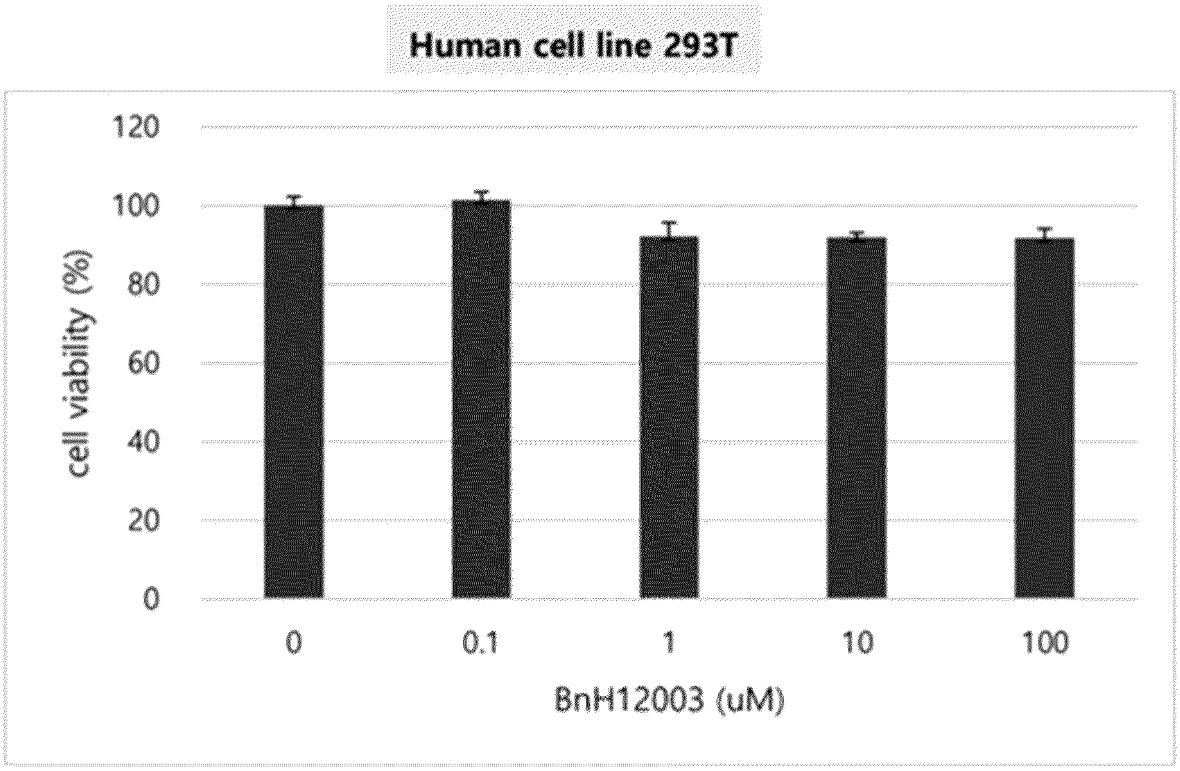
Human cell line 293T
120
100
80
60
40
20
0
cell viability (%)
0
0.1
1
10
100
BnH12003 (uM)

[FIG. 2A]

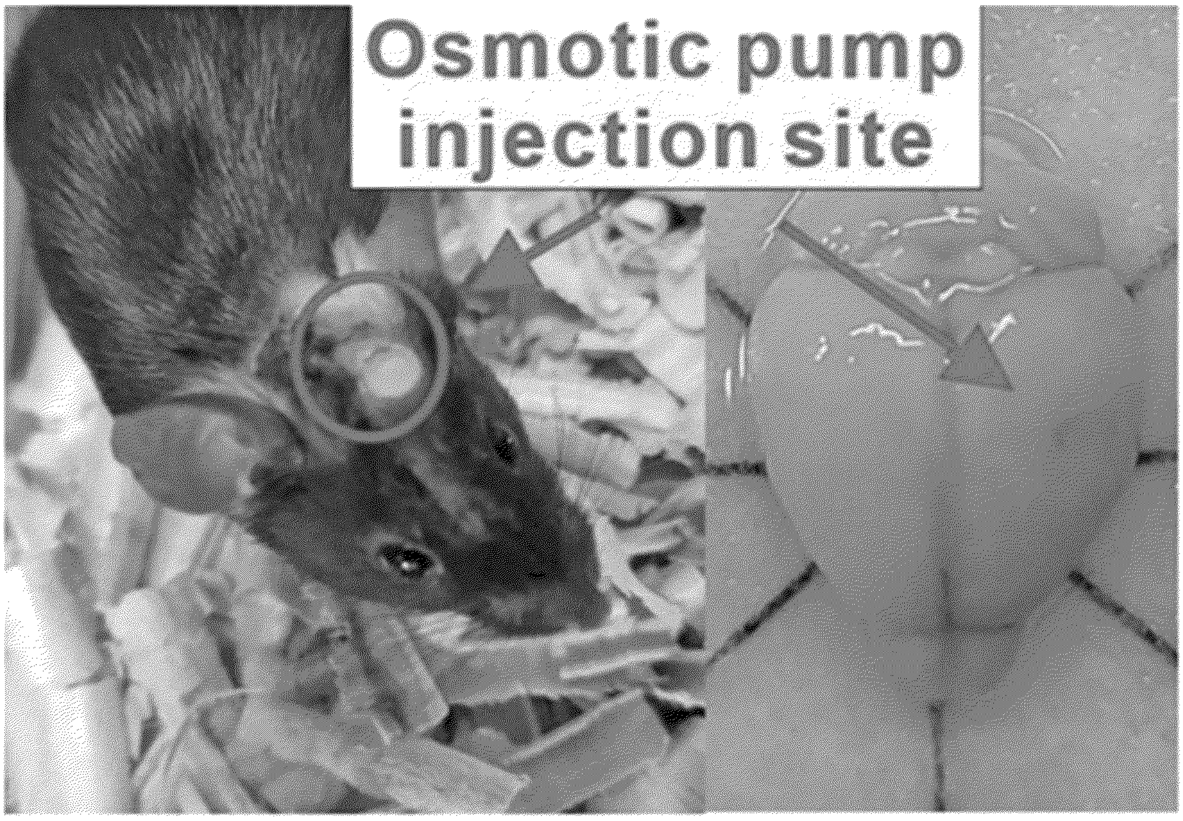

[FIG. 2B]

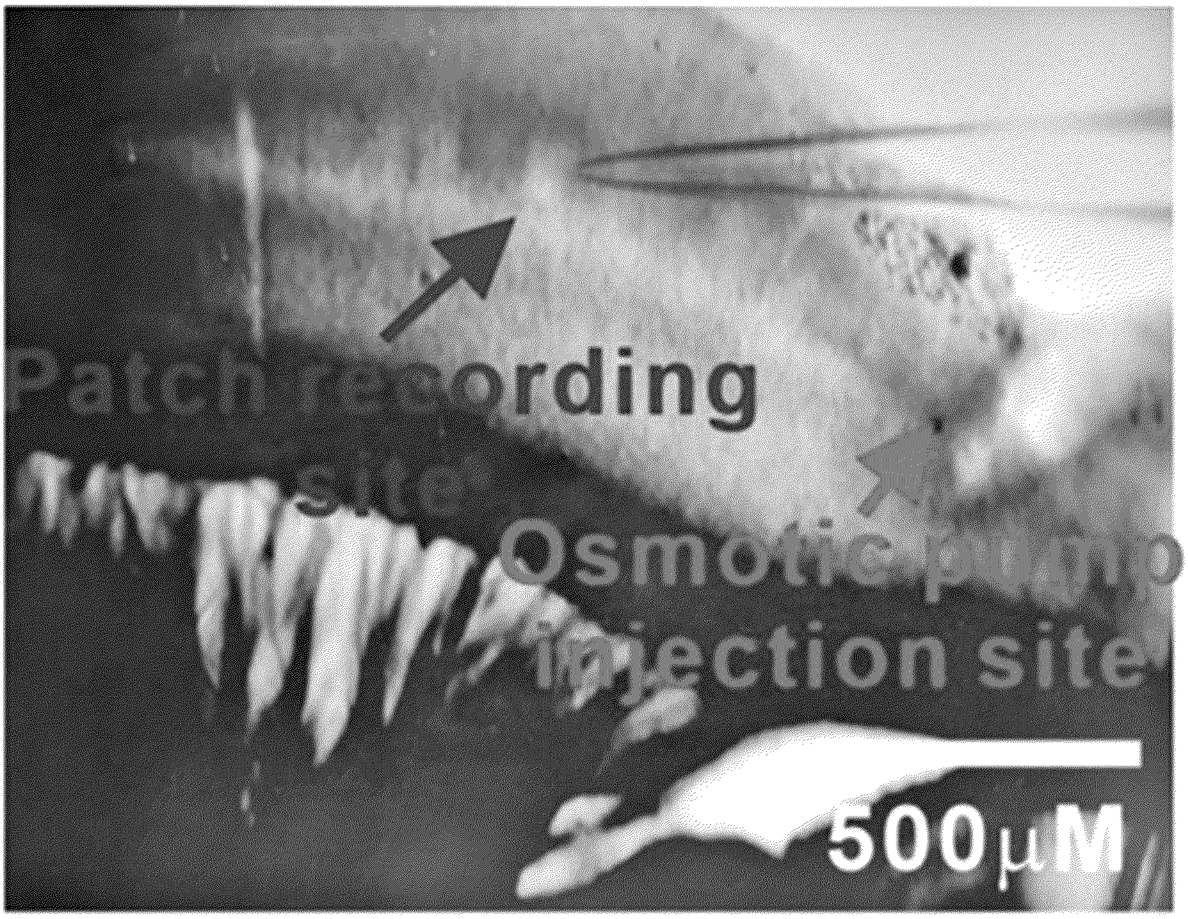

[FIG. 3A]

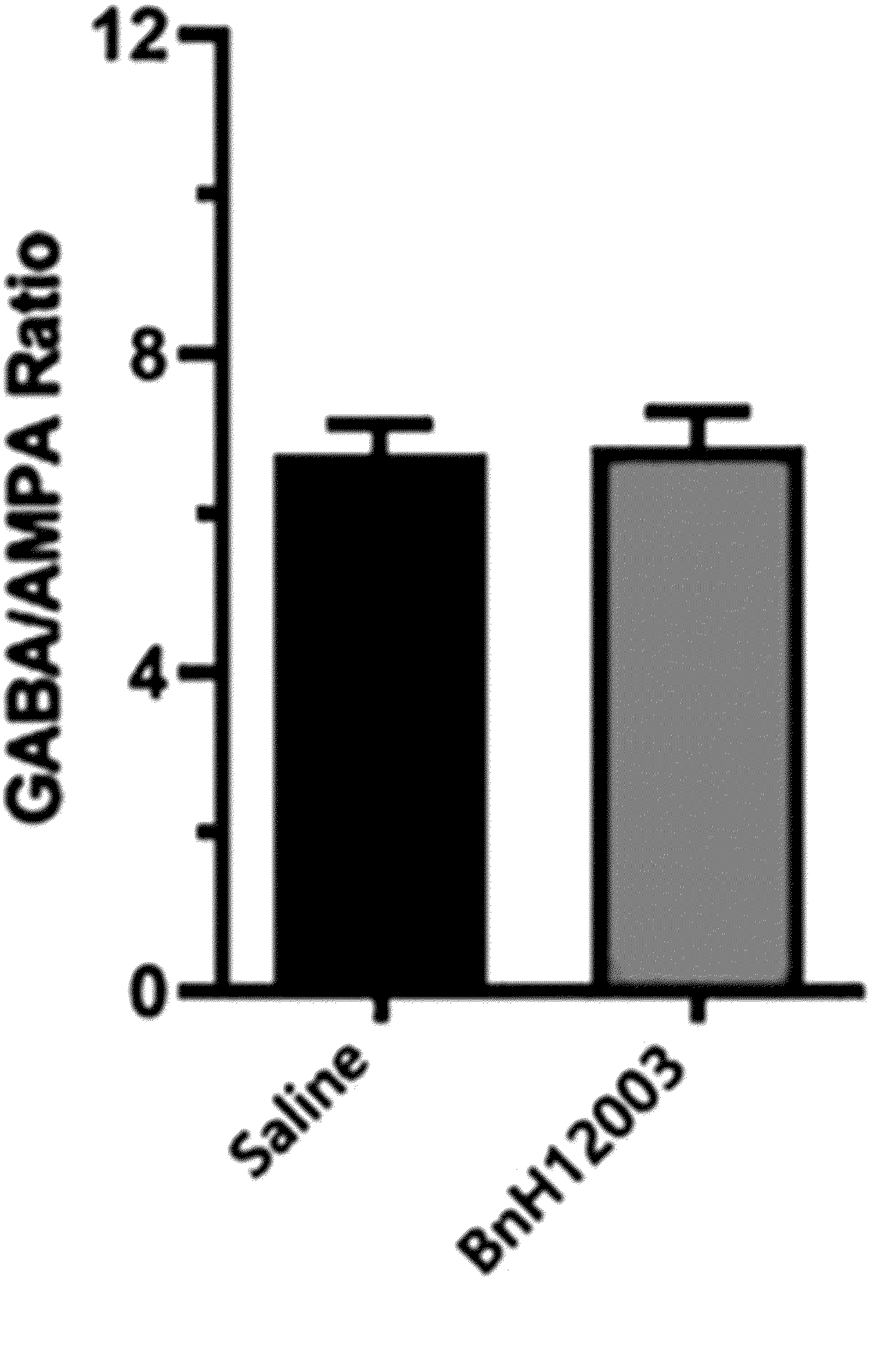

[FIG. 3B]

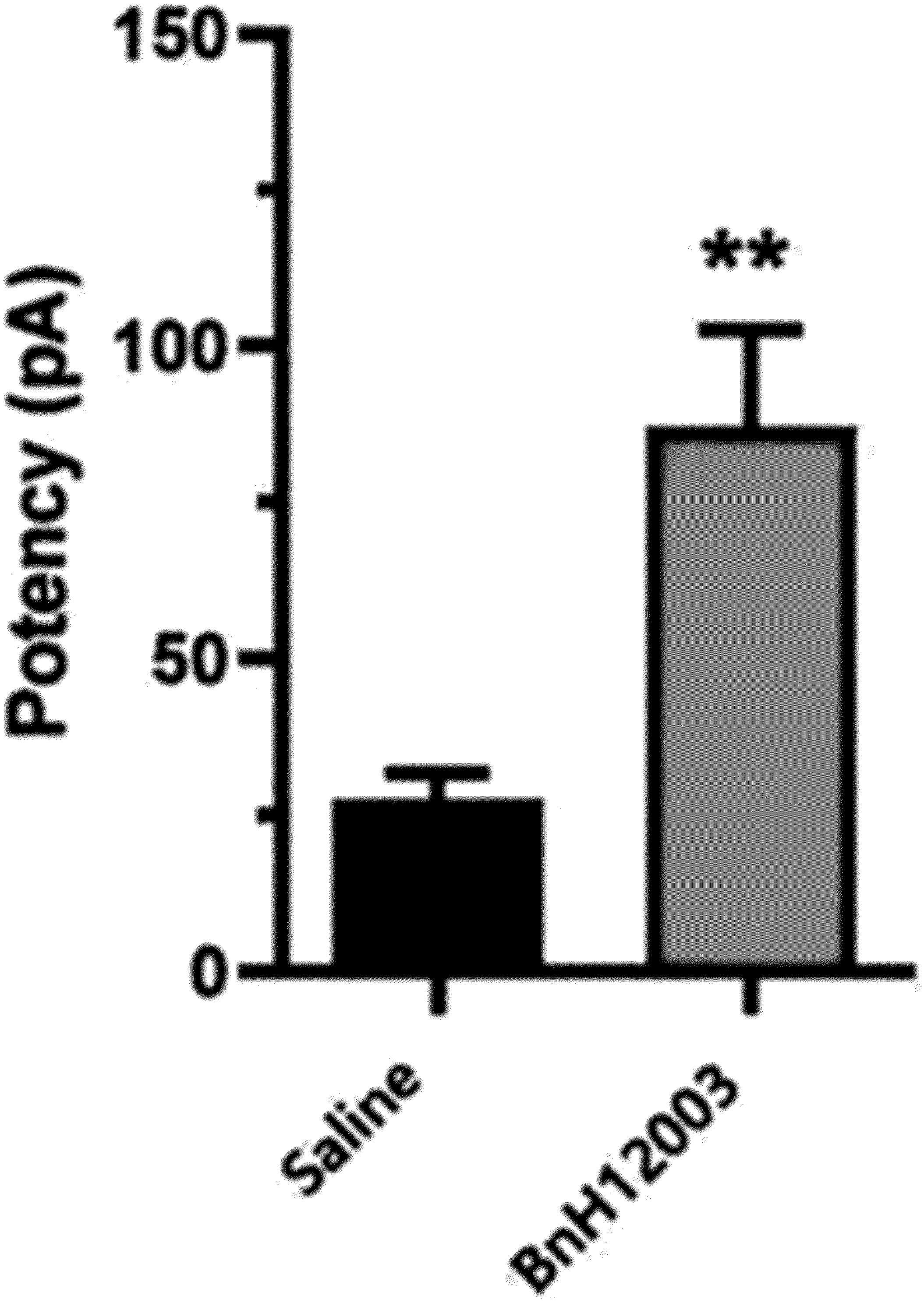

[FIG. 4A]

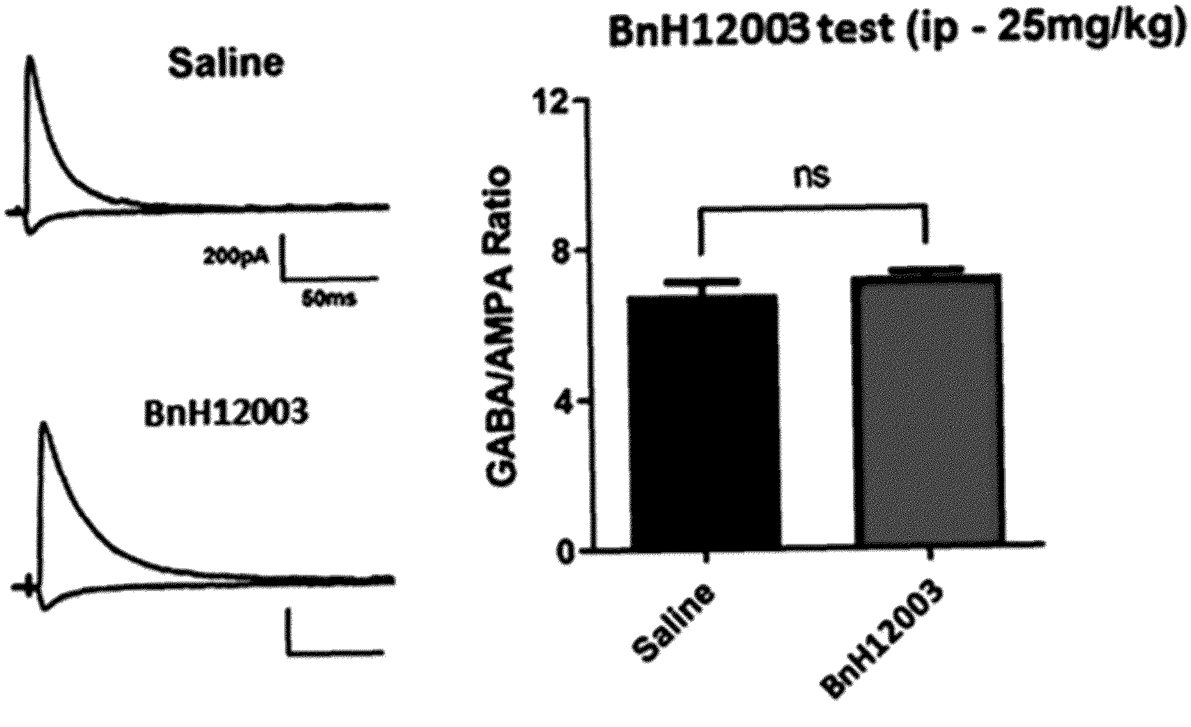
Saline
200pA
50ms
BnH12003
BnH12003 test (ip - 25mg/kg)
GABA/AMPA Ratio
12
8
4
0
ns
Saline
BnH12003

[FIG. 4B]

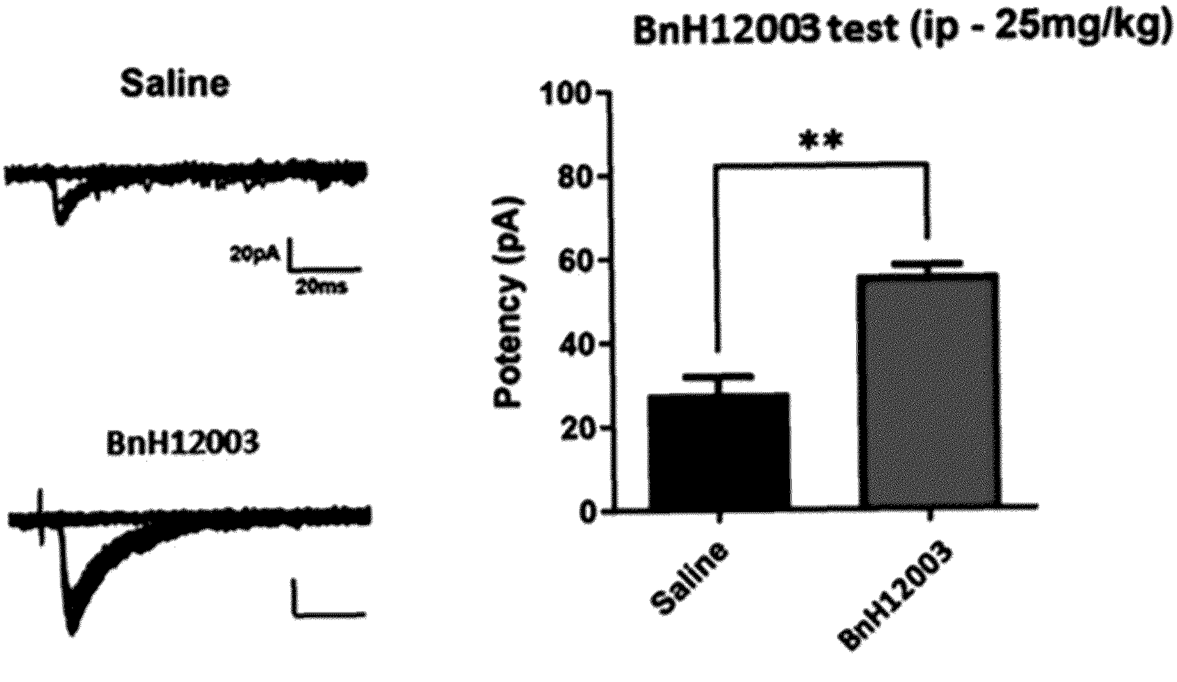
Saline
20pA
20ms
BnH12003
BnH12003 test (ip - 25mg/kg)
Potency (pA)
100
80
60
40
20
0
**
Saline
BnH12003

[FIG. 5]】

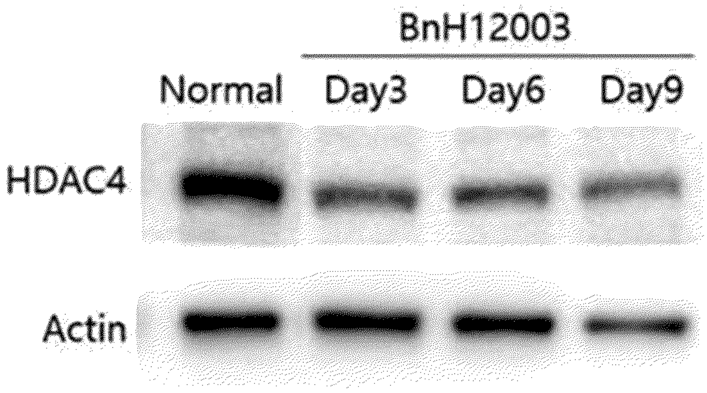
BnH12003
Normal
Day3
Day6
Day9
HDAC4
Actin

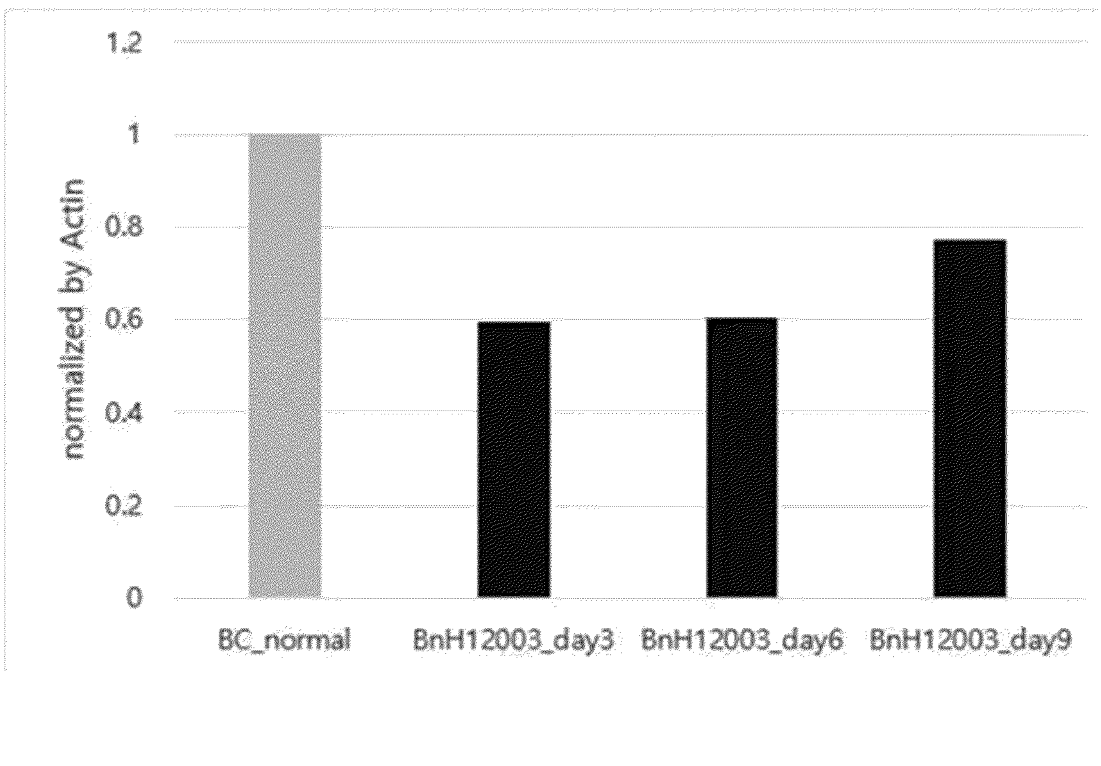
1.2
1
0.8
0.6
0.4
0.2
0
normalized by Actin
BC_normal
BnH12003_day3
BnH12003_day6
BnH12003_day9

[FIG. 6]

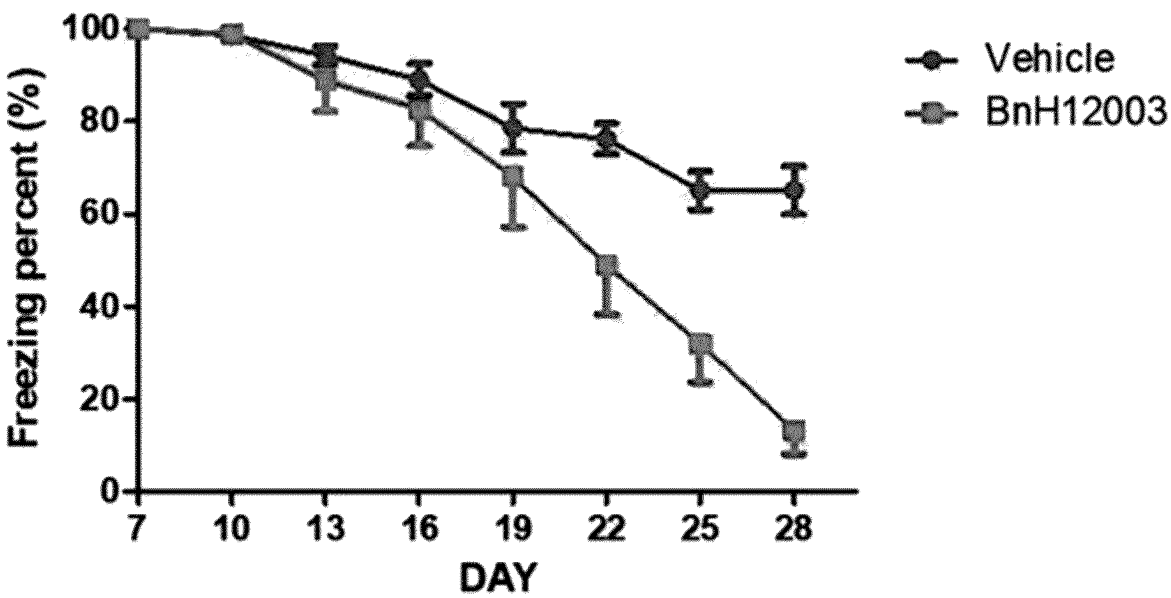
Freezing percent (%)
100
80
60
40
20
0
7
10
13
16
19
22
25
28
DAY
Vehicle
BnH12003

[FIG. 7A]

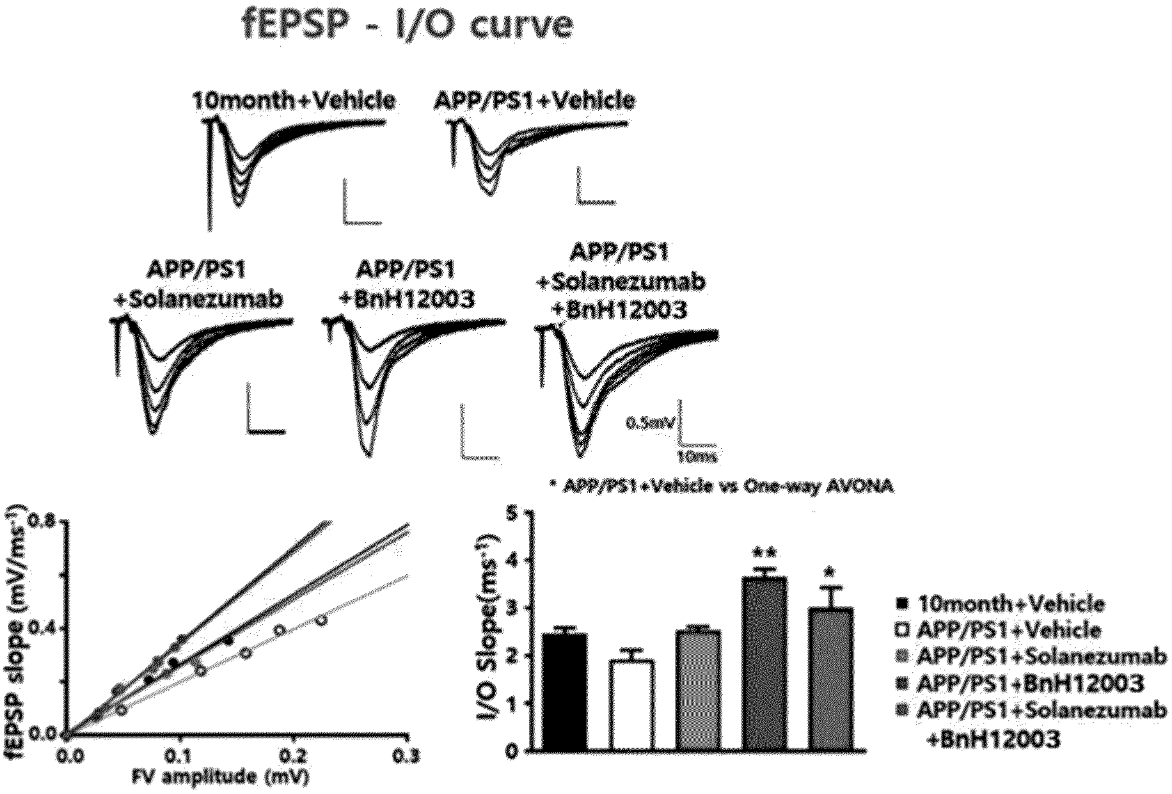
fEPSP - I/O curve
10month+Vehicle
APP/PS1+Vehicle
APP/PS1
+Solanezumab
APP/PS1
+BnH12003
APP/PS1
+Solanezumab
+BnH12003
0.5mV
10ms
* APP/PS1+Vehicle vs One-way AVONA
fEPSP slope (mV/ms-1)
FV amplitude (mV)
I/O Slope(ms-1)
**
*
10month+Vehicle
APP/PS1+Vehicle
APP/PS1+Solanezumab
APP/PS1+BnH12003
APP/PS1+Solanezumab
+BnH12003

[FIG. 7B]

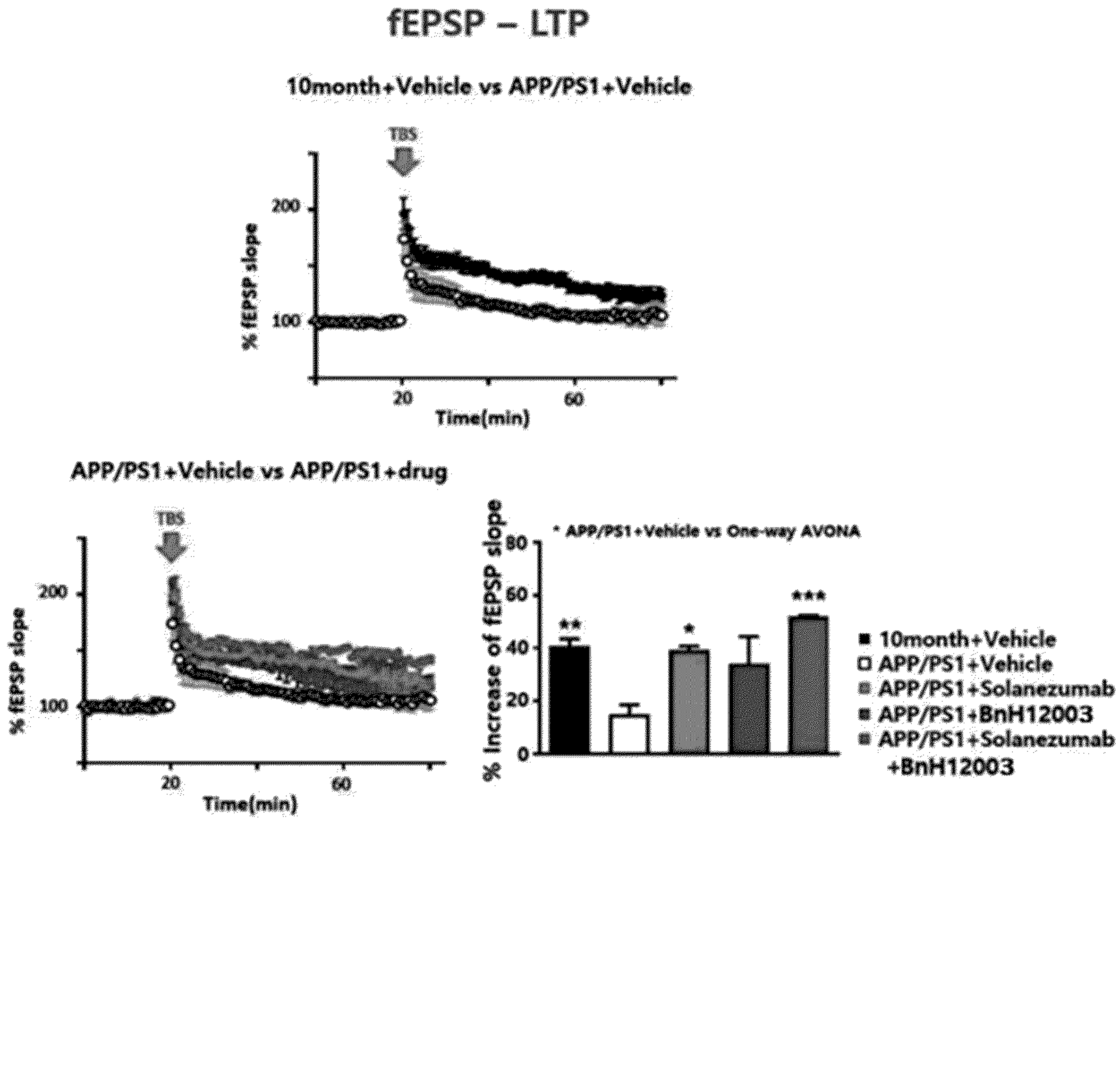
fEPSP – LTP
10month+Vehicle vs APP/PS1+Vehicle
TBS
200
100
% fEPSP slope
20
60
Time(min)
APP/PS1+Vehicle vs APP/PS1+drug
TBS
200
100
% fEPSP slope
20
60
Time(min)
* APP/PS1+Vehicle vs One-way AVONA
% Increase of fEPSP slope
80
60
40
20
0
**
*
***
10month+Vehicle
APP/PS1+Vehicle
APP/PS1+Solanezumab
APP/PS1+BnH12003
APP/PS1+Solanezumab +BnH12003

[FIG. 8A]

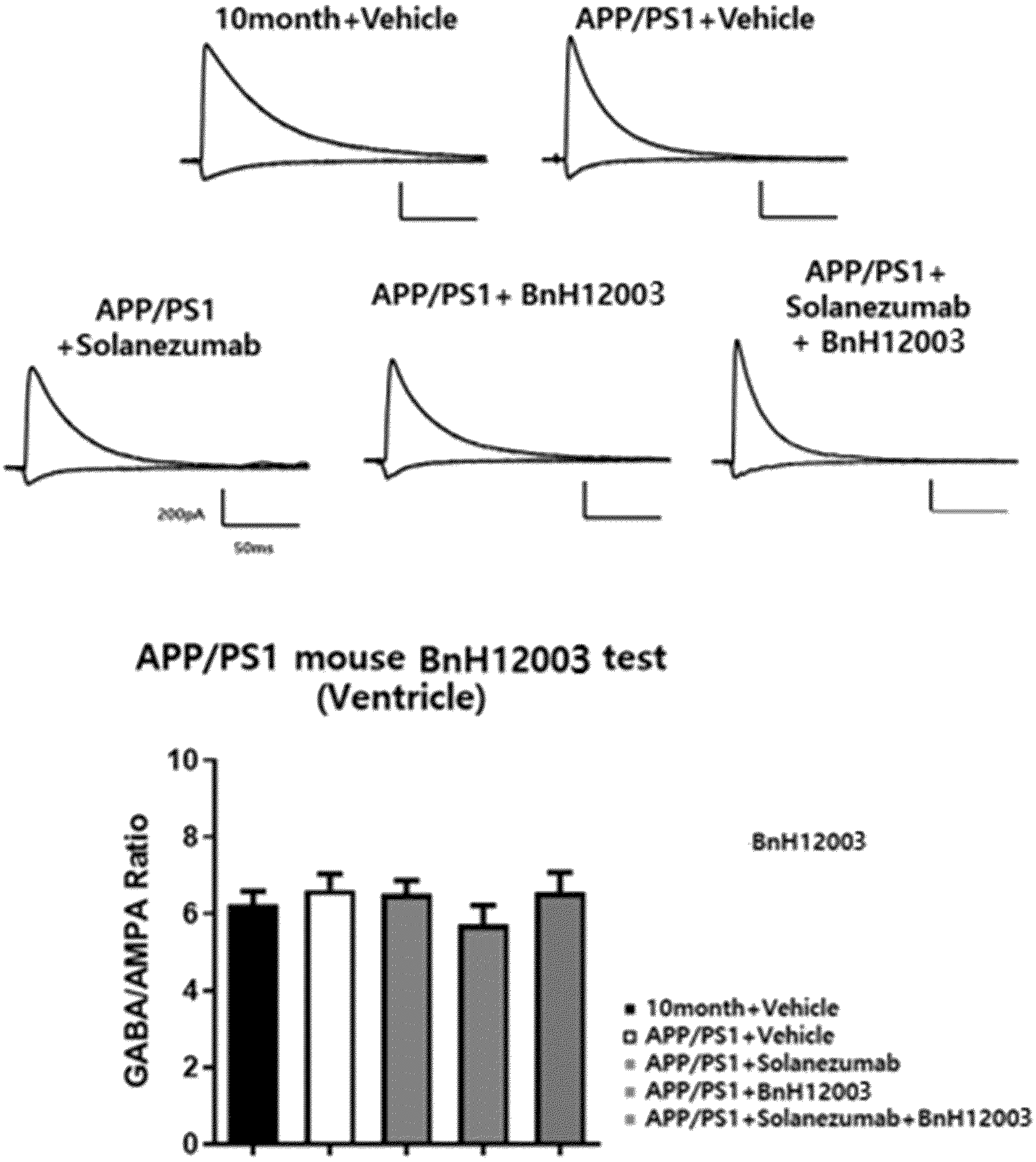
10month+Vehicle
APP/PS1+Vehicle
APP/PS1
+Solanezumab
APP/PS1+ BnH12003
APP/PS1+
Solanezumab
+ BnH12003
200pA
50ms
APP/PS1 mouse BnH12003 test
(Ventricle)
GABA/AMPA Ratio
10
8
6
4
2
0
BnH12003
10month+Vehicle
APP/PS1+Vehicle
APP/PS1+Solanezumab
APP/PS1+BnH12003
APP/PS1+Solanezumab+BnH12003

[FIG. 8B]

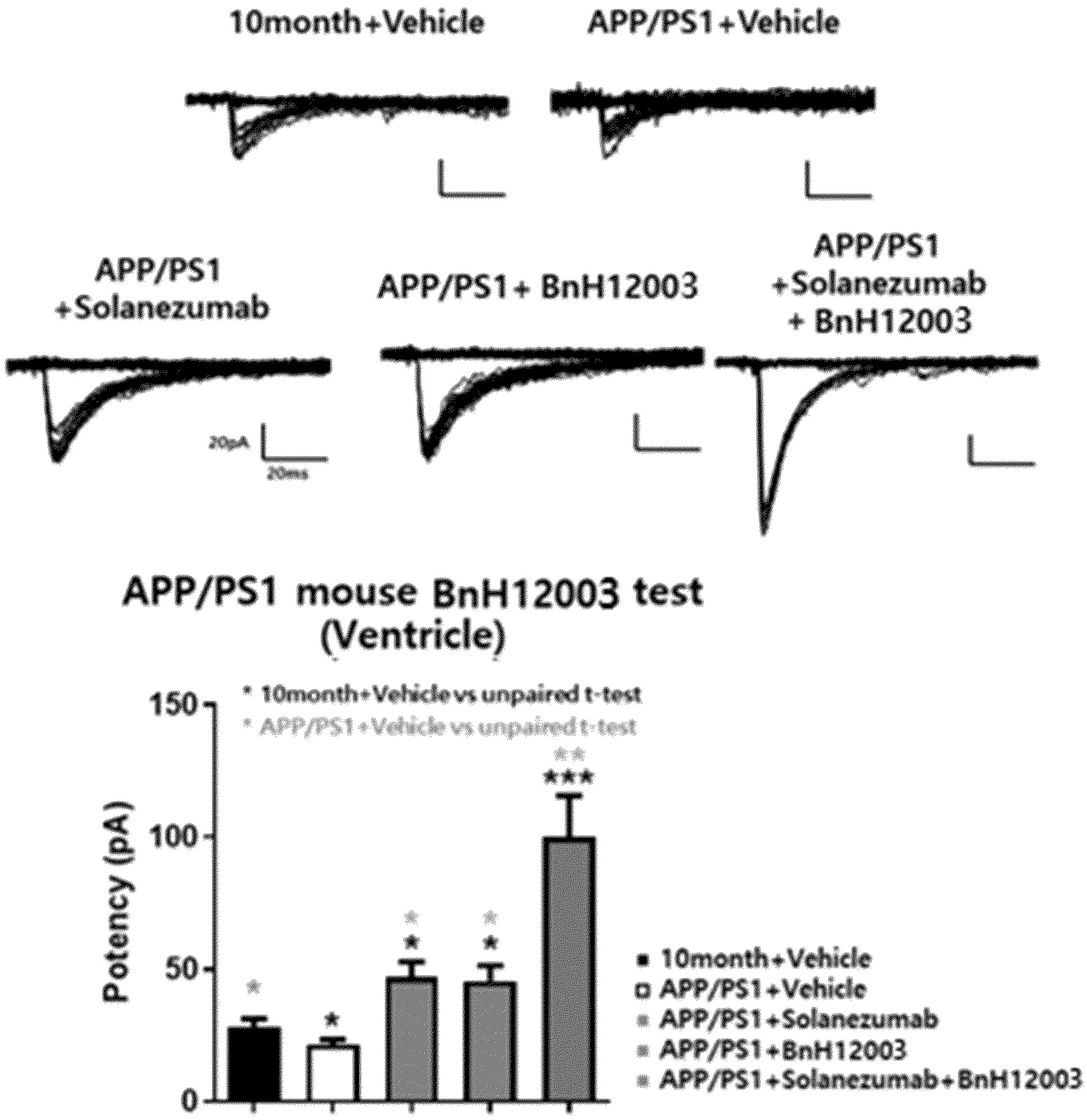
10month+Vehicle
APP/PS1+Vehicle
APP/PS1
+Solanezumab
APP/PS1+ BnH12003
APP/PS1
+Solanezumab
+ BnH12003
20pA
20ms
APP/PS1 mouse BnH12003 test
(Ventricle)
* 10month+Vehicle vs unpaired t-test
* APP/PS1+Vehicle vs unpaired t-test
Potency (pA)
150
100
50
0
10month+Vehicle
APP/PS1+Vehicle
APP/PS1+Solanezumab
APP/PS1+BnH12003
APP/PS1+Solanezumab+BnH12003

[FIG. 9]

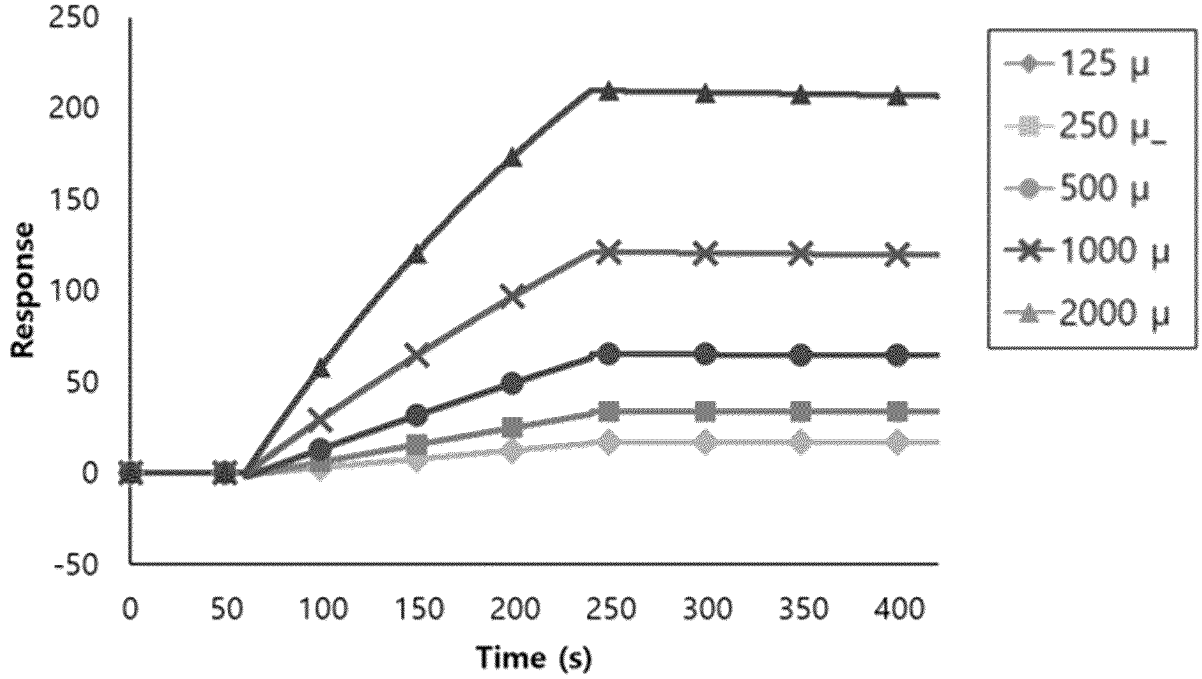
250
200
150
100
50
0
-50
Response
0
50
100
150
200
250
300
350
400
Time (s)
125 µ
250 µ_
500 µ
1000 µ
2000 µ

[FIG. 10A]

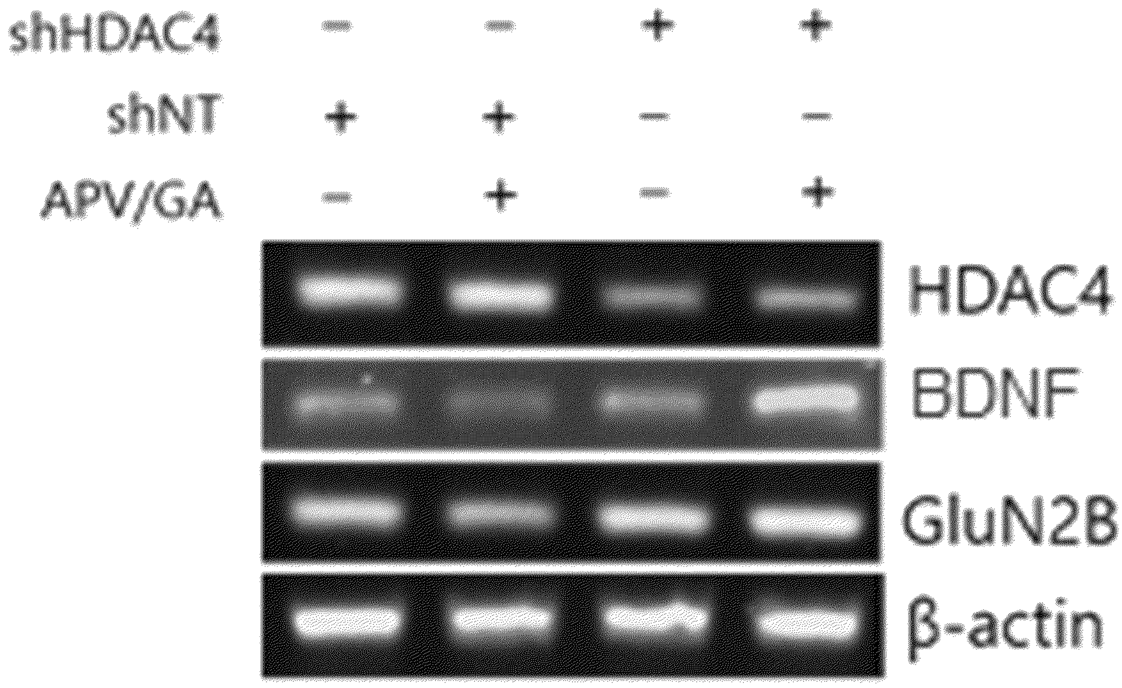

[FIG. 10B]

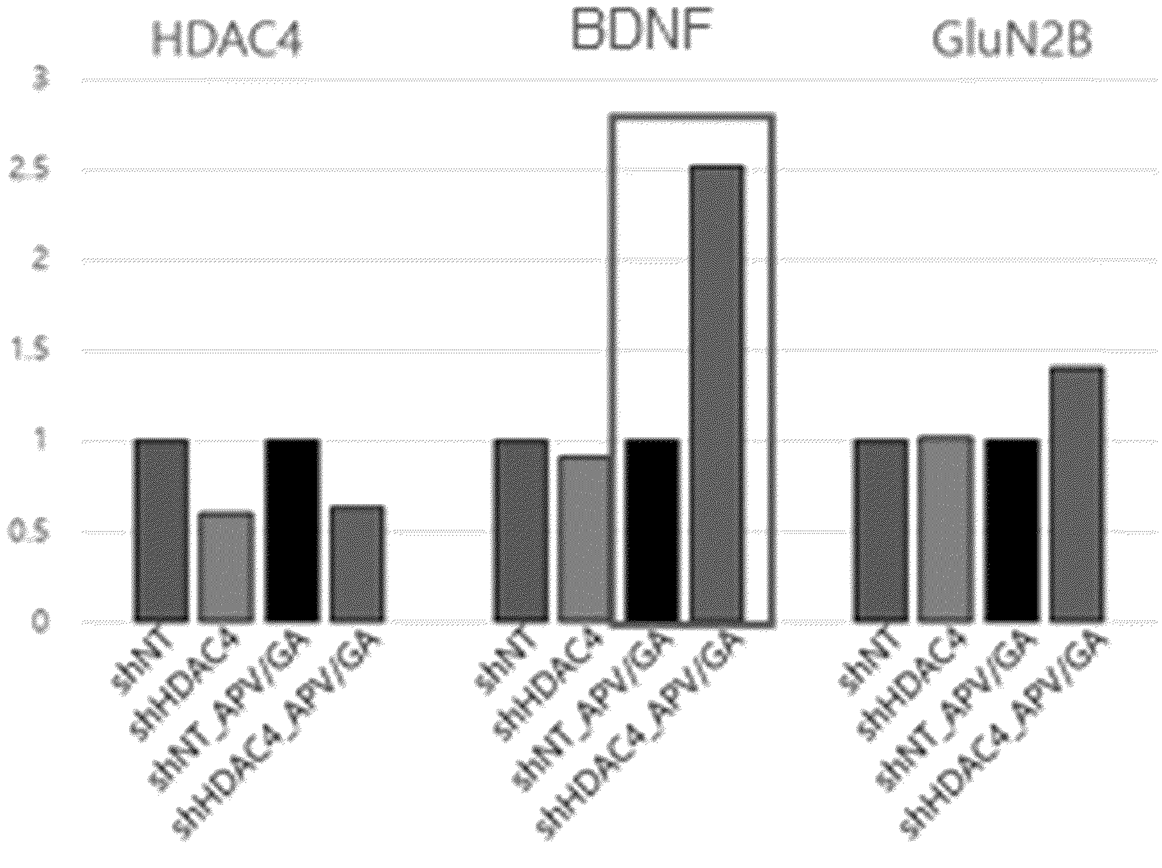
HDAC4
BDNF
GluN2B
3
2.5
2
1.5
1
0.5
0
shNT
shHDAC4
shNT_APV/GA
shHDAC4_APV/GA
shNT
shHDAC4
shNT_APV/GA
shHDAC4_APV/GA
shNT
shHDAC4
shNT_APV/GA
shHDAC4_APV/GA

[FIG. 11A]

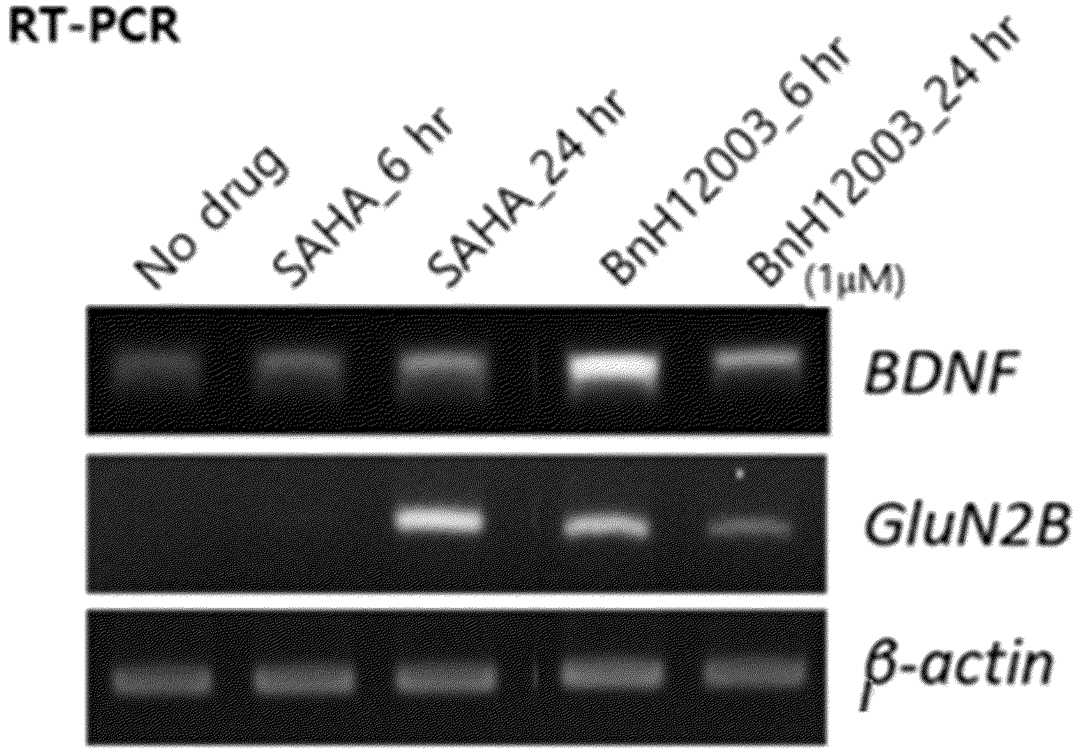
RT-PCR
No drug
SAHA_6 hr
SAHA_24 hr
BnH12003_6 hr
BnH12003_24 hr
(1μM)
BDNF
GluN2B
β-actin

[FIG. 11B]

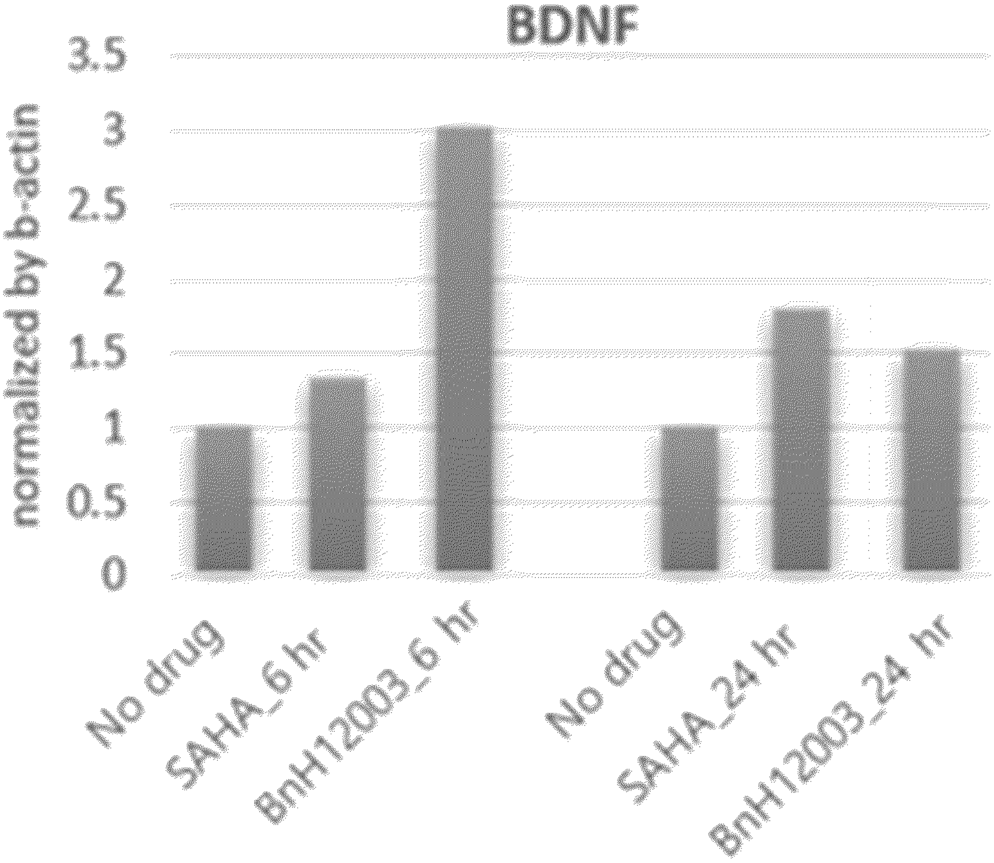
BDNF
normalized by b-actin
3.5
3
2.5
2
1.5
1
0.5
0
No drug
SAHA_6 hr
BnH12003_6 hr
No drug
SAHA_24 hr
BnH12003_24 hr

[FIG. 11C]

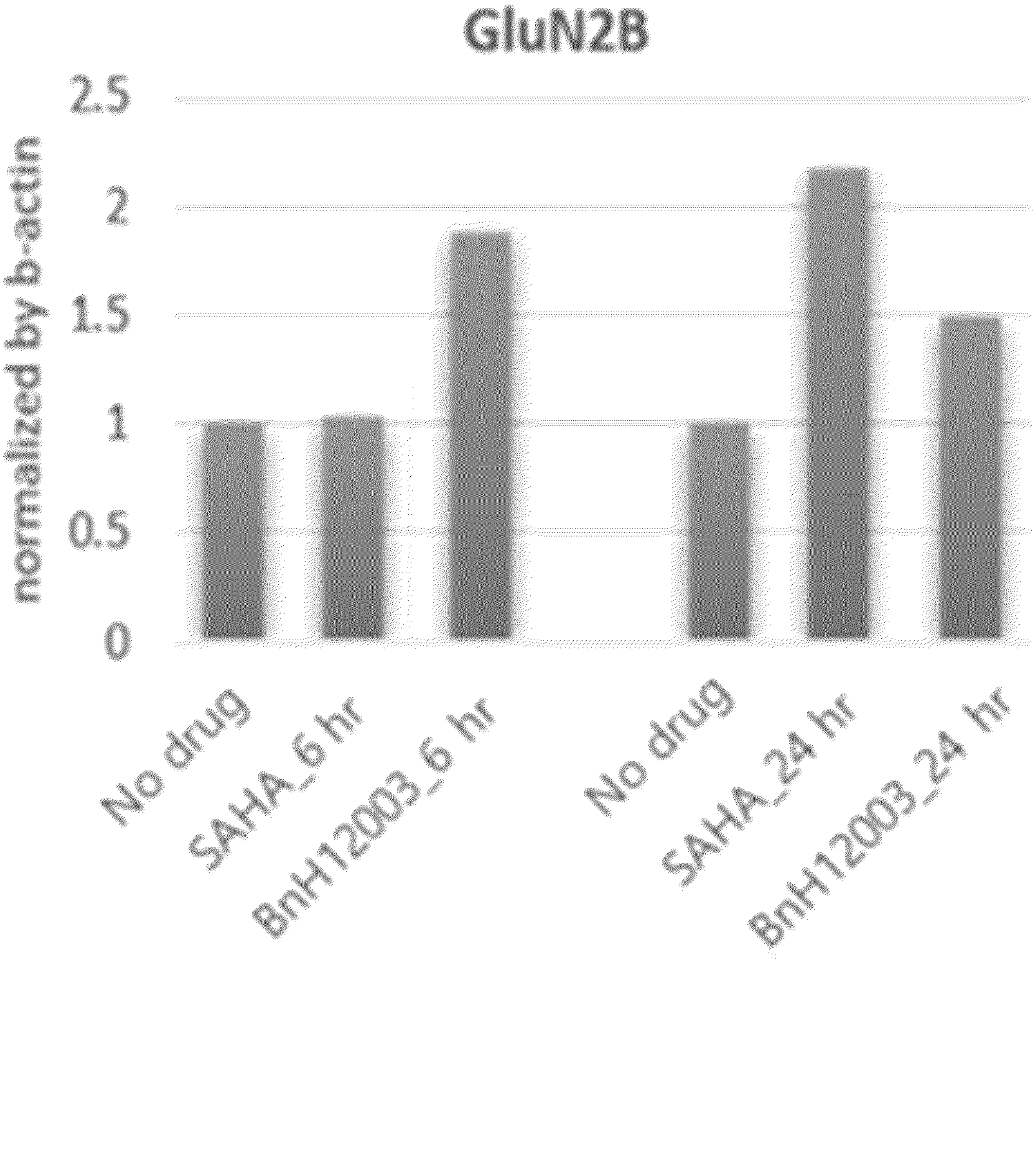
GluN2B
2.5
2
1.5
1
0.5
0
normalized by b-actin
No drug
SAHA_6 hr
BnH12003_6 hr
No drug
SAHA_24 hr
BnH12003_24 hr

[FIG. 12A]

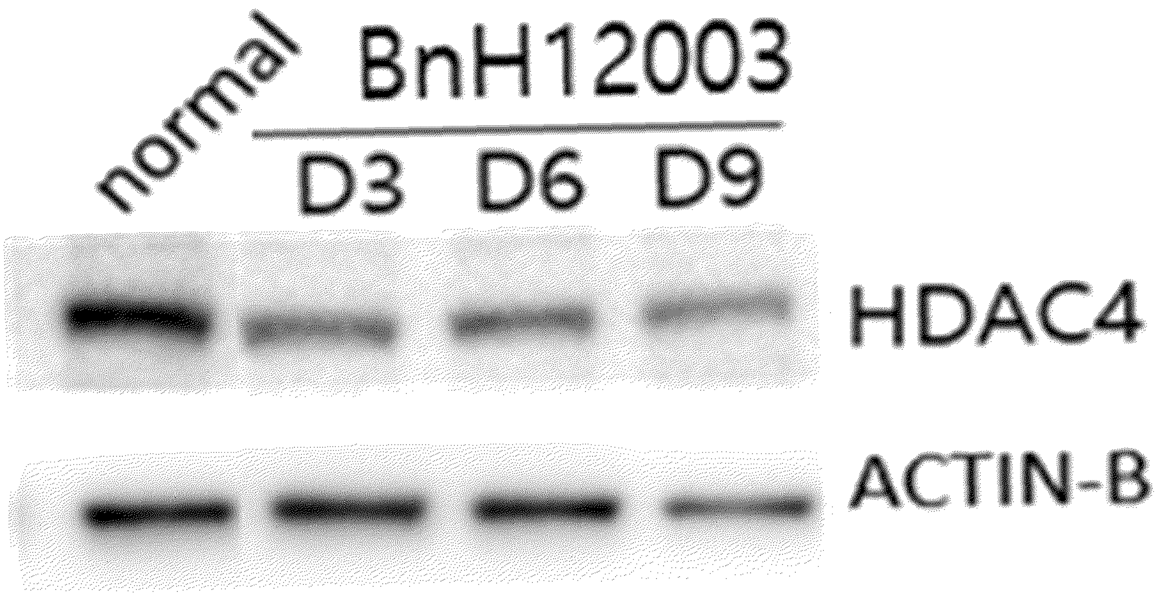

[FIG. 12B]

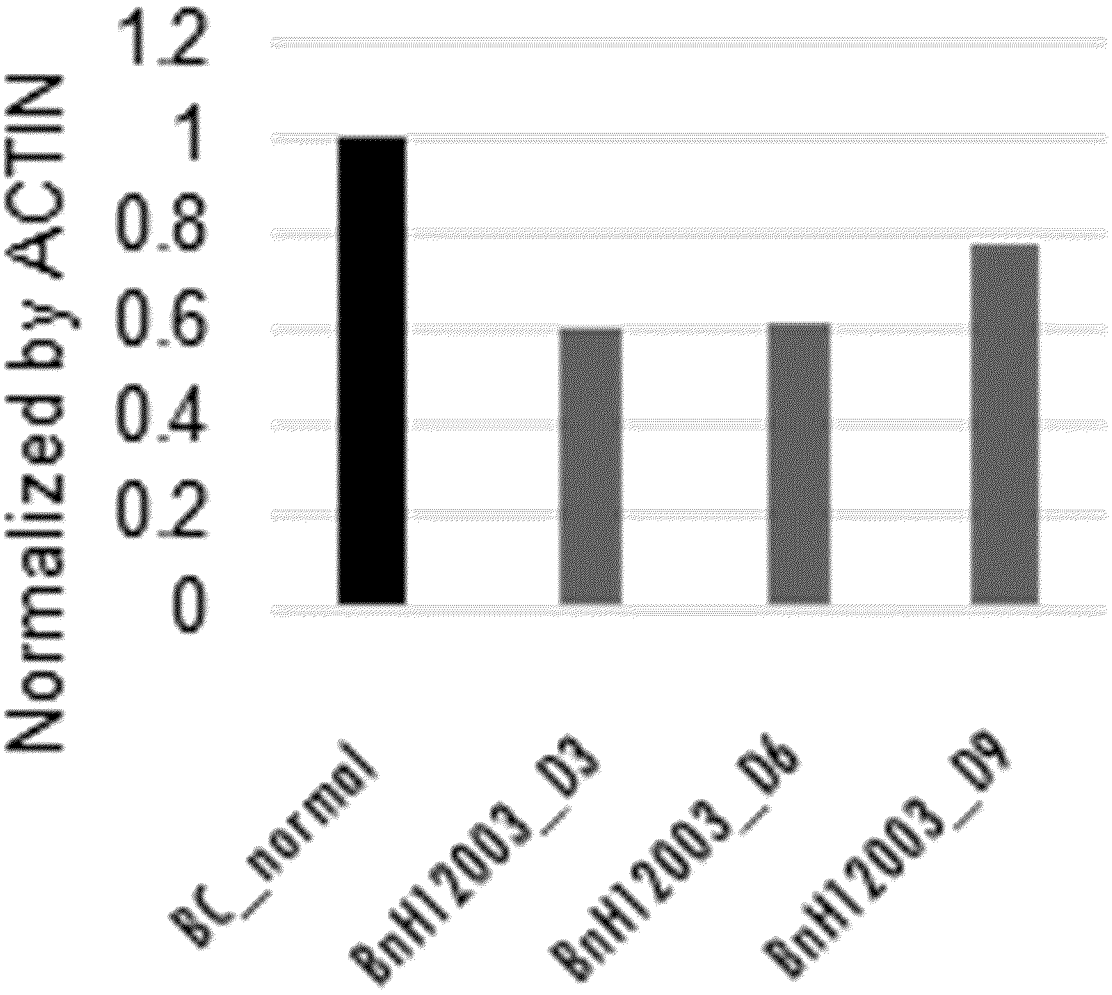
Normalized by ACTIN
1.2
1
0.8
0.6
0.4
0.2
0
BC_normal
BnHI2003_D3
BnHI2003_D6
BnHI2003_D9

# INTERNATIONAL SEARCH REPORT

International application No. **PCT/KR2023/004139**

**A. CLASSIFICATION OF SUBJECT MATTER**

**C07D 211/60**(2006.01)i; **C07D 401/10**(2006.01)i; **C07D 403/10**(2006.01)i; **C07D 401/06**(2006.01)i; **C07D 405/06**(2006.01)i; **C07D 409/10**(2006.01)i; **C07D 405/10**(2006.01)i; **C07D 413/10**(2006.01)i; **A61K 31/445**(2006.01)i; **A61P 25/28**(2006.01)i

According to International Patent Classification (IPC) or to both national classification and IPC

**B. FIELDS SEARCHED**

Minimum documentation searched (classification system followed by classification symbols)

C07D 211/60(2006.01); A61K 31/724(2006.01); A61K 45/06(2006.01); C07C 235/34(2006.01); C07D 257/04(2006.01); C07D 295/14(2006.01); C07D 295/18(2006.01); C07D 295/182(2006.01); C07D 295/205(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus), Google & keywords: 페닐(phenyl), 질소 함유 헤테로고리(nitrogen-containing heterocycle), 시냅스 가소성(synaptic plasticity), 신경퇴행성 질환(neurodegenerative disease), HDAC, 알츠하이머 질병(Alzheimer's disease), 외상 후 스트레스 장애(PTSD, post traumatic stress disorder)

**C. DOCUMENTS CONSIDERED TO BE RELEVANT**

| Category* | Citation of document, with indication, where appropriate, of the relevant passages | Relevant to claim No. |
|---|---|---|
| | WO 92-02490 A1 (MITSUI TOATSU CHEMICALS, INCORPORATED) 20 February 1992 (1992-02-20) | |
| X | See abstract; claim 1; table 1; and page 1. | 1,2,5-9,11-18 |
| Y | | 19,20 |
| A | | 3,4,10 |
| | KR 10-2021-0084480 A (CYCLO THERAPEUTICS, INC.) 07 July 2021 (2021-07-07) | |
| Y | See paragraphs [0010] and [0126]-[0127]. | 19,20 |
| | Chemical Abstract compound. STNext. RN 1682653-42-4. 13 April 2015. | |
| X | See the compounds. | 1-6,11-13 |
| | CN 111072597 A (SICHUAN UNIVERSITY) 28 April 2020 (2020-04-28) | |
| A | See entire document. | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 July 2023** | **25 July 2023** |
| Name and mailing address of the ISA/KR<br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2022)

INTERNATIONAL SEARCH REPORT

International application No.
**PCT/KR2023/004139**

**C. DOCUMENTS CONSIDERED TO BE RELEVANT**

| Category* | Citation of document, with indication, where appropriate, of the relevant passages | Relevant to claim No. |
|---|---|---|
| A | WO 2014-110103 A1 (ARENA PHARMACEUTICALS, INC.) 17 July 2014 (2014-07-17)<br>See entire document. | 1-20 |

Form PCT/ISA/210 (second sheet) (July 2022)

**INTERNATIONAL SEARCH REPORT**

International application No.
**PCT/KR2023/004139**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2023/004139**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21**
because they relate to subject matter not required to be searched by this Authority, namely:

Claim 21 pertains to a method for treatment of the human body [PCT Article 17(2)(a)(i) and PCT Rule 39.1(iv)].

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2023/004139**

| Patent document cited in search report | Publication date (day/month/year) | Patent family member(s) | Publication date (day/month/year) |
|---|---|---|---|
| WO 92-02490 A1 | 20 February 1992 | EP 0495998 A1 | 29 July 1992 |
| | | EP 0495998 A4 | 12 January 1994 |
| | | JP 05-032646 A | 09 February 1993 |
| | | US 5338745 A | 16 August 1994 |
| KR 10-2021-0084480 A | 07 July 2021 | BR 112021008139 A2 | 03 August 2021 |
| | | CA 3114021 A1 | 07 May 2021 |
| | | CN 112996562 A | 18 June 2021 |
| | | EP 3873604 A1 | 08 September 2021 |
| | | JP 2022-509358 A | 20 January 2022 |
| | | US 2022-0008453 A1 | 13 January 2022 |
| | | WO 2020-092107 A1 | 07 May 2020 |
| CN 111072597 A | 28 April 2020 | CN 111072597 B | 09 December 2022 |
| WO 2014-110103 A1 | 17 July 2014 | CN 105263914 A | 20 January 2016 |
| | | CN 105263914 B | 27 October 2017 |
| | | DK 3309150 T3 | 30 August 2021 |
| | | EP 2943473 A1 | 18 November 2015 |
| | | EP 2943473 B1 | 23 August 2017 |
| | | EP 3309150 A1 | 18 April 2018 |
| | | EP 3309150 B1 | 02 June 2021 |
| | | EP 3889141 A1 | 06 October 2021 |
| | | ES 2647965 T3 | 27 December 2017 |
| | | ES 2886914 T3 | 21 December 2021 |
| | | HK 1219953 A1 | 21 April 2017 |
| | | HK 1253019 A1 | 06 June 2019 |
| | | JP 2016-504388 A | 12 February 2016 |
| | | JP 6253668 B2 | 27 December 2017 |
| | | LT 3309150 T | 25 October 2021 |
| | | PT 3309150 T | 31 August 2021 |
| | | US 2015-0353488 A1 | 10 December 2015 |
| | | US 9365511 B2 | 14 June 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2006008517 A


### Non-patent literature cited in the description


- **YU et al.** *Neuron*, 2012, vol. 74, 731-42
- **PETRUS et al.** *Neuron*, 2014, vol. 81, 664-73
- **GAO et al.** *JNeurosci.*, 2014, vol. 34, 10770-9
- **VERDIN** ; **OTT**. *Nat Rev Mol Cell Biol*, 2015, vol. 16, 258-264
- **GROZINGER et al.** *Proc. Natl. Acad. Sci. USA*, 1999, vol. 96, 4868-4873
- **DARCY et al.** *J. Comp. Neurol.*, 2010, vol. 518, 722-740
- **SANDO et al.** *Cell*, 2012, vol. 151, 821-834
- **TAKASE et al.** *PloS ONE*, 2013, vol. 8, e58473
- **BOLGER** ; **YAO**. *JNeurosci.*, 2005, vol. 2005 (25), 9544-9553
- **MIELCAREK et al.** *PLoS Biol.*, 2013, vol. 11, e1001717
- **SAILAJA et al.** *Proc. Natl. Acad. Sci. USA*, 2012, vol. 109, E3687-E3695
- **BOLGER** ; **YAO**. *J. Neurosci.*, 2005, vol. 25, 9544-9553
- **LI et al.** *Nat. Med.*, 2012, vol. 18, 783-790
- **YANG et al.** *FEBS Lett.*, 2011, vol. 585, 761-766
- **SARKAR et al.** *Neuropsychopharmacology*, 2014, vol. 39, 2221-2232
- **FITZSIMONS et al.** *PloS ONE*, 2013, vol. 8, e83903
- **MAIN et al.** *Front. Mol. Neurosci.*, 2021, vol. 14, 616-642
- **YEH et al.** *Brain Res.*, 2013, vol. 1504, 16-24
- **WHITEHOUSE et al.** *Neuropathol. Appl. Neurobiol.*, 2014, vol. 41, 245257
- **TAKAHASHI-FUJIGASAKI** ; **FUJIGASAKI**. *Neuropathol. Appl. Neurobiol.*, 2006, vol. 32, 562-566
- **NARDONE et al.** *Transl. Psychiatry*, 2014, vol. 4, e433
- **GERVAIN et al.** *Front Syst Neurosci.*, 2013, vol. 7, 102
- **BARONCELLI L et al.** *JNeurosci*, 2016, vol. 36, 3430-40
- **BARONCELLI L et al.** *JNeurosci.*, 2016, vol. 36, 3430-40
- **BAGHERI et al.** *Int. J. Mol. Sci.*, 2019, vol. 20, 1109
- **HOCKLY et al.** *J Biol. Chem.*, 2003, vol. 278, 16059-16072
- **GARDIAN et al.** *J. Biol. Chem.*, 2005, vol. 280, 556-563
- **ZIELONKA et al.** *Front. Physiol.*, 2014, vol. 5, 380
- **MIELCAREK et al.** *PLoS Genet.*, 2014, vol. 10, e1004550
- **MIELCAREK et al.** *PloS ONE*, 2014, vol. 9, e108961
- **ZIELONKA et al.** *Exp. Clin. Cardiol.*, 2014, vol. 20, 25472554
- **MIELCAREK et al.** *PloS ONE*, 2011, vol. 6, e27746
- **KOPPEL** ; **TIMMUSK**. *Neuropharmacology*, 2013, vol. 75, 106-11
- **MEGHAN E. et al.** *Bone*, 2011, vol. 48, 1117-1126
- Diagnostic and Statistical Manual of Mental Disorders. American Psychiatric Association, 1994
- **GREENE et al.** Protective Groups in Organic Synthesis. John Wiley and Sons, 1991
- **CITROME**. *Psychopharmacol. Bull.*, 2003, vol. 37 (2), 74-88
- **JOHANNESSEN**. *CNS Drug Rev.*, 2003, vol. 9, 199-216
- **TSANKOVA et al.** *Nat. Neurosci.*, 2006, vol. 9, 519-525
- **MIELCAREK et al.** *PLoSBiol.*, 2013, vol. 11, e1001717
- **FRAY et al.** CANTAB battery: proposed utility in neurotoxicology.. *Neurotoxicol Teratol*, 1996, vol. 18 (4), 499-504
- **DEHAENE et al.** Reward-dependent learning in neuronal networks for planning and decision making.. *Brain Res.*, 2000, vol. 126, 21729
- **IVERSON et al.** Interpreting change on the WAIS-III/WMS-I11 in clinical samples.. *Arch Clin Neuropsychol.*, 2001, vol. 16 (2), 183-91

- **WEAVER et al.** Mild memory impairment in healthy older adults is distinct from normal aging.. *Cogn.*, 2006, vol. 60 (2), 146-55
- **LATTAL et al.** *Behav. Neurosci.*, 2007, vol. 121 (5), 1125-1131
- Cognitive-Behavioral Therapies. Guilford Publications, Inc., 2002
- **JUDITH S.S. BECK**. The new Handbook of Cognitive Therapy: Basics and Beyond. Guilford Publications, Inc., 1995